# EUROPEAN PATENT SPECIFICATION

(11) **EP 2 861 765 B1**
(45) Date of publication and mention of the grant of the patent: **23.01.2019**
(21) Application number: 13803548.0
(22) Date of filing: 14.06.2013
(51) Int. Cl.: C12Q 1/68, G01N 33/53, C40B 30/04

(54) **METHODS OF DETECTING DISEASES OR CONDITIONS**
VERFAHREN ZUM ERKENNEN VON KRANKHEITEN ODER LEIDEN
PROCÉDÉS DE DÉTECTION DE MALADIES OU D'ÉTATS

(30) Priority: 15.06.2012 US 201261660427 P
(43) Date of publication of application: 22.04.2015
(73) Proprietor: Progenity, Inc., San Diego, CA 92122 (US)
(72) Inventor: Stylli, Harry, La Jolla, CA 92037 (US)
(74) Representative: Vossius & Partner Patentanwälte Rechtsanwälte mbB
(86) International application number: PCT/US2013/046020
(87) International publication number: WO 2013/188846

(56) References cited:
- WO-A1-2012/012717
- WO-A2-2012/012693
- WO-A2-2012/012693
- US-A1- 2012 053 073
- A. J. SEHNERT ET AL: "Optimal Detection of Fetal Chromosomal Abnormalities by Massively Parallel DNA Sequencing of Cell-Free Fetal DNA from Maternal Blood", CLINICAL CHEMISTRY, vol. 57, no. 7, 1 July 2011 (2011-07-01), pages 1042-1049, XP055035090, ISSN: 0009-9147, DOI: 10.1373/clinchem.2011.165910
- CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, NATIONAL ACADEMY OF SCIENCES, US, vol. 105, no. 51, 23 December 2008 (2008-12-23), pages 20458-20463, XP002620454, ISSN: 0027-8424, DOI: 10.1073/PNAS.0810641105 [retrieved on 2008-12-10]
- WEILAND, M ET AL.: 'Small RNAs Have A Large Impact: Circulating microRNAs As Biomarkers For Human Diseases' RNA BIOLOGY vol. 9, no. 6, 01 June 2012, pages 850 - 859, XP055181973
- CARPAGANO, GE ET AL.: 'Microsatellite Alterations And Cell-Free DNA Analysis: Could They Increase the Cytology Sensitivity In The Diagnosis Of Malignant Pleural Effusion?' REJUVENATION RESEARCH vol. 15, no. 3, 02 May 2012, pages 265 - 273, XP055181975

## Description

### Priority Information

### Field

This invention is defined by the claims. Generally described herein are methods of using combinations of two or more different components selected from cell-free bodily fluids, phagocytic cells, circulating vesicles, and circulating diseased cells in the diagnosis, prognosis, or monitoring of a disease or condition. Also described herein are methods of using the combinations to identify markers of diseases or conditions.

### Background of the Invention

Leukocytes begin as pluripotent hematopoietic stem cells in the bone marrow and develop along either the myeloid lineage (monocytes, macrophages, neutrophils, eosinophils, and basophils) or the lymphoid lineage (T and B lymphocytes and natural killer cells). The major function of the myeloid lineage cells (e.g., neutrophils and macrophages) is the phagocytosis of infectious organisms, live unwanted damaged cells, senescent and dead cells (apoptotic and necrotic), as well as the clearing of cellular debris. Phagocytes from healthy animals do not replicate and are diploid, i.e., have a DNA content of 2n. On average, each cell contains <10 ng DNA, <20 ng RNA, and <300 ng of protein. Non-phagocytic cells are also diploid and are not involved in the internalization of dead cells or infectious organisms and also have a DNA content of 2n.

The lifetime of various white blood cell subpopulations varies from a few days (e.g., neutrophils) to several months (e.g., macrophages). Like other cell types, leukocytes age and eventually die. During their aging process, human blood- and tissue-derived phagocytes (e.g., neutrophils) exhibit all the classic markers of programmed cell death (i.e., apoptosis), including caspase activation, pyknotic nuclei, and chromatin fragmentation. These cells also display a number of "eat-me" flags (e.g., phosphatidylserine, sugars) on the extracellular surfaces of their plasma membranes. Consequently, dying and dead cells and subcellular fragments thereof are cleared from tissues and blood by other phagocytic cells.

Early diagnosis of a disease often increases the likelihood of successful treatment or cure of such disease. Current diagnostic methods, however, focus on either using whole blood or separating the blood into different components, of which a single component is chosen for testing. Although this approach may enrich the signal being detected, it also results in the loss of potentially important information. Personalized diagnostic methods are needed that enable the diagnosis, especially the early diagnosis, of the presence of a disease or a condition in individuals who are not known to have the disease or who have recurrent disease.

One object of the present invention is to provide diagnostic methods that can facilitate the detection of a disease or condition-specific markers, e.g., nucleic acids, proteins, carbohydrates, and/or lipids and the like by using combinations of two or more different components selected from cell-free bodily fluids, phagocytic cells, circulating vesicles, and circulating diseased cells. Another object of this invention is to provide methods of identifying a disease or condition-specific markers and further use such markers alone or together with any known markers to diagnose diseases or conditions.

### Summary

The present invention is defined by the claims. Accordingly, the present invention relates to a method for diagnosing or aiding in the diagnosis of a disease or condition, or assessing the risk of developing a disease or condition, or prognosing or aiding in the prognosis of a disease or condition, in a fetus comprising:
a) determining a profile of one or more markers of the disease or condition using a combination sample isolated from a maternal subject carrying the fetus, the sample comprising a cell-free bodily fluid isolated from the maternal subject and at least one of a phagocytic cell isolated from the maternal subject and a circulating diseased cell isolated from the maternal subject, wherein at least one of the phagocytic cells comprises fetal cells or fragments thereof and at least one of the circulating diseased cells is a fetal cell;
b) determining, using the profile determined from the combination sample in step a), a control maternal profile of at least one of the one or more markers; and
c) identifying a difference between the profile of a) and the profile of b), wherein the difference is indicative of the presence of said disease or condition, or of the risk of developing said disease or condition, or of the prognosis of said disease or condition, respectively, in the fetus.
The present invention also relates a method for diagnosing or aiding in the diagnosis of a disease or condition, or assessing the risk of developing a disease or condition, or prognosing or aiding in the prognosis of a disease or condition, in a fetus comprising:
a) determining a profile of one or more markers of the disease or condition using a combination sample isolated from a maternal subject carrying the fetus, the sample comprising an analyte isolated from a cell-free bodily fluid isolated from the maternal subject and at least one of an analyte isolated from a population of phagocytic cells isolated from the maternal subject and an analyte isolated from a population of circulating diseased cells isolated from the maternal subject, wherein at least one of the phagocytic cells comprises fetal cells or fragments thereof and at least one of the circulating diseased cells is a fetal cell;
b) determining using the profile determined fro the combination sample in step a), a control maternal profile of at least of the one or more markers; and
c) identifying a difference between the profile of a) and the profile of b), wherein the difference is indicative of the presence of said disease or condition, or of the risk of developing said disease or condition, or of the prognosis of said disease or condition, respectively, in the fetus.
Also described herein are the following items:
1. A method for diagnosing or aiding in the diagnosis of a disease or condition in a subject comprising:
   a) determining a first profile of one or more markers of the disease or condition from a sample comprising two or more different components selected from the group consisting of: a cell-free bodily fluid isolated from the subject, a population of phagocytic cells isolated from the subject, a population of phagocytic cells having a DNA content more than 2n (>2n phagocytic cells) isolated from the subject, a population of circulating vesicles isolated from the subject, and a population of circulating diseased cells isolated from the subject;
   b) determining a second profile of at least one of the one or more markers from a control comprising a component selected from the group consisting of: a population of phagocytic cells having a DNA content of 2n (=2n phagocytic cells) isolated from the subject, a population of non-phagocytic cells isolated from the subject, and a population of control cells isolated from the subject, wherein the control cells are substantially free of cells affected by the disease or condition; and
   c) identifying a difference between the first and second profiles, wherein the difference is indicative of the presence of said disease or condition in the subject.
2. A method for assessing the risk of developing a disease or condition in a subject comprising:
   a) determining a first profile of one or more markers of the disease or condition from a sample comprising two or more different components selected from the group consisting of: a cell-free bodily fluid isolated from the subject, a population of phagocytic cells isolated from the subject, a population of phagocytic cells having a DNA content more than 2n (>2n phagocytic cells) isolated from the subject, a population of circulating vesicles isolated from the subject, and a population of circulating diseased cells isolated from the subject;
   b) determining a second profile of at least one of the one or more markers from a control comprising a component selected from the group consisting of: a population of phagocytic cells having a DNA content of 2n (=2n phagocytic cells) isolated from the subject, a population of non-phagocytic cells isolated from the subject, and a population of control cells isolated from the subject, wherein the control cells are substantially free of cells affected by the disease or condition; and
   c) identifying a difference between the first and second profiles, wherein the difference is indicative of the risk of developing said disease or condition in the subject.
3. A method for prognosing or aiding in the prognosis of a disease or condition in a subject comprising:
   a) determining a first profile of one or more markers of the disease or condition from a sample comprising two or more different components selected from the group consisting of: a cell-free bodily fluid isolated from the subject, a population of phagocytic cells isolated from the subject, a population of phagocytic cells having a DNA content more than 2n (>2n phagocytic cells) isolated from the subject, a population of circulating vesicles isolated from the subject, and a population of circulating diseased cells isolated from the subject;
   b) determining a second profile of at least one of the one or more markers from a control comprising a component selected from the group consisting of: a population of phagocytic cells having a DNA content of 2n (=2n phagocytic cells) isolated from the subject, a population of non-phagocytic cells isolated from the subject, and a population of control cells isolated from the subject, wherein the control cells are substantially free of cells affected by the disease or condition; and
   c) identifying a difference between the first and second profiles, wherein the difference is indicative of the prognosis of said disease or condition in the subject.
4. A method for assessing the efficacy of a treatment for a disease or condition in a subject comprising:
   a) determining a first profile of one or more markers of the disease or condition from a first sample comprising two or more different components selected from the group consisting of: a cell-free bodily fluid isolated from the subject before the treatment, a population of phagocytic cells isolated from the subject before the treatment, a population of phagocytic cells having a DNA content more than 2n (>2n phagocytic cells) isolated from the subject before the treatment, a population of circulating vesicles isolated from the subject before the treatment, and a population of circulating diseased cells isolated from the subject before the treatment;
      determining a second profile of at least one of the one or more markers from a first control comprising a component selected from the group consisting of: a population of phagocytic cells having a DNA content of 2n (=2n phagocytic cells) isolated from the subject before the treatment, a population of non-phagocytic cells isolated from the subject before the treatment, and a population of control cells isolated from the subject, wherein the control cells are substantially free of cells affected by the disease or condition, before the treatment;
      identifying a difference between the first and second profiles;
   b) determining a third profile of one or more markers of the disease or condition from a second sample comprising two or more different components selected from the group consisting of: a cell-free bodily fluid isolated from the subject after the treatment, a population of phagocytic cells isolated from the subject after the treatment, a population of phagocytic cells having a DNA content more than 2n (>2n phagocytic cells) isolated from the subject after the treatment, a population of circulating vesicles isolated from the subject after the treatment, and a population of circulating diseased cells isolated from the subject after the treatment;
      determining a fourth profile of at least one of the one or more markers from a second control comprising a component selected from the group consisting of: a population of phagocytic cells having a DNA content of 2n (=2n phagocytic cells) isolated from the subject after the treatment, a population of non-phagocytic cells isolated from the subject after the treatment, and a population of control cells isolated from the subject, wherein the control cells are substantially free of cells affected by the disease or condition, after the treatment;
      identifying a difference between the third and fourth profiles; and
   c) identifying a difference between the difference identified in a) and the difference identified in b) wherein the identified difference in c) is indicative of the efficacy of the treatment for said disease or condition in the subject.
5. A method for monitoring the progression or regression of a disease or condition in a subject comprising:
   a) determining a first profile of one or more markers of the disease or condition from a first sample comprising two or more different components selected from the group consisting of: a cell-free bodily fluid isolated from the subject at a first time point, a population of phagocytic cells isolated from the subject at a first time point, a population of phagocytic cells having a DNA content more than 2n (>2n phagocytic cells) isolated from the subject at a first time point, a population of circulating vesicles isolated from the subject at a first time point, and a population of circulating diseased cells isolated from the subject at a first time point;
      determining a second profile of at least one of the one or more markers from a first control comprising a component selected from the group consisting of: a population of phagocytic cells having a DNA content of 2n (=2n phagocytic cells) isolated from the subject at a first time point, a population of non-phagocytic cells isolated from the subject at a first time point, and a population of control cells isolated from the subject, wherein the control cells are substantially free of cells affected by the disease or condition, at a first time point;
      identifying a difference between the first and second profiles;
   b) determining a third profile of one or more markers of the disease or condition from a second sample comprising two or more different components selected from the group consisting of: a cell-free bodily fluid isolated from the subject at a second time point, a population of phagocytic cells isolated from the subject at a second time point, a population of phagocytic cells having a DNA content more than 2n (>2n phagocytic cells) isolated from the subject at a second time point, a population of circulating vesicles isolated from the subject at a second time point, and a population of circulating diseased cells isolated from the subject at a second time point;
      determining a fourth profile of at least one of the one or more markers from a second control comprising a component selected from the group consisting of: a population of phagocytic cells having a DNA content of 2n (=2n phagocytic cells) isolated from the subject at a second time point, a population of non-phagocytic cells isolated from the subject at a second time point, and a population of control cells isolated from the subject, wherein the control cells are substantially free of cells affected by the disease or condition, at a second time point;
      identifying a difference between the third and fourth profiles; and
   c) identifying a difference between the difference identified in a) and the difference identified in b) wherein the identified difference in c) is indicative of the progression or regression of said disease or condition in the subject.
6. A method for identifying a compound capable of ameliorating or treating a disease or condition in a subject comprising:
   a) determining a first profile of one or more markers of the disease or condition from a first sample comprising two or more different components selected from the group consisting of: a cell-free bodily fluid isolated from the subject before administering the compound to the subject, a population of phagocytic cells isolated from the subject before administering the compound to the subject, a population of phagocytic cells having a DNA content more than 2n (>2n phagocytic cells) isolated from the subject before administering the compound to the subject, a population of circulating vesicles isolated from the subject before administering the compound to the subject, and a population of circulating diseased cells isolated from the subject before administering the compound to the subject;
      determining a second profile of at least one of the one or more markers from a first control comprising a component selected from the group consisting of: a population of phagocytic cells having a DNA content of 2n (=2n phagocytic cells) isolated from the subject before administering the compound to the subject, a population of non-phagocytic cells isolated from the subject before administering the compound to the subject, and a population of control cells isolated from the subject, wherein the control cells are substantially free of cells affected by the disease or condition, before administering the compound to the subject;
      identifying a difference between the first and second profiles;
   b) determining a third profile of one or more markers of the disease or condition from a second sample comprising two or more different components selected from the group consisting of: a cell-free bodily fluid isolated from the subject after administering the compound to the subject, a population of phagocytic cells isolated from the subject after administering the compound to the subject, a population of phagocytic cells having a DNA content more than 2n (>2n phagocytic cells) isolated from the subject after administering the compound to the subject, a population of circulating vesicles isolated from the subject after administering the compound to the subject, and a population of circulating diseased cells isolated from the subject after administering the compound to the subject;
      determining a fourth profile of at least one of the one or more markers from a second control comprising a component selected from the group consisting of: a population of phagocytic cells having a DNA content of 2n (=2n phagocytic cells) isolated from the subject after administering the compound to the subject, a population of non-phagocytic cells isolated from the subject after administering the compound to the subject, and a population of control cells isolated from the subject, wherein the control cells are substantially free of cells affected by the disease or condition, after administering the compound to the subject;
      identifying a difference between the third and fourth profiles; and
   c) identifying a difference between the difference identified in a) and the difference identified in b) wherein the identified difference in c) indicates that the compound is capable of ameliorating or treating said disease or condition in the subject.
7. A method for assessing the efficacy of a treatment for a disease or condition in a subject comprising:
   a) determining a first profile of one or more markers of the disease or condition from a first sample comprising two or more different components selected from the group consisting of: a cell-free bodily fluid isolated from the subject before the treatment, a population of phagocytic cells isolated from the subject before the treatment, a population of phagocytic cells having a DNA content more than 2n (>2n phagocytic cells) isolated from the subject before the treatment, a population of circulating vesicles isolated from the subject before the treatment, and a population of circulating diseased cells isolated from the subject before the treatment;
   b) determining a second profile of one or more markers of the disease or condition from a second sample comprising two or more different components selected from the group consisting of: a cell-free bodily fluid isolated from the subject after the treatment, a population of phagocytic cells isolated from the subject after the treatment, a population of phagocytic cells having a DNA content more than 2n (>2n phagocytic cells) isolated from the subject after the treatment, a population of circulating vesicles isolated from the subject after the treatment, and a population of circulating diseased cells isolated from the subject; and
   c) identifying a difference between the first profile and the second profile, wherein the identified difference is indicative of the efficacy of the treatment for said disease or condition in the subject.
8. A method for monitoring the progression or regression of a disease or condition in a subject comprising:
   a) determining a first profile of one or more markers of the disease or condition from a first sample comprising two or more different components selected from the group consisting of: a cell-free bodily fluid isolated from the subject at a first time point, a population of phagocytic cells isolated from the subject at a first time point, a population of phagocytic cells having a DNA content more than 2n (>2n phagocytic cells) isolated from the subject at a first time point, a population of circulating vesicles isolated from the subject at a first time point, and a population of circulating diseased cells isolated from the subject at a first time point;
   b) determining a second profile of one or more markers of the disease or condition from a second sample comprising two or more different components selected from the group consisting of: a cell-free bodily fluid isolated from the subject at a second time point, a population of phagocytic cells isolated from the subject at a second time point, a population of phagocytic cells having a DNA content more than 2n (>2n phagocytic cells) isolated from the subject at a second time point, a population of circulating vesicles isolated from the subject at a second time point, and a population of circulating diseased cells isolated from the subject at a second time point; and
   c) identifying a difference between the first profile and the second profile, wherein the identified difference is indicative of the progression or regression of said disease or condition in the subject.
9. A method for identifying a compound capable of ameliorating or treating a disease or condition in a subject comprising:
   a) determining a first profile of one or more markers of the disease or condition from a first sample comprising two or more different components selected from the group consisting of: a cell-free bodily fluid isolated from the subject before administering the compound to the subject, a population of phagocytic cells isolated from the subject before administering the compound to the subject, a population of phagocytic cells having a DNA content more than 2n (>2n phagocytic cells) isolated from the subject before administering the compound to the subject, a population of circulating vesicles isolated from the subject before administering the compound to the subject, and a population of circulating diseased cells isolated from the subject before administering the compound to the subject;
   b) determining a second profile of one or more markers of the disease or condition from a second sample comprising two or more different components selected from the group consisting of: a cell-free bodily fluid isolated from the subject after administering the compound to the subject, a population of phagocytic cells isolated from the subject after administering the compound to the subject, a population of phagocytic cells having a DNA content more than 2n (>2n phagocytic cells) isolated from the subject after administering the compound to the subject, a population of circulating vesicles isolated from the subject after administering the compound to the subject, and a population of circulating diseased cells isolated from the subject after administering the compound to the subject; and
   c) identifying a difference between the first profile and the second profile, wherein the identified difference indicates that the compound is capable of ameliorating or treating said disease or condition in the subject.
10. A method for diagnosing or aiding in the diagnosis of a disease or condition in a subject comprising:
   a) determining a first profile of one or more markers of the disease or condition from a sample comprising components selected from the group consisting of: an analyte isolated from a cell-free bodily fluid isolated from the subject, an analyte isolated from a population of phagocytic cells isolated from the subject, an analyte isolated from a population of phagocytic cells having a DNA content more than 2n (>2n phagocytic cells) isolated from the subject, an analyte isolated from a population of circulating vesicles isolated from the subject, and an analyte isolated from a population of circulating diseased cells isolated from the subject;
   b) determining a second profile of at least one of the one or more markers from a control comprising a component selected from the group consisting of: an analyte isolated from a population of phagocytic cells having a DNA content of 2n (=2n phagocytic cells) isolated from the subject, an analyte isolated from a population of non-phagocytic cells isolated from the subject, and an analyte isolated from a population of control cells isolated from the subject, wherein the control cells are substantially free of cells affected by the disease or condition; and
   c) identifying a difference between the first and second profiles, wherein the difference is indicative of the presence of said disease or condition in the subject.
11. A method for assessing the risk of developing a disease or condition in a subject comprising:
   a) determining a first profile of one or more markers of the disease or condition from a sample comprising two or more different components selected from the group consisting of: an analyte isolated from a cell-free bodily fluid isolated from the subject, an analyte isolated from a population of phagocytic cells isolated from the subject, an analyte isolated from a population of phagocytic cells having a DNA content more than 2n (>2n phagocytic cells) isolated from the subject, an analyte isolated from a population of circulating vesicles isolated from the subject, and an analyte isolated from a population of circulating diseased cells isolated from the subject;
   b) determining a second profile of at least one of the one or more markers from a control comprising a component selected from the group consisting of: an analyte isolated from a population of phagocytic cells having a DNA content of 2n (=2n phagocytic cells) isolated from the subject, an analyte isolated from a population of non-phagocytic cells isolated from the subject, and an analyte isolated from a population of control cells isolated from the subject, wherein the control cells are substantially free of cells affected by the disease or condition; and
   c) identifying a difference between the first and second profiles, wherein the difference is indicative of the risk of developing said disease or condition in the subject.
12. A method for prognosing or aiding in the prognosis of a disease or condition in a subject comprising:
   a) determining a first profile of one or more markers of the disease or condition from a sample comprising two or more different components selected from the group consisting of: an analyte isolated from a cell-free bodily fluid isolated from the subject, an analyte isolated from a population of phagocytic cells isolated from the subject, an analyte isolated from a population of phagocytic cells having a DNA content more than 2n (>2n phagocytic cells) isolated from the subject, an analyte isolated from a population of circulating vesicles isolated from the subject, and an analyte isolated from a population of circulating diseased cells isolated from the subject;
   b) determining a second profile of at least one of the one or more markers from a control comprising a component selected from the group consisting of: an analyte isolated from a population of phagocytic cells having a DNA content of 2n (=2n phagocytic cells) isolated from the subject, an analyte isolated from a population of non-phagocytic cells isolated from the subject, and an analyte isolated from a population of control cells isolated from the subject, wherein the control cells are substantially free of cells affected by the disease or condition; and
   c) identifying a difference between the first and second profiles, wherein the difference is indicative of the prognosis of said disease or condition in the subject.
13. A method for assessing the efficacy of a treatment for a disease or condition in a subject comprising:
   a) determining a first profile of one or more markers of the disease or condition from a first sample comprising two or more different components selected from the group consisting of: an analyte isolated from a cell-free bodily fluid isolated from the subject before the treatment, an analyte isolated from a population of phagocytic cells isolated from the subject before the treatment, an analyte isolated from a population of phagocytic cells having a DNA content more than 2n (>2n phagocytic cells) isolated from the subject before the treatment, an analyte isolated from a population of circulating vesicles isolated from the subject before the treatment, and an analyte isolated from a population of circulating diseased cells isolated from the subject before the treatment;
      determining a second profile of at least one of the one or more markers from a first control comprising a component selected from the group consisting of: an analyte isolated from a population of phagocytic cells having a DNA content of 2n (=2n phagocytic cells) isolated from the subject before the treatment, an analyte isolated from a population of non-phagocytic cells isolated from the subject before the treatment, and an analyte isolated from a population of control cells isolated from the subject, wherein the control cells are substantially free of cells affected by the disease or condition, before the treatment;
      identifying a difference between the first and second profiles;
   b) determining a third profile of one or more markers of the disease or condition from a second sample comprising two or more different components selected from the group consisting of: an analyte isolated from a cell-free bodily fluid isolated from the subject after the treatment, an analyte isolated from a population of phagocytic cells isolated from the subject after the treatment, an analyte isolated from a population of phagocytic cells having a DNA content more than 2n (>2n phagocytic cells) isolated from the subject after the treatment, an analyte isolated from a population of circulating vesicles isolated from the subject after the treatment, and an analyte isolated from a population of circulating diseased cells isolated from the subject after the treatment;
      determining a fourth profile of at least one of the one or more markers from a second control comprising a component selected from the group consisting of: an analyte isolated from a population of phagocytic cells having a DNA content of 2n (=2n phagocytic cells) isolated from the subject after the treatment, an analyte isolated from a population of non-phagocytic cells isolated from the subject after the treatment, and an analyte isolated from a population of control cells isolated from the subject, wherein the control cells are substantially free of cells affected by the disease or condition, after the treatment;
      identifying a difference between the third and fourth profiles; and
   c) identifying a difference between the difference identified in a) and the difference identified in b) wherein the identified difference in c) is indicative of the efficacy of the treatment for said disease or condition in the subject.
14. A method for monitoring the progression or regression of a disease or condition in a subject comprising:
   a) determining a first profile of one or more markers of the disease or condition from a first sample comprising two or more different components selected from the group consisting of: an analyte isolated from a cell-free bodily fluid isolated from the subject at a first time point, an analyte isolated from a population of phagocytic cells isolated from the subject at a first time point, an analyte isolated from a population of phagocytic cells having a DNA content more than 2n (>2n phagocytic cells) isolated from the subject at a first time point, an analyte isolated from a population of circulating vesicles isolated from the subject at a first time point, and an analyte isolated from a population of circulating diseased cells isolated from the subject at a first time point;
      determining a second profile of at least one of the one or more markers from a first control comprising a component selected from the group consisting of: an analyte isolated from a population of phagocytic cells having a DNA content of 2n (=2n phagocytic cells) isolated from the subject at a first time point, an analyte isolated from a population of non-phagocytic cells isolated from the subject at a first time point, and an analyte isolated from a population of control cells isolated from the subject, wherein the control cells are substantially free of cells affected by the disease or condition, at a first time point;
      identifying a difference between the first and second profiles;
   b) determining a third profile of one or more markers of the disease or condition from a second sample comprising two or more different components selected from the group consisting of: an analyte isolated from a cell-free bodily fluid isolated from the subject at a second time point, an analyte isolated from a population of phagocytic cells isolated from the subject at a second time point, an analyte isolated from a population of phagocytic cells having a DNA content more than 2n (>2n phagocytic cells) isolated from the subject at a second time point, an analyte isolated from a population of circulating vesicles isolated from the subject at a second time point, and an analyte isolated from a population of circulating diseased cells isolated from the subject at a second time point;
      determining a fourth profile of at least one of the one or more markers from a second control comprising a component selected from the group consisting of: an analyte isolated from a population of phagocytic cells having a DNA content of 2n (=2n phagocytic cells) isolated from the subject at a second time point, an analyte isolated from a population of non-phagocytic cells isolated from the subject at a second time point, and an analyte isolated from a population of control cells isolated from the subject, wherein the control cells are substantially free of cells affected by the disease or condition, at a second time point;
      identifying a difference between the third and fourth profiles; and
   c) identifying a difference between the difference identified in a) and the difference identified in b) wherein the identified difference in c) is indicative of the progression or regression of said disease or condition in the subject.
15. A method for identifying a compound capable of ameliorating or treating a disease or condition in a subject comprising:
   a) determining a first profile of one or more markers of the disease or condition from a first sample comprising two or more different components selected from the group consisting of: an analyte isolated from a cell-free bodily fluid isolated from the subject before administering the compound to the subject, an analyte isolated from a population of phagocytic cells isolated from the subject before administering the compound to the subject, an analyte isolated from a population of phagocytic cells having a DNA content more than 2n (>2n phagocytic cells) isolated from the subject before administering the compound to the subject, an analyte isolated from a population of circulating vesicles isolated from the subject before administering the compound to the subject, and an analyte isolated from a population of circulating diseased cells isolated from the subject before administering the compound to the subject;
      determining a second profile of at least one of the one or more markers from a first control comprising a component selected from the group consisting of: an analyte isolated from a population of phagocytic cells having a DNA content of 2n (=2n phagocytic cells) isolated from the subject before administering the compound to the subject, an analyte isolated from a population of non-phagocytic cells isolated from the subject before administering the compound to the subject, and an analyte isolated from a population of control cells isolated from the subject, wherein the control cells are substantially free of cells affected by the disease or condition, before administering the compound to the subject;
      identifying a difference between the first and second profiles;
   b) determining a third profile of one or more markers of the disease or condition from a second sample comprising two or more different components selected from the group consisting of: an analyte isolated from a cell-free bodily fluid isolated from the subject after administering the compound to the subject, an analyte isolated from a population of phagocytic cells isolated from the subject after administering the compound to the subject, an analyte isolated from a population of phagocytic cells having a DNA content more than 2n (>2n phagocytic cells) isolated from the subject after administering the compound to the subject, an analyte isolated from a population of circulating vesicles isolated from the subject after administering the compound to the subject, and an analyte isolated from a population of circulating diseased cells isolated from the subject after administering the compound to the subject;
      determining a fourth profile of at least one of the one or more markers from a second control comprising a component selected from the group consisting of: an analyte isolated from a population of phagocytic cells having a DNA content of 2n (=2n phagocytic cells) isolated from the subject after administering the compound to the subject, an analyte isolated from a population of non-phagocytic cells isolated from the subject after administering the compound to the subject, and an analyte isolated from a population of control cells isolated from the subject, wherein the control cells are substantially free of cells affected by the disease or condition, after administering the compound to the subject;
      identifying a difference between the third and fourth profiles; and
   c) identifying a difference between the difference identified in a) and the difference identified in b) wherein the identified difference in c) indicates that the compound is capable of ameliorating or treating said disease or condition in the subject.
16. A method for assessing the efficacy of a treatment for a disease or condition in a subject comprising:
   a) determining a first profile of one or more markers of the disease or condition from a first sample comprising two or more different components selected from the group consisting of: an analyte isolated from a cell-free bodily fluid isolated from the subject before the treatment, a population of phagocytic cells isolated from the subject before the treatment, an analyte isolated from a population of phagocytic cells having a DNA content more than 2n (>2n phagocytic cells) isolated from the subject before the treatment, an analyte isolated from a population of circulating vesicles isolated from the subject before the treatment, and an analyte isolated from a population of circulating diseased cells isolated from the subject before the treatment;
   b) determining a second profile of one or more markers of the disease or condition from a second sample comprising two or more different components selected from the group consisting of: an analyte isolated from a cell-free bodily fluid isolated from the subject after the treatment, an analyte isolated from a population of phagocytic cells isolated from the subject after the treatment, an analyte isolated from a population of phagocytic cells having a DNA content more than 2n (>2n phagocytic cells) isolated from the subject after the treatment, an analyte isolated from a population of circulating vesicles isolated from the subject after the treatment, and an analyte isolated from a population of control cells isolated from the subject, wherein the control cells are substantially free of cells affected by the disease or condition; and
   c) identifying a difference between the first profile and the second profile, wherein the identified difference is indicative of the efficacy of the treatment for said disease or condition in the subject.
17. A method for monitoring the progression or regression of a disease or condition in a subject comprising:
   a) determining a first profile of one or more markers of the disease or condition from a first sample comprising two or more different components selected from the group consisting of: an analyte isolated from a cell-free bodily fluid isolated from the subject at a first time point, an analyte isolated from a population of phagocytic cells isolated from the subject at a first time point, an analyte isolated from a population of phagocytic cells having a DNA content more than 2n (>2n phagocytic cells) isolated from the subject at a first time point, an analyte isolated from a population of circulating vesicles isolated from the subject at a first time point, and an analyte isolated from a population of circulating diseased cells isolated from the subject at a first time point;
   b) determining a second profile of one or more markers of the disease or condition from a second sample comprising two or more different components selected from the group consisting of: an analyte isolated from a cell-free bodily fluid isolated from the subject at a second time point, an analyte isolated from a population of phagocytic cells isolated from the subject at a second time point, an analyte isolated from a population of phagocytic cells having a DNA content more than 2n (>2n phagocytic cells) isolated from the subject at a second time point, an analyte isolated from a population of circulating vesicles isolated from the subject at a second time point, and an analyte isolated from a population of circulating diseased cells isolated from the subject at a second time point; and
   c) identifying a difference between the first profile and the second profile, wherein the identified difference is indicative of the progression or regression of said disease or condition in the subject.
18. A method for identifying a compound capable of ameliorating or treating a disease or condition in a subject comprising:
   a) determining a first profile of one or more markers of the disease or condition from a first sample comprising two or more different components selected from the group consisting of: an analyte isolated from a cell-free bodily fluid isolated from the subject before administering the compound to the subject, an analyte isolated from a population of phagocytic cells isolated from the subject before administering the compound to the subject, an analyte isolated from a population of phagocytic cells having a DNA content more than 2n (>2n phagocytic cells) isolated from the subject before administering the compound to the subject, an analyte isolated from a population of circulating vesicles isolated from the subject before administering the compound to the subject, and an analyte isolated from a population of circulating diseased cells isolated from the subject before administering the compound to the subject;
   b) determining a second profile of one or more markers of the disease or condition from a second sample comprising two or more different components selected from the group consisting of: an analyte isolated from a cell-free bodily fluid isolated from the subject after administering the compound to the subject, an analyte isolated from a population of phagocytic cells isolated from the subject after administering the compound to the subject, an analyte isolated from a population of phagocytic cells having a DNA content more than 2n (>2n phagocytic cells) isolated from the subject after administering the compound to the subject, an analyte isolated from a population of circulating vesicles isolated from the subject after administering the compound to the subject, and an analyte isolated from a population of circulating diseased cells isolated from the subject after administering the compound to the subject; and
   c) identifying a difference between the first profile and the second profile, wherein the identified difference indicates that the compound is capable of ameliorating or treating said disease or condition in the subject.
19. A method for diagnosing or aiding in the diagnosis of a disease or condition in a subject comprising:
   a) determining a first profile of one or more markers of the disease or condition from a sample comprising two or more different components selected from the group consisting of: a cell-free bodily fluid isolated from the subject, a population of phagocytic cells isolated from the subject, a population of phagocytic cells having a DNA content more than 2n (>2n phagocytic cells) isolated from the subject, a population of circulating vesicles isolated from the subject, and a population of circulating diseased cells isolated from the subject; and
   b) identifying a difference between the first profile and a second profile of at least one of the one or more markers from a repository of said markers of said disease or condition, wherein the difference is indicative of the presence of said disease or condition in the subject.
20. A method for assessing the risk of developing a disease or condition in a subject comprising:
   a) determining a first profile of one or more markers of the disease or condition from a sample comprising two or more different components selected from the group consisting of: a cell-free bodily fluid isolated from the subject, a population of phagocytic cells isolated from the subject, a population of phagocytic cells having a DNA content more than 2n (>2n phagocytic cells) isolated from the subject, a population of circulating vesicles isolated from the subject, and a population of circulating diseased cells isolated from the subject; and
   b) identifying a difference between the first profile and a second profile of at least one of the one or more markers from a repository of said markers of said disease or condition , wherein the difference is indicative of the risk of developing said disease or condition in the subject.
21. A method for prognosing or aiding in the prognosis of a disease or condition in a subject comprising:
   a) determining a first profile of one or more markers of the disease or condition from a sample comprising two or more different components selected from the group consisting of: a cell-free bodily fluid isolated from the subject, a population of phagocytic cells isolated from the subject, a population of phagocytic cells having a DNA content more than 2n (>2n phagocytic cells) isolated from the subject, a population of circulating vesicles isolated from the subject, and a population of circulating diseased cells isolated from the subject; and
   b) identifying a difference between the first profile and a second profile of at least one of the one or more markers from a repository of said markers of said disease or condition, wherein the difference is indicative of the prognosis of said disease or condition in the subject.
22. A method for diagnosing or aiding in the diagnosis of a disease or condition in a subject comprising:
   a) determining a first profile of one or more markers of the disease or condition from a sample comprising components selected from the group consisting of: an analyte isolated from a cell-free bodily fluid isolated from the subject, an analyte isolated from a population of phagocytic cells isolated from the subject, an analyte isolated from a population of phagocytic cells having a DNA content more than 2n (>2n phagocytic cells) isolated from the subject, an analyte isolated from a population of circulating vesicles isolated from the subject, and an analyte isolated from a population of circulating diseased cells isolated from the subject; and
   b) identifying a difference between the first profile and a second profile of at least one of the one or more markers from a repository of said markers of said disease or condition , wherein the difference is indicative of the presence of said disease or condition in the subject.
23. A method for assessing the risk of developing a disease or condition in a subject comprising:
   a) determining a first profile of one or more markers of the disease or condition from a sample comprising two or more different components selected from the group consisting of: an analyte isolated from a cell-free bodily fluid isolated from the subject, an analyte isolated from a population of phagocytic cells isolated from the subject, an analyte isolated from a population of phagocytic cells having a DNA content more than 2n (>2n phagocytic cells) isolated from the subject, an analyte isolated from a population of circulating vesicles isolated from the subject, and an analyte isolated from a population of circulating diseased cells isolated from the subject; and
   b) identifying a difference between the first profile and a second profile of at least one of the one or more markers from a repository of said markers of said disease or condition , wherein the difference is indicative of the risk of developing said disease or condition in the subject.
24. A method for prognosing or aiding in the prognosis of a disease or condition in a subject comprising:
   a) determining a first profile of one or more markers of the disease or condition from a sample comprising two or more different components selected from the group consisting of: an analyte isolated from a cell-free bodily fluid isolated from the subject, an analyte isolated from a population of phagocytic cells isolated from the subject, an analyte isolated from a population of phagocytic cells having a DNA content more than 2n (>2n phagocytic cells) isolated from the subject, an analyte isolated from a population of circulating vesicles isolated from the subject, and a population of circulating diseased cells isolated from the subject; and
   b) identifying a difference between the first profile and a second profile of at least one of the one or more markers from a repository of said markers of said disease or condition , wherein the difference is indicative of the prognosis of said disease or condition in the subject.
25. The method of any one of items 1-3 and 10-12, wherein at least one of the one or more markers is up-regulated or activated in the sample compared to the control.
26. The method of any one of items 1-3 and 10-12, wherein at least one of the one or more markers is down-regulated or inhibited in the sample compared to the control.
27. The method of any one of items 4-6 and 13-15, wherein at least one of the one or more markers is up-regulated or activated in the first sample compared to the first control or in the second sample compared to the second control.
28. The method of any one of items 4-6 and 13-15, wherein at least one of the one or more markers is down-regulated or inhibited in the first sample compared to the first control or in the second sample compared to the second control.
29. The method of any one of items 7-9 and 16-18, wherein at least one of the one or more markers is up-regulated or activated in the first sample compared to the second sample.
30. The method of any one of items 7-9 and 16-18, wherein at least one of the one or more markers is down-regulated or inhibited in the first sample compared to the second sample.
31. The method of any one of items 19-24, wherein at least one of the one or more markers is up-regulated or activated in the sample compared to the repository.
32. The method of any one of items 19-24, wherein at least one of the one or more markers is down-regulated or inhibited in the sample compared to the repository.
33. The method of any one of items 1-32, wherein the first profile or the second profile comprises the absence of at least one of the one or more markers of said disease or condition.
34. The method of any one of items 4-6 and 13-15, wherein the third profile or the fourth profile comprises the absence of at least one of the one or more markers of said disease or condition.
35. The method of any one of items 1-9 and 19-21, wherein, when said method comprises circulating diseased cells, control cells not affected by the disease or condition, phagocytic cells, >2n phagocytic cells, =2n phagocytic cells, or non-phagocytic cells, the method further comprises lysing the circulating diseased cells, control cells not affected by the disease or condition, phagocytic cells, >2n phagocytic cells, =2n phagocytic cells, or non-phagocytic cells.
36. The method of any one of items 1-9, 19-21, and 35 wherein, when said method comprises circulating diseased cells, control cells not affected by the disease or condition, phagocytic cells, >2n phagocytic cells, =2n phagocytic cells, or non-phagocytic cells, the method further comprises extracting at least some of the cellular contents from the circulating diseased cells, control cells not affected by the disease or condition, phagocytic cells, >2n phagocytic cells, =2n phagocytic cells, or non-phagocytic cells.
37. The method of any one of items 1-9 and 19-21, 35, and 36, wherein, when said method comprises the cell-free bodily fluid, the method further comprises extracting the one or more markers from the cell-free bodily fluid.
38. The method of any one of items 1-9 and 19-21, wherein, when said method comprises the cell-free bodily fluid, the cell-free bodily fluid comprises a transrenal nucleic acid.
39. The method of any one of items 1-38, wherein at least one of the one or more markers of said disease or condition is present in the circulating diseased cells, cell-free bodily fluid sample, phagocytic cells, or >2n phagocytic cells.
40. The method of any one of items 1-39, wherein at least one of the one or more markers of said disease or condition is not present in the circulating diseased cells, cell-free bodily fluid sample, phagocytic cells, or >2n phagocytic cells.
41. The method of any one of items 1-40, wherein one or more of the circulating diseased cells, control cells not affected by the disease or condition, phagocytic cells, >2n phagocytic cells, =2n phagocytic cells, or non-phagocytic cells are enucleated.
42. The method of item 41, wherein the cells are enucleated using physical removal, chemical treatments, photoablation, or ultraviolet irradiation.
43. The method of item 42, wherein the physical removal uses a microneedle, optical tweezer or aspiration.
44. The method of any one of items 19-24, wherein the repository is obtained by data mining.
45. The method of any one of items 1-44, wherein the phagocytic cells, >2n phagocytic cells, or =2n phagocytic cells are neutrophils, macrophages, monocytes, dendritic cells, foam cells, mast cells, eosinophils, keratinocytes, or mixtures thereof.
46. The method of any one of items 1-45, wherein the non-phagocytic cells are T cells, B cells, null cells, basophils, or mixtures thereof.
47. The method of any one of items 1-46, wherein the circulating diseased cells are blood cells, tumor cells, lymphoma cells, fetal cells, apoptotic cells, epithelia cells, endothelial cells, stem cells, progenitor cells, mesenchymal cells, osteoblast cells, osteocytes, hematopoietic stem cells, foam cells, adipose cells, transcervical cells, circulating cardiocytes, circulating fibrocytes, circulating myocytes, circulating cells from kidney, circulating cells from gastrointestinal tract, circulating cells from lung, circulating cells from reproductive organs, circulating cells from central nervous system, circulating hepatic cells, circulating cells from spleen, circulating cells from thymus, circulating cells from thyroid , circulating cells from an endocrine gland, circulating cells from parathyroid, circulating cells from pituitary, circulating cells from adrenal gland, circulating cells from islets of Langerhans, circulating cells from pancreas, circulating cells from hypothalamus, circulating cells from prostate tissues, circulating cells from breast tissues, circulating cells from circulating retinal cells, circulating ophthalmic cells, circulating auditory cells, circulating epidermal cells, circulating cells from the urinary tract, or mixtures thereof.
48. The method of any one of items 1-47, wherein the control cells are normal cells.
49. The method of any one of items 1-48, wherein the control cells are circulating cells.
50. The method of any one of items 1-49, wherein the circulating vesicles are selected from the group consisting of circulating microvesicles, apoptotic bodies, micro-particles, membrane-bound vesicles, multivesicular bodies, nanovesicles, microparticles, and ARRDC-1 mediated microvesicles (ARMM).
51. The method of item 50, wherein the circulating microvesicles are exosomes or urinary exosomes.
52. The method of any one of items 1-51, wherein the cell-free bodily fluid is separated from a bodily fluid.
53. The method of item 52, wherein the bodily fluid is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid.
54. The method of any one of items 52 and 53, wherein the cell-free bodily fluid is separated by filtration, centrifugation, flow cytometry, fluorescence activated cell sorting, gradient-based centrifugation, elution, microfluidics, magnetic separation technique, fluorescent-magnetic separation technique, nanostructure, quantum dots, high throughput microscope-based platforms, or a combination thereof.
55. The method of any one of items 52-54, wherein the cell-free bodily fluid is separated by using a substance present in the sample.
56. The method of item 55, wherein the substance is a product of a marker of said disease or condition.
57. The method of any one of items 1-56, wherein the cell-free bodily fluid is plasma or serum.
58. The method of any one of items 1-57, wherein the phagocytic cells, >2n phagocytic cells, =2n phagocytic cells, or non-phagocytic cells are isolated from a bodily fluid, tissues, or cells of the subject.
59. The method of item 58, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid.
60. The method of item 58, wherein the cells are white blood cells.
61. The method of any one of items 58-60, wherein the phagocytic cells, >2n phagocytic cells, =2n phagocytic cells, or non-phagocytic cells are isolated using antibodies.
62. The method of any one of items 58-60, wherein the phagocytic cells, >2n phagocytic cells, =2n phagocytic cells, or non-phagocytic cells are isolated by flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platforms, or a combination thereof.
63. The method of any one of items 1-62, wherein the one or more markers are nucleic acids, proteins, lipids, carbohydrates, metabolites, or combinations thereof.
64. The method of item 63, wherein the nucleic acids are nucleotides, oligonucleotides, DNAs, RNAs, or DNA-RNA hybrids.
65. The method of item 64, wherein the DNAs are double-stranded DNAs, single-stranded DNAs, multi-stranded DNAs, complementary DNAs, genomic DNAs, or non-coding DNAs.
66. The method of item 64, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs).
67. The method of item 63, wherein the proteins are amino acids, peptides, enzymes, antigens, antibodies, cytokines, lipoproteins, glycoproteins, or hormones.
68. The method of item 63, wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, low density lipoproteins (LDLs), very low density lipoproteins (VLDLs), high density lipoproteins (HDLs), sphingoid base-1-phosphates, or derivatives thereof.
69. The method of item 63, wherein the carbohydrates are monosaccharides, disaccharides, polysaccharides, oligosaccharides, or derivatives thereof.
70. The method of item 63, wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof.
71. The method of any one of items 1-3, 10-12, and 19-24, wherein the profile is a nucleic acid profile, a protein profile, a lipid profile, a carbohydrate profile, a metabolite profile, or a combination thereof.
72. The method of item 65, wherein the profile is determined by a qualitative assay, a quantitative assay, or a combination thereof.
73. The method of any one of items 4-9 and 13-18, wherein the first profile or the second profile is a nucleic acid profile, a protein profile, a lipid profile, a carbohydrate profile, a metabolite profile, or a combination thereof.
74. The method of item 65, wherein the first profile or the second profile is determined by a qualitative assay, a quantitative assay, or a combination thereof.
75. The method of any one of items 4-6 and 74, wherein the third profile or the fourth profile is a nucleic acid profile, a protein profile, a lipid profile, a carbohydrate profile, a metabolite profile, or a combination thereof.
76. The method of item 75, wherein the third profile or the fourth profile is determined by a qualitative assay, a quantitative assay, or a combination thereof.
77. The method of any one of items 72, 66, and 76, wherein the quantitative assay uses sequencing, targeted sequencing, single molecule real-time sequencing, electron microscopy-based sequencing, transistor-mediated sequencing, direct sequencing, random shotgun sequencing, Sanger dideoxy termination sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, multiplex PCR, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, co-amplification at lower denaturation temperature-PCR (COLD-PCR), multiplex PCR, quantitative PCR, real-time PCR, fluorescence assay, colorimetric assay, chemiluminescent assay, or a combination thereof.
78. The method of item 77, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof.
79. The method of item 77, wherein the nucleic acid profile is determined by polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, co-amplification at lower denaturation temperature-PCR (COLD-PCR), multiplex PCR, methyl-binding PCR analysis, or a combination thereof.
80. The method of item 77, wherein the nucleic acid profile is determined by a sequencing technique selected from the group consisting of targeted sequencing, single molecule real-time sequencing, exon sequencing, electron microscopy-based sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof.
81. The method of item 77, wherein the protein profile is a protein expression profile, a protein activation profile, or a combination thereof.
82. The method of item 77, wherein the protein profile is determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.
83. The method of item 77, wherein the protein activation profile comprises determining a phosphorylation state, an ubiquitination state, a myristoylation state, a conformational state, or a combination thereof of the one or more markers.
84. The method of item 77, wherein the lipid profile is determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.
85. The method of item 77, wherein the carbohydrate profile is determined by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassay, microfluidic chip-based assay, detection of fluorescence, detection of chemiluminescence, or a combination thereof.
86. The method of any one of items 1-85, wherein the subject has at least two diseases or conditions.
87. The method of item 86, wherein the subject has at least one prenatal or pregnancy-related disease or condition
88. The method of any one of items 1-87, wherein the subject is a mammal.
89. The method of item 89, wherein the mammal is a human.
90. The method of any one of items 1-89, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.
91. The method of any one of items 1-90, wherein the difference is greater than a 1-fold difference.
92. The method of item 91, wherein the difference is at least 1.05-fold, 1.1 -fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference.
93. The method of any one of items 4-6 and 13-15, wherein the second profile and the fourth profile are the same.
94. A method for identifying one or more markers for a disease or condition comprising:
   a) determining a first profile of analytes from a sample comprising a cell-free bodily fluid from a subject having said disease or condition and a population of phagocytic cells, or a population of >2n phagocytic cells, from a subject having said disease or condition;
      determining a second profile of analytes from a population of =2n phagocytic cells, or a population of non-phagocytic cells, from the subject having said disease or condition;
      identifying a set of differences between the first and second profiles, wherein the first set of differences is specific to the first profile relative to the second profile;
   b) determining a third profile of analytes from a sample comprising a cell-free bodily fluid from a subject having said disease or condition and a population of phagocytic cells, or a population of >2n phagocytic cells, from a control subject not having said disease or condition;
      determining a fourth profile of analytes from a population of =2n phagocytic cells, or a population of non-phagocytic cells, from the control subject not having said disease or condition;
      identifying a set of differences between the third and fourth profiles, wherein the second set of differences is specific to the third profile relative to the fourth profile; and
   c) identifying one or more analytes specific to the set of differences identified in a) relative to the set of differences identified in b), the identified analytes in c) being markers of said disease or condition.
95. A method for identifying one or more markers for a disease or condition comprising:
   a) determining a first profile of analytes from a sample comprising a cell-free bodily fluid from a subject having said disease or condition and a population of phagocytic cells, or a population of >2n phagocytic cells, from a subject having said disease or condition;
   b) comparing the first profile to a second profile derived from a repository of analytes from a control subject not having said disease or condition;
   c) identifying a set of differences between the first and second profiles, wherein the set of differences is specific to the first profile relative to the second profile; and
   d) identifying one or more analytes specific to the set of differences, the identified analytes being markers of said disease or condition.
96. The method of item 95, further comprising:
   a) obtaining a fifth profile of analytes from cells or tissues affected by said disease or condition in the subject having said disease or condition;
      obtaining a sixth profile of analytes from cells or tissues not affected by said disease or condition in the subject having said disease or condition;
      identifying a set of differences between the fifth and sixth profiles, wherein the set of differences is specific to the fifth profile relative to the sixth profile; and
   b) identifying at least one of the one or more markers of c) present in the set of differences identified in d).
97. The method of any one of items 90-96, further comprising extracting the one or more markers from the cell-free bodily fluid.
98. The method of any one of items 90-97, further comprising lysing the phagocytic cells, the >2n phagocytic cells, the =2n phagocytic cells, or the non-phagocytic cells before a).
99. The method of any one of items 90-98, further comprising extracting at least some of the cellular contents from the phagocytic cells, the >2n phagocytic cells, the =2n phagocytic cells, or the non-phagocytic cells before a).
100. The method of any one of items 90-99, wherein the phagocytic cells or the >2n phagocytic cells comprise viable diseased cells, dead diseased cells, apoptotic diseased cells, circulating tumor cells, infectious agents, fetal cells, trophoblasts, or fragments thereof.
101. The method of any one of items 90-100, wherein at least one of the one or more markers of said disease or condition is present in the cell-free bodily fluid sample, the phagocytic cells, or the >2n phagocytic cells.
102. The method of any one of items 90-101, wherein at least one of the one or more markers of said disease or condition is not present in the cell-free bodily fluid sample, the phagocytic cells, or the >2n phagocytic cells.
103. The method of any one of items 90-102, further comprising comparing the identified difference of c) to a repository of one or more known markers of said disease or condition.
104. The method of item 97, wherein the repository is obtained by data mining.
105. The method of any one of items 90-104, wherein the phagocytic cells, >2n phagocytic cells, or =2n phagocytic cells are neutrophils, macrophages, monocytes, dendritic cells, foam cells, mast cells, eosinophils, keratinocytes, or mixtures thereof.
106. The method of any one of the items 90-105, wherein the non-phagocytic cells are T cells, B cells, null cells, basophils, or mixtures thereof
107. The method of any one of items 90-106, wherein the cell-free bodily fluid is separated from a bodily fluid.
108. The method of item 107, wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, or ocular fluid.
109. The method of any one of items 107 and 108, wherein the cell-free bodily fluid sample is separated by filtration, centrifugation, flow cytometry, fluorescence activated cell sorting, gradient-based centrifugation, elution, microfluidics, magnetic separation technique, fluorescent-magnetic separation technique, nanostructure, quantum dots, high throughput microscope-based platforms, or a combination thereof.
110. The method of item 109, wherein the cell-free bodily fluid sample is separated by using a substance present in the sample.
111. The method of any one of items 90-110, wherein the one or more markers are nucleic acids, proteins, lipids, carbohydrates, metabolites, or combinations thereof.
112. The method of item 111, wherein the nucleic acids are nucleotides, oligonucleotides, DNAs, RNAs, or DNA-RNA hybrids.
113. The method of item 112, wherein the DNAs are double-stranded DNAs, single-stranded DNAs, multi-stranded DNAs, complementary DNAs, genomic DNAs, or non-coding DNAs.
114. The method of item 112, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs).
115. The method of item 111, wherein the proteins are amino acids, peptides, enzymes, antigens, antibodies, cytokines, lipoproteins, glycoproteins, or hormones.
116. The method of item 111, wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, low density lipoproteins (LDLs), very low density lipoproteins (VLDLs), high density lipoproteins (HDLs), sphingoid base-1-phosphates, or derivatives thereof.
117. The method of item 111, wherein the carbohydrates are monosaccharides, disaccharides, polysaccharides, oligosaccharides, or derivatives thereof.
118. The method of item 111, wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof.
119. The method of any one of items 90-118, wherein the profile is a nucleic acid profile, a protein profile, a lipid profile, a carbohydrate profile, a metabolite profile, or a combination thereof.
120. The method of item 119, wherein the profile is determined by a qualitative assay, a quantitative assay, or a combination thereof.
121. The method of item 120, wherein the quantitative assay uses sequencing, targeted sequencing, single molecule real-time sequencing, exon sequencing, electron microscopy-based sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.
122. The method of item 119, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof.
123. The method of item 119, wherein the nucleic acid profile is determined by polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, co-amplification at lower denaturation temperature-PCR (COLD-PCR), multiplex PCR, methyl-binding PCR analysis, or a combination thereof.
124. The method of item 119, wherein the nucleic acid profile is determined by a sequencing technique selected from the group consisting of targeted sequencing, single molecule real-time sequencing, exon sequencing, electron microscopy-based sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof.
125. The method of item 119, wherein the protein profile is a protein expression profile, a protein activation profile, or a combination thereof.
126. The method of item 119, wherein the protein profile is determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.
127. The method of item 119, wherein the protein activation profile comprises determining a phosphorylation state, an ubiquitination state, a myristoylation state, a conformational state, or a combination thereof of the one or more markers.
128. The method of item 119, wherein the lipid profile is determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.
129. The method of item 119, wherein the carbohydrate profile is determined by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassay, microfluidic chip-based assay, detection of fluorescence, detection of chemiluminescence, or a combination thereof.
130. The method of any one of items 90-129, wherein the subject is a mammal
131. The method of item 130, wherein the mammal is a human.
132. The method of any one of items 90-131, wherein the disease or condition is a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.
133. The method of any one of items 90-132, wherein the difference is greater than a 1-fold difference.
134. The method of item 133, wherein the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference.
135. The method of any one of items 1-93, further comprising determining at least one diagnostic parameter of said disease or condition.
136. The method of item 135, wherein the diagnostic parameter is determined by physical inspection, visual inspection, biopsy, scanning, histology, radiology, imaging, ultrasound, use of a commercial kit, genetic testing, immunological testing, analysis of bodily fluids, or monitoring neural activity.
137. The method of any one of the items 1-93 and 135-136, wherein the one or more markers comprise at least one gene selected from the group consisting of AKT2, BAK1, EGFR, ERBB2, ETS2, FOS, JUN, MAP2K1, MMP2, PDGFB, RB1, SERPINB2, SNCG, and SPP1.
138. The method of any one of the items 1-93 and 135-137, wherein the one or more markers comprise at least one gene selected from the group consisting of AKT1, AKT2, BAK2, CDC25A, E2F1, EGFR, ERBB2, FOS, JUN, MAP2K1, MMP2, NFKB1, PDGFB, PIK3R1, PNN, RBI, SERPINB2, SERPINB5, SNCG, SPP1, TERT, TIMP3, and TP53.
139. The method of any one of the items 1-93 and 135-138, wherein the one or more markers comprise at least one gene selected from the group consisting of CASP8, CASP9, COL18A1, ETS2, HTATIP2, MMP9, SRC, and TWIST1.
140. The method of any one of the items 1-93 and 135-139, wherein the one or more markers comprise at least one gene selected from the group consisting of AKT1, APAF1, ATM, CDC25A, CDKN1A, ETS2, FOS, IL8, ITGA4, ITGA6, ITGAV, JUN, MAP2K1, NFKBIA, PLAU, PLAUR, RAF1, SERPINB2, SYK, TIMP1, TNF, TNFRSF10B, and TNFRSF1A.
141. The method of any one of the items 1-93 and 135-140, wherein the one or more markers comprise at least one gene selected from the group consisting of ACP2, AK2, AKT3, ARL5B, ATP2B3, BGN, BRAF, BTG2, CAMKK2, CAPG, CAPN12, CPLX2, DENND5A, DNA2, FAM104A, FNIP1, GFRA4, GLUD1, GNAQ, GP1BB, HNRPLL, HOXA2, HPS3, INPP4A, ITGAV, KLHL23, LANCL2, LYPD6, MAPKAPK3, MEF2A (includes, EG:4205), MEF2C, NVL, PCYT1A, PGLYRP4, PLOD1, PPP1CB, PRKAB2, PROS1, PTPRE, RASA4 (includes,EG:10156), RBMS2, RBPJ, STAT5B, THBS1, TRIB1, TRIM2, TSPAN6, and ZDHHC21.
142. The method of any one of the items 1-93 and 135-141, wherein the one or more markers comprise at least one gene selected from the group consisting of B4GALT5, BOP1, CCL2, CCL3, CCL3L1, CCRL2, CD83, CLEC4G, CLIC4, CTSC, CTSO, CXCL10, FCGR3A, FPR3, HBA1, HBB, LRMP, MAP1LC3B2, MS4A4A, MSR1, MYADML, NID1, PF4, PION, RNF217, SAMD9L, SERPING1, and SPARC.
143. The method of any one of the items 1-93 and 135-142, wherein the one or more markers comprise at least one gene selected from the group consisting of ACOT9, AMPD2, ARHGAP15, BATF2, C3AR1, C5orf41, CCL3, CCL3L1, CD63, CHST11, CHSY1, CLEC4G, CTSZ, CXorf21, CYTH4, CYTIP, DLEU2, DNAJA1, DOCK8, DTX3L, DUSP6, EPSTI1, ERF, F2RL1, FYB, GABRB2, GBP5, GLRX, GNB4, ICAM1, IFI35, IFIH1, IFNAR2, IL1R1, IRF1, ITGA5, LAP3, LAPTM5, LCP2, MAP1LC3B, MAP1LC3B2, MICAL2, MT1DP, MT1JP, MT1M, MT2A, MYADML, NEK6, NINJ2, NNMT, NT5C3L, NUB1, PDE4B, PLOD1, PML, PRKCB, PSMB9, RCN3, RGS4, RNASE6, RTP4, SAMD9L, SEL1L, SERPING1, SETX, SIGLEC10, SKIL, SLC7A7, SNORA21, SP100, SP110, SP140, SSFA2, STAT2, STK17B, STK3, TDRD7, TMCC1, TMPRSS11E2, TNFRSF1B, TPM1, TRIM21,TXNDC4, UBE2L6, UBE2W, USP18, VAV1, WARS, WIPF1, and WIPI1.
144. The method of any one of the items 1-93 and 135-143, wherein the one or more markers comprise at least one gene selected from the group consisting of ADAR, ADM, ALAS1, ANKRD22, ARHGAP27, B3GNT5, BCL10, C12orf35, C15orf29, C2orf59, CD177, CEACAM1, CPEB2, DDX58, F2RL1, GDPD3, GNAI3, HIST2H3A, HIST2H3D, HIST2H4A, HMGCR, HSPA6, HSPC159, IL4R, IMPA2, KPNB1, KREMEN1, KRT23, LDLR, LOC100130904, LTB4R, MAEA, MARK2, MBOAT2, MPZL3, N4BP1, NBEAL2, NMI, NPEPPS, PARP14, PGM2, PPIF, PXN, RALBP1, ROD1, RPS6KA1, S100P, SERTAD2, SLC9A1, SLPI, SP110, SPINT1, ST14, TBC1D3, TNFRSF9, TRIM21, UPP1, VPS24, ZBTB34, and ZNF256.
145. The method of any one of the items 1-93 and 135-144, wherein the one or more markers comprise at least one or more of the markers identified by the methods of any one the items 94-134.
146. A kit comprising a plurality of marker detection agents that detect at least one or more of the markers identified by the methods of any one of the items 94-134.
147. A method of treating or preventing a disease or condition in a subject comprising administering to said subject a composition comprising a compound identified by the method of any one of items 6 and 15.
148. The method of any one of the items 1-93 and 135-145, wherein the circulating diseased cells are infected by an infectious agent.
149. The method of item 148, wherein the infectious agent is a virus, bacteria, fungus, parasite, protozoan, infectious protein or microorganism.
150. The method of any one of items 1-93, 135-145, and 148-149, wherein, when said method comprises phagocytic cells or >2n phagocytic cells, said phagocytic cells or >2n phagocytic cells comprise a transrenal nucleic acid.

### Brief Description of the Drawings

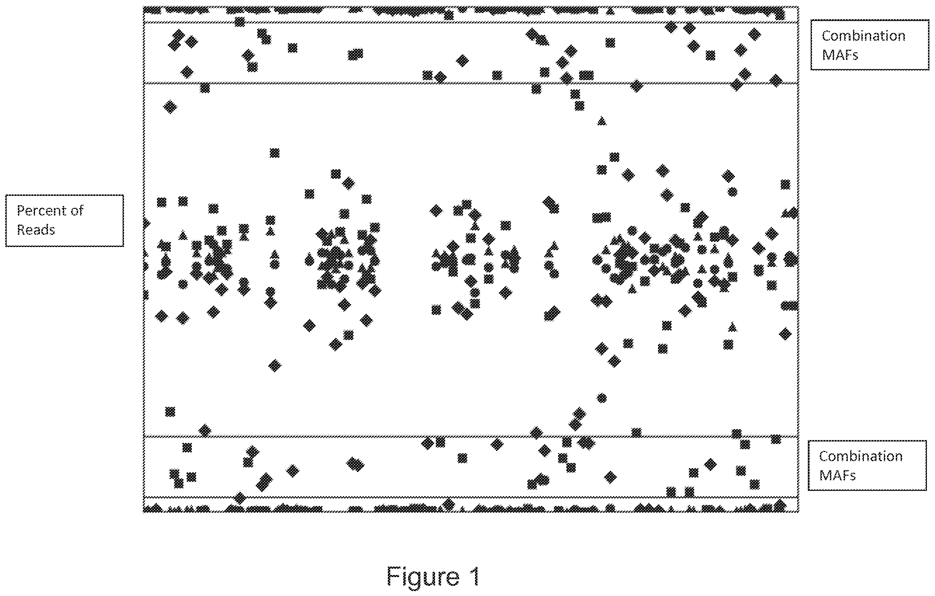

Figure 1 depicts a set of potentially informative markers and the relative percentage of reads of associated allelic frequencies in maternal reference samples of CD2-positive peripheral blood cells and combination samples. Along the x-axis are the markers and along the y-axis are the percent reads for the different alleles. Triangle: percent of maternal sample reference allele; circle: percent of maternal sample alternate allele; square: percent of combination sample reference allele; and diamond: percent of combination sample alternate allele.

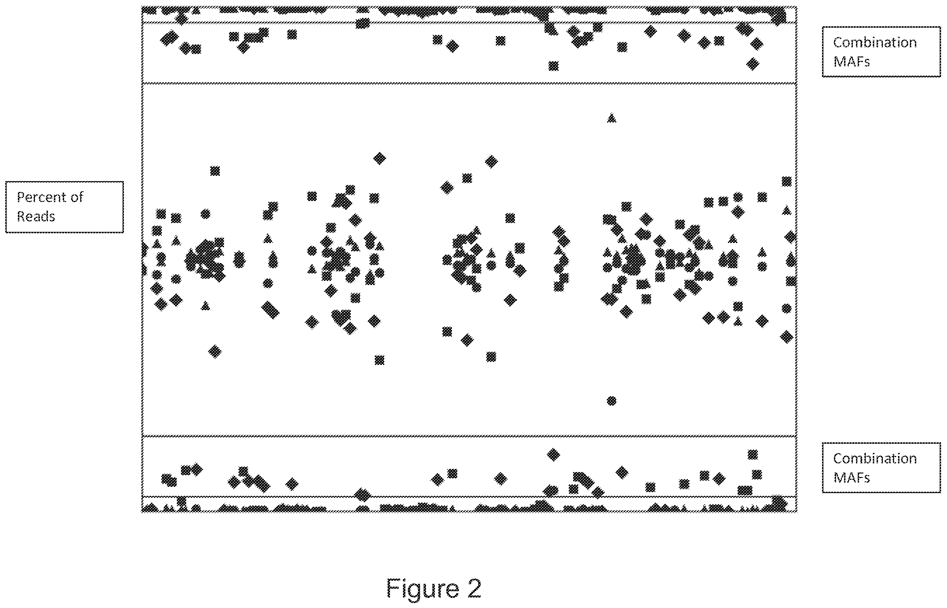

Figure 2 depicts a set of potentially informative markers and the relative percentage of reads of associated allelic frequencies in maternal reference samples and combination samples. Along the x-axis are the markers and along the y-axis are the percent reads for the different alleles. Triangle: percent of maternal sample reference allele; circle: percent of maternal sample alternate allele; square: percent of combination sample reference allele; and diamond: percent of combination sample alternate allele.

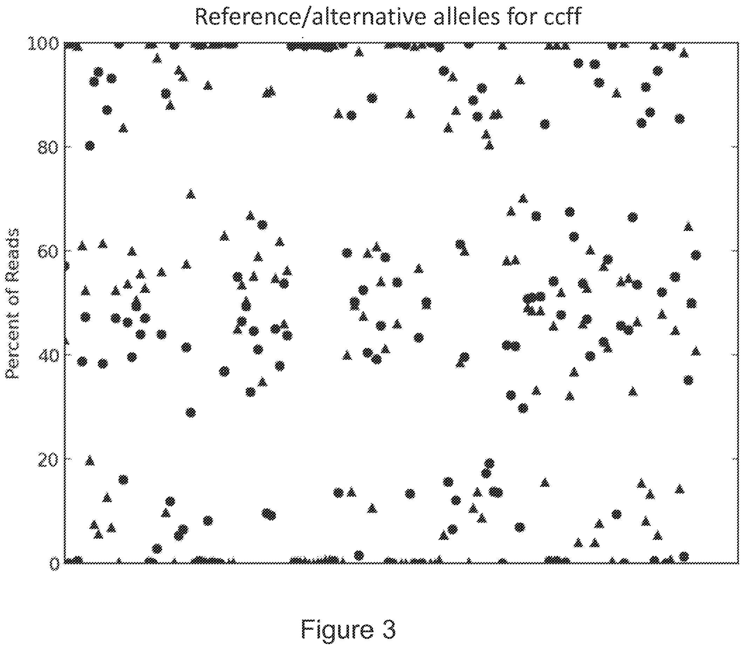

Figure 3 depicts a set of potentially informative markers and the relative percentage of reads of associated allelic frequencies as compared between a circulating cell-free fluid (ccff) sample and a maternal reference sample. Along the x-axis are the markers and along the y-axis are the percent reads for the different alleles. Triangle: reference allele; circle: alternative allele.

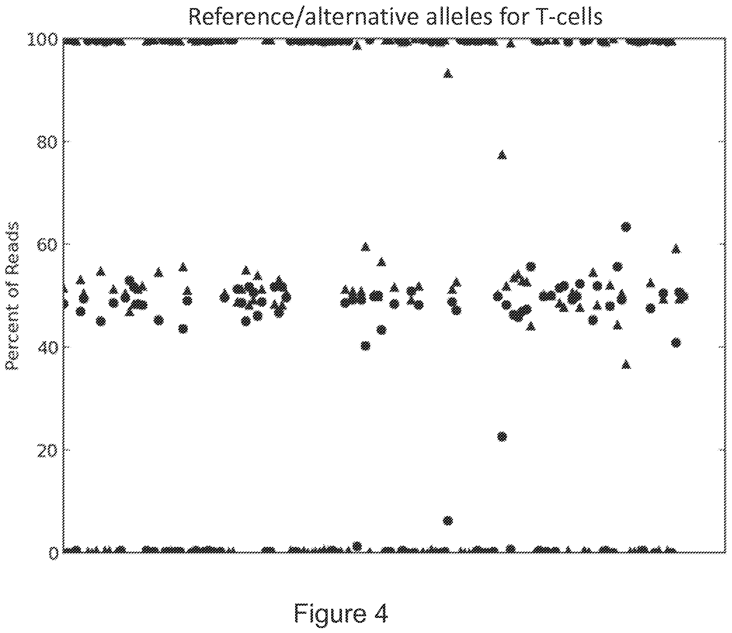

Figure 4 depicts a set of potentially informative markers and the relative percentage of reads of associated allelic frequencies in a T-cell sample. Along the x-axis are the markers and along the y-axis are the percent reads for the different alleles. Triangle: reference allele; circle: alternative allele.

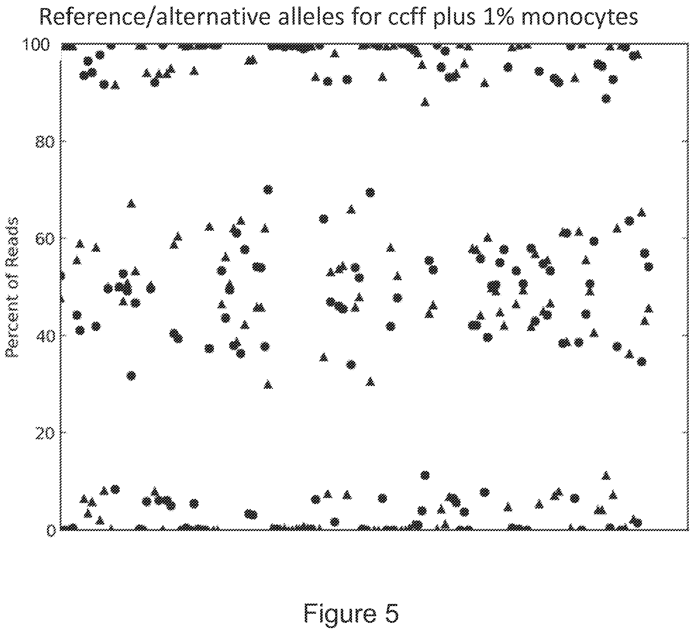

Figure 5 depicts a set of potentially informative markers and the relative percentage of reads of associated allelic frequencies in a combination sample of a circulating cell-free fluid (ccff) plus 1% monocyte sample. Along the x-axis are the markers and along the y-axis are the percent reads for the different alleles. Triangle: reference allele; circle: alternative allele.

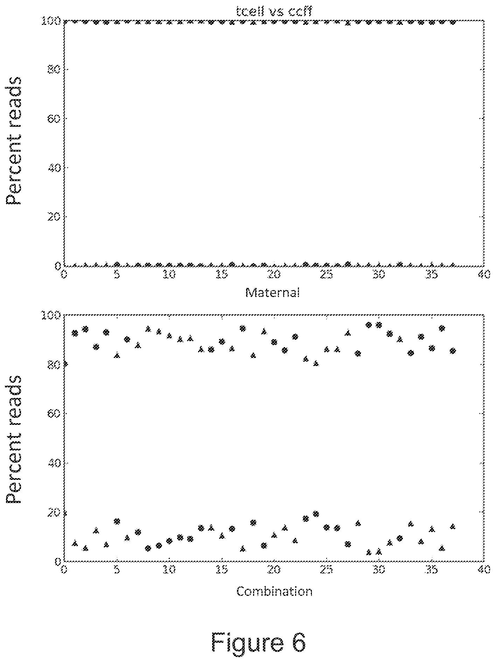

Figure 6 depicts the emergence of informative markers in a circulating cell-free fluid fraction of the maternal blood compared to the lack of informative markers in the maternal T-cells. Along the x-axis are the markers and along the y-axis are the percent reads for the different alleles.

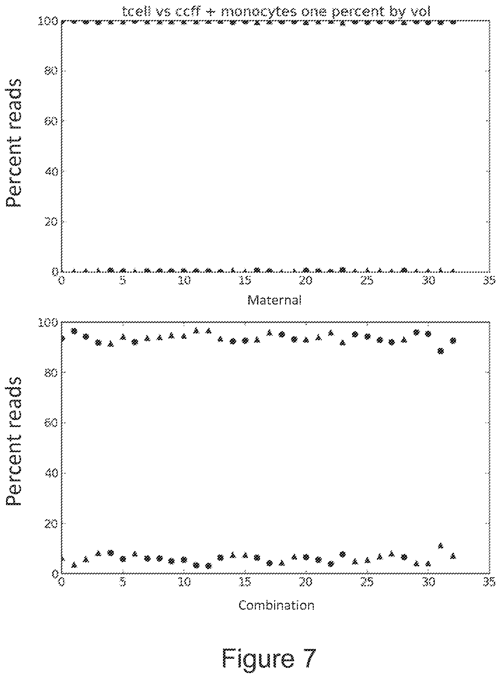

Figure 7 depicts the emergence of the informative markers in a combination sample containing cell-free bodily fluid plus monocytes from maternal blood compared to a lack of informative markers in the maternal reference sample containing T-cells. Along the x-axis are the markers and along the y-axis are the percent reads for the different alleles.

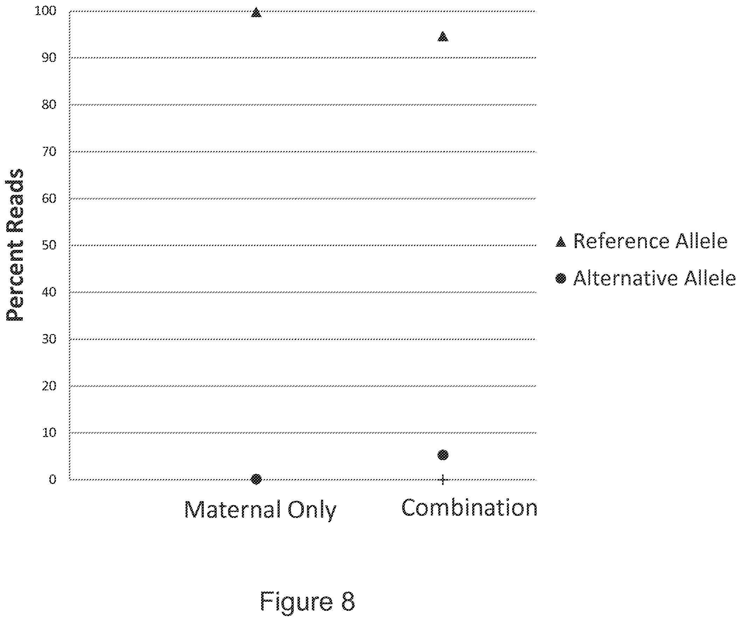

Figure 8 depicts an exemplary informative marker in a maternal only sample and a combination sample. Triangle: reference allele; circle: alternative allele.

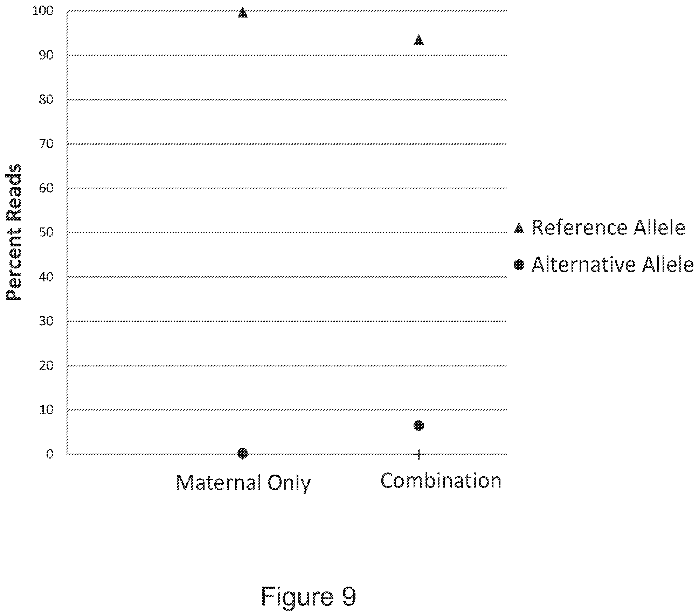

Figure 9 depicts an exemplary informative marker in a maternal only sample and a combination sample. Triangle: reference allele; circle: alternative allele.

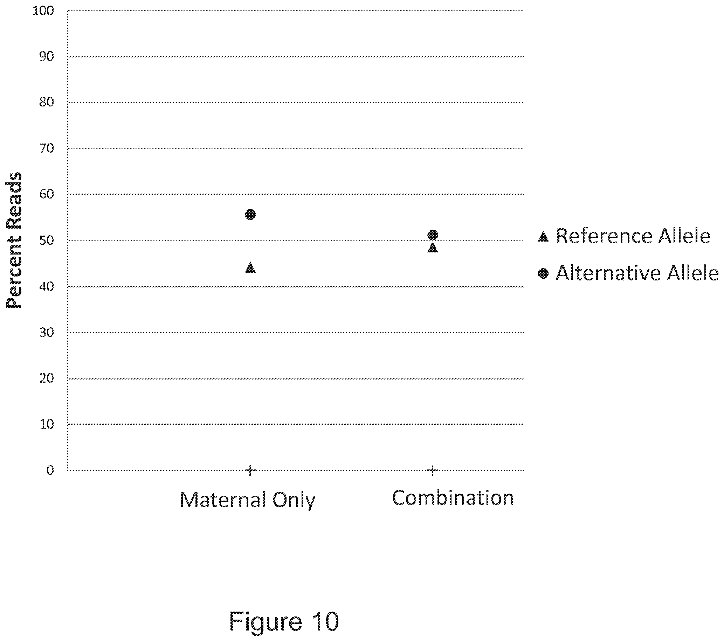

Figure 10 depicts an exemplary non-informative marker in a maternal only sample and a combination sample. Triangle: reference allele; circle: alternative allele.

### Detailed Description

Unless otherwise defined herein, scientific and technical terms used in this application shall have the meanings that are commonly understood by those of ordinary skill in the art. Generally, nomenclature used in connection with, and techniques of, cell and tissue culture, molecular biology, cell and cancer biology, neurobiology, neurochemistry, virology, immunology, microbiology, pharmacology, genetics and protein and nucleic acid chemistry, described herein, are those well known and commonly used in the art.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

A "patient", "subject", or "individual" are used interchangeably and refer to either a human or a non-human animal. These terms include mammals, such as humans, primates, livestock animals (e.g., bovines, porcines), companion animals (e.g., canines, felines) and rodents (e.g., mice and rats).

As used herein, a control subject refers to any individual that has not been diagnosed as having the disease or condition being assayed. The terms "normal control", "healthy control", and "not-diseased cells" likewise mean a sample (e.g., cells, serum, tissue) taken from a source (e.g., subject, control subject, cell line) that does not have the condition or disease being assayed and therefore may be used to determine the baseline for the condition or disorder being measured. It is also understood that the control subject, normal control, and healthy control, include data obtained and used as a standard, i.e. it can be used over and over again for multiple different subjects. In other words, for example, when comparing a subject sample to a control sample, the data from the control sample could have been obtained in a different set of experiments, for example, it could be an average obtained from a number of healthy subjects and not actually obtained at the time the data for the subject was obtained.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, e.g., a marker, the presence, absence, amount, or change in amount of which is indicative of the presence, severity, or absence of the condition.

The term "prognosis" as used herein refers to is used herein to refer to the likelihood of a disease or condition progression, including recurrence of a disease or condition.

### Description of Methods

By using a profile derived from a combination of sources, the skilled worker is able to capture data that is normally lost in the process of separating components out of a sample for testing. At the same time, the analytical components being combined are enriched for the markers used. Thus, the methods described herein do not introduce unnecessary "noise" into the signal. Methods using a subject-specific profile comparison also eliminate the dependence on a population-derived average profile for a particular disease or condition. Using a population-derived average profile may introduce error into the detection or diagnosis of a particular disease or condition in the subject. Therefore, methods described herein allow detection, diagnosis, and treatment to be personalized to the individual.

The methods described herein have high specificity, sensitivity, and accuracy, and are capable of detecting disease or condition-specific markers present within a bodily fluid sample, cells or tissues. The methods described herein may have improved specificity, sensitivity, and accuracy compared to current methods. The methods described herein may also reduce the problem of losing valuable signal when, for example, a patient sample is collected and separated into individual components, of which only one is chosen for testing. Accordingly, in certain aspects, the disclosure provides non-invasive assays for the early detection of a disease or condition, i.e., before the disease can be diagnosed by conventional diagnostic techniques, e.g., imaging techniques, and, therefore, provide a foundation for improved decision-making relative to the needs and strategies for intervention, prevention, and treatment of individuals with such disease or condition.

The present disclosure provides methods for diagnosing or aiding in the diagnosis of a disease or condition by comparing profiles (e.g., gene/protein/lipid/carbohydrate expression profiles, genotypes, gene copy number, gene dosage, DNA methylation, etc.) of disease or condition-associated markers (e.g., nucleic acids, proteins, lipids, carbohydrates, metabolites). One profile used for the comparison may be a profile from a sample comprising combinations of two or more different components (e.g., cell-free bodily fluids, phagocytic cells, circulating vesicles, and circulating diseased cells) from a subject. Alternatively, profile used for the comparison may be a profile from a sample comprising analytes isolated from, e.g., cell-free bodily fluids, phagocytic cells, phagocytic cells having a DNA content more than 2n (>2n phagocytic cells), circulating vesicles, and circulating diseased cells from a subject. For example, a sample comprising analytes from phagocytes and a cell-free bodily fluid could be generated by isolating analytes (e.g., nucleic acids or proteins) from phagocytes (e.g., by lysing the cells and using affinity-based techniques to isolate the analytes) and analytes (e.g., nucleic acids or proteins) from a cell-free bodily fluid, and combining the analytes to create a sample useful in creating a profile. For ease of reference, a sample comprising combinations of two or more different components (e.g., cell-free bodily fluids, phagocytic cells, >2n phagocytic cells, circulating vesicles, and circulating diseased cells, or analytes isolated therefrom), may be referred to herein as a "combination sample." The combination sample may comprise a lysate, or a fraction or portion of a lysate, of a population of phagocytic cells, a population of >2n phagocytic cells, a population of circulating vesicles, or a population of circulating diseased cells in place of the population of phagocytic cells, the population of >2n phagocytic cells, the population of circulating vesicles, or the population of circulating diseased cells, respectivelyThe combination sample may comprise an analyte isolated from a lysate, or a fraction or portion of an analyte isolated from a lysate, of a population of phagocytic cells, a population of >2n phagocytic cells, a population of circulating vesicles, or a population of circulating diseased cells in place of the population of phagocytic cells, the population of >2n phagocytic cells, the population of circulating vesicles, or the population of circulating diseased cells, respectively. The combination sample may comprise an analyte isolated from a cell-free bodily fluid, or a portion or fraction of an analyte isolated from a cell-free bodily fluid, rather than the cell-free bodily fluid itself. A control profile may be from phagocytic cells, phagocytic cells having a DNA content of 2n (=2n phagocytic cells), non-phagocytic cells, or control cells that are substantially free of cells affected by the disease or condition, taken from the same individual. Alternatively, the control profile may be a profile from a repository of markers of a disease or condition. In the context herein, "control cells that are substantially free of cells affected by the disease or condition" refers to a population of cells, as compared to circulating diseased cells, comprise significantly fewer amounts of cells affected by the disease or condition. "Control cells that are substantially free of cells affected by the disease or condition" are cells that are at least 75%, 80%, 85%, 90%, 91%, 92%, 93%, 94%, 95%, 96%, 97%, 98%, 99%, 99.5%, or 100% free of cells affected by the disease or condition. Control cells that can be used in the methods described herein are substantially free of fetal material (e.g., nucleic acids, proteins, and any analyte described herein), such as control cells that are at least 75%, 80%, 85%, 90%, 91%, 92%, 93%, 94%, 95%, 96%, 97%, 98%, 99%, 99.5%, or 100% free of fetal material.

This disclosure also provides methods for assessing the risk of developing a disease or condition, prognosing said disease, monitoring said disease progression or regression, assessing the efficacy of a treatment, or identifying a compound capable of ameliorating or treating said disease or condition.

One or more components of whole blood that are substantially free of markers of a disease or condition may be isolated from a whole blood sample. Here, the one or more components of whole blood that are substantially free of markers may be used as a control sample (e.g., to determine a control profile), and the remaining portion of the whole blood sample may be used as an analytical sample (e.g., to determine an analytical profile). For example, a population of non-phagocytic cells may be isolated from a whole blood sample and used to determine a control profile, while the remaining portion of the whole blood sample is used to determine an analytical profile.

The methods described herein can be used together with any known diagnostic methods, such as physical inspection, visual inspection, biopsy, scanning, histology, radiology, imaging, ultrasound, use of a commercial kit, genetic testing, immunological testing, analysis of bodily fluids, or monitoring neural activity.

Phagocytic cells that can be used in the methods described herein include all types of cells that are capable of ingesting various types of substances (e.g., apoptotic cells, infectious agents, dead cells, viable cells, cell-free DNAs, cell-free RNAs, cell-free proteins). The phagocytic cells may be neutrophils, macrophages, monocytes, dendritic cells, foam cells, mast cells, eosinophils, or keratinocytes. The phagocytic cells can be a mixture of different types of phagocytic cells. The phagocytic cells can be activated phagocytic cells., e.g., activated macrophages or neutrohils. A phagocyte may be a histiocyte, e.g., a Langerhans cell.

As used herein, ">2n phagocytic cells" refer to phagocytic cells that have a DNA content of greater than 2n, while "=2n phagocytic cells" refer to phagocytic cells that have a DNA content of 2n. According to the present disclosure, some phagocytic cells engulf live/dying/dead diseased cells (and sub-cellular fragments thereof) and/or cell-free disease-specific nucleic acids, proteins, carbohydrates and/or lipids present in bodily fluids. Such phagocytosis leads to the internalization of these disease markers into the phagocytic cell and, therefore, the DNA content of these phagocytic cells will become greater than 2n. By contrast, some phagocytic cells have not engulfed living/dying/dead diseased cells or fragments and/or cell-free disease-specific nucleic acids, proteins, lipids, and/or carbohydrates present in bodily fluids. The DNA contents of this group of phagocytic cells remain 2n. The disease-specific markers (e.g., DNA with disease-specific mutations) can be expressed by the >2n phagocytic cells. For example, the mutated DNA of diseased cells is integrated into the normal DNA of the >2n phagocytic cells. The subsequent transcription of the "integrated" DNA of the >2n phagocytic cells into RNA and the translation of the latter into proteins produces a phenotype different from the phagocytic cells that have not phagocytosed the diseased cells (i.e., the =2n phagocytic cells). The internalized disease-specific markers may not be expressed by the >2n phagocytic cells. The markers may be translocated onto the membranes of the >2n phagocytic cells, or secreted out by the >2n phagocytic cells.

Circulating diseased cells that can be used in the methods described herein include all types of circulating cells that may be affected by a disease or condition or infected by an infectious agent. A circulating cell refers to a cell present in the bodily fluid. A circulating cell may not necessarily circulate throughout the entire body or in the circulatory system. For example, a circulating cell may be present locally, such as in synovial fluid, or cerebrospinal fluid, or lymph fluid. A circulating diseased cell may also be detached from a tissue or organ that has been affected by a disease or condition or infected by an infectious agent.

Cells that can be used as control cells in the methods described herein includes all types of normal cells, or healthy cells, or cells that are substantially free of a disease or condition, or cells that are substantially free of an infectious agent. Control cells may be circulating cells or non-circulating cells (e.g., biopsied cells) that are representative of a normal or non-diseased state to which measurements on circulating diseased cells are compared to determine whether one or more diseased-associated marker is present in different levels between the circulating diseased cells and the control cells. The nature of the control cell may be a matter of design choice for a particular assay and may be derived or determined from normal tissue of the patient him- or herself.

The circulating diseased cells may be blood cells, tumor cells, lymphoma cells, fetal cells, apoptotic cells, epithelia cells, endothelial cells, stem cells, progenitor cells, mesenchymal cells, osteoblast cells, osteocytes, hematopoietic stem cells, foam cells, adipose cells, transcervical cells, circulating cardiocytes, circulating fibrocytes, circulating cancer stem cells, circulating myocytes, circulating cells from kidney, circulating cells from gastrointestinal tract, circulating cells from lung, circulating cells from reproductive organs, circulating cells from central nervous system, circulating hepatic cells, circulating cells from spleen, circulating cells from thymus, circulating cells from thyroid , circulating cells from an endocrine gland, circulating cells from parathyroid, circulating cells from pituitary, circulating cells from adrenal gland, circulating cells from islets of Langerhans, circulating cells from pancreas, circulating cells from hypothalamus, circulating cells from prostate tissues, circulating cells from breast tissues, circulating cells from circulating retinal cells, circulating ophthalmic cells, circulating auditory cells, circulating epidermal cells, or circulating cells from the urinary tract. The circulating diseased cells can be a mixture of different types of circulating diseased cells.

The circulating diseased cells that can be used in the methods described herein may be affected by various diseases or conditions. Exemplary diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a mitochondrial disorder, a respiratory-gastrointestinal tract disease or condition, a reproductive disease or condition, an ophthalmic disease or condition, a musculo-skeletal disease or condition, or a dermal disease or condition.

The circulating diseased cells that can be used in the methods described herein may be infected by an infectious agent, such as a virus, bacteria, fungus, parasite, protozoan, infectious protein or microorganism.

A cell useful in the methods described herein (e.g., a circulating diseased cell, control cell not affected by the disease or condition, phagocytic cell, >2n phagocytic cell, =2n phagocytic cell, or non-phagocytic cell) may be enucleated. Cells may be enucleated, for example, by using physical removal (e.g., via microneedle, optical tweezer, or aspiration), chemical treatments, photoablation, or ultraviolet irradiation.

As used herein, a "circulating vesicle" refers to a membrane-bound vesicle of cellular origin. The circulating vesicle may not necessarily circulate throughout the entire body or in the circulatory system. For example, the circulating vesicle may be present locally, such as in synovial fluid, or cerebrospinal fluid, or lymph fluid. The circulating vesicles may be selected from the group consisting of circulating microvesicles, apoptotic bodies, micro-particles, membrane-bound vesicles, multivesicular bodies, nanovesicles, microparticles, and ARRDC-1 mediated microvesicles (ARMM). Also, the circulating microvesicles may be exosomes or urinary exosomes.

A combination sample may comprise two or more different components selected from the group consisting of: a cell-free bodily fluid isolated from a subject, a population of phagocytic cells isolated from the subject, a population of >2n phagocytic cells isolated from the subject, a population of circulating vesicles isolated from the subject, and a population of circulating diseased cells isolated from the subject. A combination sample may comprise an analyte isolated from a cell-free bodily fluid isolated from the subject, an analyte isolated from a population of phagocytic cells isolated from the subject, an analyte isolated from a population of >2n phagocytic cells isolated from the subject, an analyte isolated from a population of circulating vesicles isolated from the subject, and an analyte isolated from a population of circulating diseased cells isolated from the subject.

As used herein, a "profile" of a marker of a disease or condition can broadly refer to any information concerning the marker. This information can be either qualitative (e.g., presence or absence) or quantitative (e.g., levels, copy numbers, or dosages). A profile of a marker can indicate the absence of this marker. The profile can be a nucleic acid (e.g., DNA or RNA) profile, a protein profile, a lipid profile, a carbohydrate profile, a metabolite profile, or a combination thereof. A "marker" as used herein generally refers to an analyte which is differentially detectable in phagocytes and is indicative of the presence of a disease or condition. An analyte is differentially detectable if it can be distinguished quantitatively or qualitatively in phagocytes.

The methods described herein can be applied to various diseases or conditions. Exemplary diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder, or condition, a mitochondrial disorder, a respiratory-gastrointestinal tract disease or condition, a reproductive disease or condition, an ophthalmic disease or condition, a musculo-skeletal disease or condition, or a dermal disease or condition.

As used herein, the term "cardiovascular disease or condition" refers to any condition that affects systems of heart or blood vessels (arteries and veins). Examples of cardiovascular diseases include, but are not limited to myocardial infarction, coronary artery disease, percutaneous transluminal coronary angioplasty (PTCA), coronary artery bypass surgery (CABG), restenosis, peripheral arterial disease, stroke, abdominal aorta aneurysm, intracranial aneurysm, large artery atherosclerotic stroke, cardiogenic stroke, an early onset myocardial infarction, heart failure, pulmonary embolism, acute coronary syndrome (ACS), angina, cardiac hypertrophy, arteriosclerosis, myocarditis, pancarditis, endocarditis, hypertension, congestive heart failure, atherosclerosis, cerebrovascular disease, declining cardiac health, ischemic heart disease, pericarditis, cardiogenic shock, alcoholic cardiomyopathy, congenital heart disease, ischemic cardiomyopathy, hypertensive cardiomyopathy, valvular cardiomyopathy, inflammatory cardiomyopathy, cardiomyopathy secondary to a systemic metabolic disease, dilated cardiomyopathy, hypertrophic cardiomyopathy, arrhythmogenic right ventricular cardiomyopathy, restrictive cardiomyopathy, noncompaction cardiomyopathy, valvular heart disease, hypertensive heart disease, myocardial ischemic attack, unstable angina, myocardial rupture, cardiogenic shock, embolism, deep vein thrombosis, arrhythmia, arrhythmogenic right ventricular cardiomyopathy, diabetic cardiomyopathy, mitral regurgitation, mitral valve prolapse, peripheral vascular disease, artery disease, carotid artery disease, deep vein thrombosis, venous diseases, cerebrovascular disease, arterial aneurysm, left ventricular hypertrophy, hypertensive renal disease, hypertensive retinal disease, vasculitis, left main disease, arterial vascular disease, venous vascular disease, thrombosis of the microcirculation, a transient cerebrovascular accident, limb ischemia, aneurysm, thrombosis, superficial venous thrombosis, and deep venous thrombosis.

As used herein, the term "kidney-associated disease or condition" refers to any disease or condition that affects kidney or renal system. Examples of kidney-associated disease include, but are not limited to, chronic kidney diseases, primary kidney diseases, non-diabetic kidney diseases, glomerulonephritis, interstitial nephritis, diabetic kidney diseases, diabetic nephropathy, glomerulosclerosis, rapid progressive glomerulonephritis, renal fibrosis, Alport syndrome, IDDM nephritis, mesangial proliferative glomerulonephritis, membrano proliferative glomerulonephritis, crescentic glomerulonephritis, renal insterstitial fibrosis, focal segmental glomerulosclerosis, membranous nephropathy, minimal change disease, pauci-immune rapid progressive glomerulonephritis, IgA nephropathy, polycystic kidney disease, Dent's disease, nephrocytinosis, Heymann nephritis, autosomal dominant (adult) polycystic kidney disease, autosomal recessive (childhood) polycystic kidney disease, acute kidney injury, nephrotic syndrome, renal ischemia, podocyte diseases or disorders, proteinuria, glomerular diseases, membranous glomerulonephritis, focal segmental glomerulonephritis, pre-eclampsia, eclampsia, kidney lesions, collagen vascular diseases, benign orthostatic (postural) proteinuria, IgM nephropathy, membranous nephropathy, sarcoidosis, diabetes mellitus, kidney damage due to drugs, Fabry's disease, aminoaciduria, Fanconi syndrome, hypertensive nephrosclerosis, interstitial nephritis, Sickle cell disease, hemoglobinuria, myoglobinuria, Wegener's Granulomatosis, Glycogen Storage Disease Type 1, chronic kidney disease, chronic renal failure, low Glomerular Filtration Rate (GFR), nephroangiosclerosis, lupus nephritis, ANCA-positive pauci-immune crescentic glomerulonephritis, chronic allograft nephropathy, nephrotoxicity, renal toxicity, kidney necrosis, kidney damage, glomerular and tubular injury, kidney dysfunction, nephritic syndrome, acute renal failure, chronic renal failure, proximal tubal dysfunction, acute kidney transplant rejection, chronic kidney transplant refection, non IgA mesangioproliferative glomerulonephritis, postinfectious glomerulonephritis, vasculitides with renal involvement of any kind, any hereditary renal disease, any interstitial nephritis, renal transplant failure, kidney cancer, kidney disease associated with other conditions (e.g., hypertension, diabetes, and autoimmune disease), Dent's disease, nephrocytinosis, Heymann nephritis, a primary kidney disease, a collapsing glomerulopathy, a dense deposit disease, a cryoglobulinemia-associated glomerulonephritis, an Henoch-Schonlein disease, a postinfectious glomerulonephritis, a bacterial endocarditis, a microscopic polyangitis, a Churg-Strauss syndrome, an anti-GBM-antibidy mediated glomerulonephritis, amyloidosis, a monoclonal immunoglobulin deposition disease, a fibrillary glomerulonephritis, an immunotactoid glomerulopathy, ischemic tubular injury, a medication-induced tubulo-interstitial nephritis, a toxic tubulo-interstitial nephritis, an infectious tubulo-interstitial nephritis, a bacterial pyelonephritis, a viral infectious tubulo-interstitial nephritis which results from a polyomavirus infection or an HIV infection, a metabolic-induced tubulo-interstitial disease, a mixed connective disease, a cast nephropathy, a crystal nephropathy which may results from urate or oxalate or drug-induced crystal deposition, an acute cellular tubulo-interstitial allograft rejection, a tumoral infiltrative disease which results from a lymphoma or a post-transplant lymphoproliferative disease, an obstructive disease of the kidney, vascular disease, a thrombotic microangiopathy, a nephroangiosclerosis, an atheroembolic disease, a mixed connective tissue disease, a polyarteritis nodosa, a calcineurin-inhibitor induced- vascular disease, an acute cellular vascular allograft rejection, an acute humoral allograft rejection, early renal function decline (ERFD), end stage renal disease (ESRD), renal vein thrombosis, acute tubular necrosis, acute interstitial nephritis, established chronic kidney disease, renal artery stenosis, ischemic nephropathy, uremia, drug and toxin-induced chronic tubulointerstitial nephritis, reflux nephropathy, kidney stones, Goodpasture's syndrome, and hydronephrosis.

As used herein, the term "prenatal or pregnancy-related disease or condition" refers to any disease, disorder, or condition affecting a pregnant woman, embryo, or fetus. Prenatal or pregancy-related conditions can also refer to any disease, disorder, or condition that is associated with or arises, either directly or indirectly, as a result of pregnancy. These diseases or conditions can include any and all birth defects, congenital conditions, or hereditary diseases or conditions. Examples of prenatal or pregnancy-related diseases include, but are not limited to, Rhesus disease, hemolytic disease of the newborn, beta-thalassemia, sex determination, determination of pregnancy, a hereditary Mendelian genetic disorder, chromosomal aberrations, a fetal chromosomal aneuploidy, fetal chromosomal trisomy, fetal chromosomal monosomy, trisomy 8, trisomy 13 (Patau Syndrom), trisomy 16, trisomy 18 (Edwards syndrome), trisomy 21 (Down syndrome), X-chromosome linked disorders, trisomy X (XXX syndrome), monosomy X (Turner syndrome), XXY syndrome, XYY syndrome, XYY syndrome, XXXY syndrome, XXYY syndrome, XYYY syndrome, XXXXX syndrome, XXXXY syndrome, XXXYY syndrome, XXYYY syndrome, Fragile X Syndrome, fetal growth restriction, cystic fibrosis, a hemoglobinopathy, fetal death, fetal alcohol syndrome, sickle cell anemia, hemophilia, Klinefelter syndrome, dup(17)(p11.2p1.2) syndrome, endometriosis, Pelizaeus-Merzbacher disease, dup(22)(q11.2q11.2) syndrome, cat eye syndrome, cri-du-chat syndrome, Wolf-Hirschhorn syndrome, Williams-Beuren syndrome, Charcot-Marie-Tooth disease, neuropathy with liability to pressure palsies, Smith-Magenis syndrome, neurofibromatosis, Alagille syndrome, Velocardiofacial syndrome, DiGeorge syndrome, steroid sulfatase deficiency, Prader-Willi syndrome, Kallmann syndrome, microphthalmia with linear skin defects, adrenal hypoplasia, glycerol kinase deficiency, Pelizaeus-Merzbacher disease, testis-determining factor on Y, azospermia (factor a), azospermia (factor b), azospermia (factor c), 1p36 deletion, phenylketonuria, Tay-Sachs disease, adrenal hyperplasia, Fanconi anemia, spinal muscular atrophy, Duchenne's muscular dystrophy, Huntington's disease, myotonic dystrophy, Robertsonian translocation, Angelman syndrome, tuberous sclerosis, ataxia telangieltasia, open spina bifida, neural tube defects, ventral wall defects, small-for-gestational-age, congenital cytomegalovirus, achondroplasia, Marfan's syndrome, congenital hypothyroidism, congenital toxoplasmosis, biotinidase deficiency, galactosemia, maple syrup urine disease, homocystinuria, medium-chain acyl Co-A dehydrogenase deficiency, structural birth defects, heart defects, abnormal limbs, club foot, anencephaly, arhinencephaly/holoprosencephaly, hydrocephaly, anophthalmos/microphthalmos, anotia/microtia, transposition of great vessels, tetralogy of Fallot, hypoplastic left heart syndrome, coarctation of aorta, cleft palate without cleft lip, cleft lip with or without cleft palate, oesophageal atresia/stenosis with or without fistula, small intestine atresia/stenosis, anorectal atresia/stenosis, hypospadias, indeterminate sex, renal agenesis, cystic kidney, preaxial polydactyly, limb reduction defects, diaphragmatic hernia, blindness, cataracts, visual problems, hearing loss, deafness, X-linked adrenoleukodystrophy, Rett syndrome, lysosomal disorders, cerebral palsy, autism, aglossia, albinism, ocular albinism, oculocutaneous albinism, gestational diabetes, Arnold-Chiari malformation, CHARGE syndrome, congenital diaphragmatic hernia, brachydactlia, aniridia, cleft foot and hand, heterochromia, Dwarnian ear, Ehlers Danlos syndrome, epidermolysis bullosa, Gorham's disease, Hashimoto's syndrome, hydrops fetalis, hypotonia, Klippel-Feil syndrome, muscular dystrophy, osteogenesis imperfecta, progeria, Smith Lemli Opitz symdrom, chromatelopsia, X-linked lymphoproliferative disease, omphalocele, gastroschisis, pre-eclampsia, eclampsia, pre-term labor, premature birth, miscarriage, delayed intrauterine growth, ectopic pregnancy, hyperemesis gravidarum, morning sickness, or likelihood for successful induction of labor.

As used herein, the term "a neurological or neuropsychiatric disease or condition" refers to any disease or condition that affects nervous systems. Examples of neurological or neuropsychiatric diseases or conditions include, but are not limited to, head trauma, stroke, stroke, ischemic stroke, hemorrhagic stroke, subarachnoid hemorrhage, intra cranial hemorrhage, transient ischemic attack, vascular dementia, corticobasal ganglionic degeneration, encephalitis, epilepsy, Landau-Kleffner syndrome, hydrocephalus, pseudotumor cerebri, thalamic diseases, meningitis, myelitis, movement disorders, essential tremor, spinal cord diseases, syringomyelia, Alzheimer's disease (early onset), Alzheimer's disease (late onset), multi-infarct dementia, Pick's disease, Huntingdon's disease, Parkinson's disease, Parkinson syndromes, dementia, frontotemporal dementia, corticobasal degeneration, multiple system atrophy, progressive supranuclear palsy, Lewy body disease, Creutzfeldt-Jakob disease, Dandy-Walker syndrome, Friedreich ataxia, Machado-Joseph disease, migraine, schizophrenia, mood disorders and depression. dementia with lewy bodies (DLB), frontotemporal dementia (FTD), various forms of vascular dementia (VD), subcortical vascular dementia (Binswanger's disease), autism, developmental retardations, motor neuron diseases, amyotrophic lateral sclerosis (ALS), neuronal or brain damage, hypoxia of the brain, cerebral palsy (CP), memory disorders, movement disorders, corticalbasal ganglionic degeneration, forms of multiple system atrophy, stroke-related disorders, cerebrovascular accidents, post-irradiation encephalopathy with seizures, vascular Parkinsonism, thalamic cerebrovascular accidents, chronic inflammatory demyelinating polyneuropathy, alcohol related dementia, semantic dementia, ataxia, atypical Parkinsonism, dystonia, progressive supranuclear palsy, essential tremor, mild cognitive impairment, amyotrophic lateral sclerosis, multiple sclerosis, neuropathies, Pick's disease, congophilic amyloid angiopathy, Creutzfeldt-Jakob Disease, AIDS dementia complex, depression, anxiety disorder, phobia, Bell's Palsy, epilepsy, encephalitis, neuromuscular disorders, neurooncological disorders, brain tumors, neurovascular disorders, neuroimmunological disorders, neurootological disease, neurotrauma including spinal cord injury, pain including neuropathic pain, pediatric neurological and neuropsychiatric disorders, sleep disorders, Tourette syndrome, corticalbasal ganglionic degeneration, alcohol related dementia, semantic dementia, Alzheimer's disease combined with multi-infarct dementia, Alzheimer's disease combined with Lewy body dementia, Parkinson's disease combined with Lewy body dementia, Alzheimer's and Parkinson's disease combined with Lewy body dementia, frontotemporal dementia combined with chronic inflammatory demyelinating polyneuropathy, attention deficit hyperactivity disorder, schizophrenia, obsessive-compulsive disorder, mental retardation, autistic spectrum disorders, opsoclonus-myoclonus syndrome (OMS) seizures, articulation disorder, learning disabilities (i.e., reading or arithmetic), verbal or performance aptitude deficits, attention deficit disorder, amyloid diseases, prion diseases, Tauopathies, Alpha-Synucleinopathies, addictive states such as those caused by at least one of: cocaine, nicotine, alcohol, food, ecstasy, kat, caffeine, opium, heroin, marijuana, amphetamine, methamphetamine or gambling, and Fabry's disease.

As used herein, the term "an autoimmune or immune-related disease or condition" refers to any disease or condition that affects the function of immune systems. Examples of autoimmune or immune-related diseases or conditions include, but are not limited to, antiphospholipid syndrome, systemic lupus erythematosus, rheumatoid arthritis, autoimmune vasculitis, celiac disease, autoimmune thyroiditis, post-transfusion immunization, maternal-fetal incompatibility, transfusion reactions, immunological deficiency such IgA deficiency, common variable immunodeficiency, drug-induced lupus, diabetes mellitus, Type I diabetes, Type II diabetes, juvenile onset diabetes, juvenile rheumatoid arthritis, psoriatic arthritis, multiple sclerosis, immunodeficiency, allergies, asthma, psoriasis, atopic dermatitis, allergic contact dermatitis, chronic skin diseases, amyotrophic lateral sclerosis, chemotherapy-induced injury, graft-vs-host diseases, bone marrow transplant rejection, Ankylosing spondylitis, atopic eczema, Pemphigus, Behcet's disease, chronic fatigue syndrome fibromyalgia, chemotherapy-induced injury, myasthenia gravis, glomerulonephritis, allergic retinitis, systemic sclerosis, subacute cutaneous lupus erythematosus, cutaneous lupus erythematosus including chilblain lupus erythematosus, Sjogren's syndrome, autoimmune nephritis, autoimmune vasculitis, autoimmune hepatitis, autoimmune carditis, autoimmune encephalitis, autoimmune mediated hematological diseases, lc-SSc (limited cutaneous form of scleroderma), dc-SSc (diffused cutaneous form of scleroderma), autoimmune thyroiditis (AT), Grave's disease (GD), myasthenia gravis, multiple sclerosis (MS), ankylosing spondylitis. transplant rejection, immune aging, rheumatic/autoimmune diseases, mixed connective tissue disease, spondyloarthropathy, psoriasis, psoriatic arthritis, myositis, scleroderma, dermatomyositis, autoimmune vasculitis, mixed connective tissue disease, idiopathic thrombocytopenic purpura, Crohn's disease, human adjuvant disease, osteoarthritis, juvenile chronic arthritis, a spondyloarthropathy, an idiopathic inflammatory myopathy, systemic vasculitis, sarcoidosis, autoimmune hemolytic anemia, autoimmune thrombocytopenia, thyroiditis, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease, psoriatic arthritis, psoriasis, dermatitis, polymyositis/dermatomyositis, toxic epidermal necrolysis, systemic scleroderma and sclerosis, responses associated with inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti-phospholipid antibody syndrome, allergic neuritis, Bechet disease, Castleman's syndrome, Goodpasture's syndrome, Lambert-Eaton Myasthenic Syndrome, Reynaud's syndrome, Sjorgen's syndrome, Stevens-Johnson syndrome, pemphigoid bullous, pemphigus, autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimmune orchitis and oophoritis, primary hypothyroidism, autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Sheehan's syndrome, autoimmune hepatitis, lymphoid interstitial pneumonitis (HIV), bronchiolitis obliterans (non-transplant) vs NSIP, Guillain-Barre' Syndrome, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, and amyotrophic lateral sclerosis (ALS).

As used herein, the term "cancer" refers to various types of malignant neoplasms, most of which can invade surrounding tissues, and may metastasize to different sites (see, for example, PDR Medical Dictionary, 1st edition (1995)). The terms "neoplasm" and "tumor" refer to an abnormal tissue that grows by cellular proliferation more rapidly than normal and continues to grow after the stimuli that initiated proliferation is removed. Such abnormal tissue shows partial or complete lack of structural organization and functional coordination with the normal tissue which may be either benign (i.e., benign tumor) or malignant (i.e., malignant tumor). Examples of general categories of cancer include, but are not limited to, carcinomas (i.e., malignant tumors derived from epithelial cells such as, for example, common forms of breast, prostate, lung and colon cancer), sarcomas (i.e., malignant tumors derived from connective tissue or mesenchymal cells), lymphomas (i.e., malignancies derived from hematopoietic cells), leukemias (i.e., malignancies derived from hematopoietic cells), germ cell tumors (i.e., tumors derived from totipotent cells. In adults most often found in the testicle or ovary; in fetuses, babies and young children, most often found on the body midline, particularly at the tip of the tailbone), blastic tumors (i.e., a typically malignant tumor which resembles an immature or embryonic tissue) and the like. Examples of the types of neoplasms intended to be encompassed by the present disclosure include but are not limited to those neoplasms associated with cancers of neural tissue, blood forming tissue, breast, skin, bone, prostate, ovaries, uterus, cervix, liver, lung, brain, larynx, gallbladder, pancreas, rectum, parathyroid, thyroid, adrenal gland, immune system, head and neck, colon, stomach, bronchi, and/or kidneys.

As used herein, the term "an infectious disease or condition" refers to any disease or condition that results from an infectious agent. Infectious agents include, but are not limited to bacteria, viruses, fungi, protozoa, infectious proteins, parasitic microbes, and other parasites. Examples of infectious diseases or conditions include, but are not limited to, bacterial infections, viral infections, fungal infections, protozoan infections, parasitic infections, hepatitis (e.g., hepatitis A, B, C, D, and E), herpes, influenza, human papillomavirus (HPV) infection, AIDS, anthrax, pneumonia (bacterial or viral), cellulitis, human parainfluenza, the common cold, Legionnaires' disease (Legionellosis), cholera, Creutzfeldt-Jakob disease (CJD), variant Creutzfeldt-Jakob disease (vCJD), fatal familial insomnia (FFI), Gerstmann-Straussler-Scheinker (GSS) syndrome, Chlamydia, chicken pox, ebola hemorrhagic fever, Dengue fever, giardiasis, Lyme disease, malaria, measles, mumps, rubella, pertussis, gonorrhea, staphylococcal infection, streptococcal infection, pneumococcal infection, rabies, helicobacter pylori infection, respiratory syncitial virus infection, Rocky Mountain spotted fever, SARS, sepsis, tuberculosis, and West Nile fever.

Viruses include, but are not limited to, DNA or RNA animal viruses. As used herein, RNA viruses include, but are not limited to, virus families such as Picornaviridae (e.g., polioviruses), Reoviridae (e.g., rotaviruses), Togaviridae (e.g., encephalitis viruses, yellow fever virus, rubella virus), Orthomyxoviridae (e.g., influenza viruses), Paramyxoviridae (e.g., respiratory syncytial virus, measles virus, mumps virus, parainfluenza virus), Rhabdoviridae (e.g., rabies virus), Coronaviridae, Bunyaviridae, Flaviviridae, Filoviridae, Arenaviridae, Bunyaviridae and Retroviridae (e.g., human T cell lymphotropic viruses (HTLV), human immunodeficiency viruses (HIV)). As used herein, DNA viruses include, but are not limited to, virus families such as Papovaviridae (e.g., papilloma viruses), Adenoviridae (e.g., adenovirus), Herpesviridae (e.g., herpes simplex viruses), and Poxviridae (e.g., variola viruses).

Bacteria include, but are not limited to, gram positive bacteria, gram negative bacteria, acid-fast bacteria and the like. Gram positive bacteria include, but are not limited to, Actinomedurae, Actinomyces israelii, Bacillus anthracis, Bacillus cereus, Clostridium botulinum, Clostridium difficile, Clostridium perfringens, Clostridium tetani, Corynebacterium, Enterococcus faecalis, Listeria monocytogenes, Nocardia, Propionibacterium acnes, Staphylococcus aureus, Staphylococcus epiderm, Streptococcus mutans, Streptococcus pneumoniae and the like. Gram negative bacteria include, but are not limited to, Afipia felis, Bacteriodes, Bartonella bacilliformis, Bortadella pertussis, Borrelia burgdorferi, Borrelia recurrentis, Brucella, Calymmatobacterium granulomatis, Campylobacter, Escherichia coli, Francisella tularensis, Gardnerella vaginalis, Haemophilius aegyptius, Haemophilius ducreyi, Haemophilius influenziae, Heliobacter pylori, Legionella pneumophila, Leptospira interrogans, Neisseria meningitidia, Porphyromonas gingivalis, Providencia sturti, Pseudomonas aeruginosa, Salmonella enteridis, Salmonella typhi, Serratia marcescens, Shigella boydii, Streptobacillus moniliformis, Streptococcus pyogenes, Treponema pallidum, Vibrio cholerae, Yersinia enterocolitica, Yersinia pestis and the like. As used herein, acid-fast bacteria include, but are not limited to, Myobacterium avium, Myobacterium leprae, Myobacterium tuberculosis and the like. Other bacteria not falling into the other three categories include, but are not limited to, Bartonella henseiae, Chlamydia psittaci, Chlamydia trachomatis, Coxiella burnetii, Mycoplasma pneumoniae, Rickettsia akari, Rickettsia prowazekii, Rickettsia rickettsii, Rickettsia tsutsugamushi, Rickettsia typhi, Ureaplasma urealyticum, Diplococcus pneumoniae, Ehrlichia chafensis, Enterococcus faecium, Meningococci and the like.

As used herein, fungi include, but are not limited to, Aspergilli, Candidae, Candida albicans, Coccidioides immitis, Cryptococci, and combinations thereof.

As used herein, parasitic microbes include, but are not limited to, Balantidium coli, Cryptosporidium parvum, Cyclospora cayatanensis, Encephalitozoa, Entamoeba histolytica, Enterocytozoon bieneusi, Giardia lamblia, Leishmaniae, Plasmodii, Toxoplasma gondii, Trypanosomae, trapezoidal amoeba and the like. Other parasites include worms (e.g., helminthes), particularly parasitic worms including, but not limited to, Nematoda (roundworms, e.g., whipworms, hookworms, pinworms, ascarids, filarids and the like), and Cestoda (e.g., tapeworms).

As used herein, infectious proteins include prions (e.g., PrP^{Sc} forms, the CJD prion, the vCJD prion, the FFI prion, and the GSS prion).

As used herein, the term "a pediatric disease, disorder, or condition" refers to any disease or disorder that affects infants or children or that begins during development or childhood. Examples of pediatric diseases, disorders, and conditions include, but are not limited to, autism, Kawasaki's disease, congenital deafness, pediatric cancers, Type I diabetes, congenital heart defects, tetralogy of Fallot, Duchenne Muscular Dystrophy, osteogenesis importfect, Krabe disease, Pompe disease, Gaucher disease, Fabry disease, Wolff-Parkinson-White syndrome, Hirschsprung's disease, Crohn's disease, Eagle-Barrett Syndrome, cystic fibrosis, irritable bowel syndrome, and cerebral palsy. Examples of pediatric conditions also include genetic attributes of the developing fetus. For example, a pediatric conditions include, but are not limited to, intelligence, eye color, hair color, and muscle type.

As used herein, "treating" a disease or condition refers to taking steps to obtain beneficial or desired results, including clinical results. Beneficial or desired clinical results include, but are not limited to, alleviation or amelioration of one or more symptoms associated with diseases or conditions.

As used herein, "administering" or "administration of" a compound or an agent to a subject can be carried out using one of a variety of methods known to those skilled in the art. For example, a compound or an agent can be administered, intravenously, arterially, intradermally, intramuscularly, intraperitonealy, intravenously, subcutaneously, ocularly, sublingually, orally (by ingestion), intranasally (by inhalation), intraspinally, intracerebrally, and transdermally (by absorbtion, e.g., through a skin duct). A compound or agent can also appropriately be introduced by rechargeable or biodegradable polymeric devices or other devices, e.g., patches and pumps, or formulations, which provide for the extended, slow, or controlled release of the compound or agent. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient. A compound or an agent may be administered orally, e.g., to a subject by ingestion, or intravenously, e.g., to a subject by injection. The orally administered compound or agent may be in an extended release or slow release formulation, or administered using a device for such slow or extended release.

Markers used in the methods described herein are up-regulated or activated in the combination sample compared to the phagocytic cells, =2n phagocytic cells, non-phagocytic cells, or control cells that are substantially free of cells affected by the disease or condition. Different diseases or conditions can be associated with either up-regulation (or activation) or down-regulation (or inhibition) of different markers. As used herein, "up-regulation or up-regulated" can refer to an increase in expression levels (e.g., gene expression or protein expression), gene copy numbers, gene dosages, and other qualitative or quantitative detectable state of the markers. Similarly, "down-regulation or down-regulated" can refer to an increase in expression levels, gene copy numbers, gene dosages, and other qualitative or quantitative detectable state of the markers. Markers used in the methods described herein may be down-regulated or inhibited in the combination sample compared to the phagocytic cells, =2n phagocytic cells, non-phagocytic cells, or control cells that are substantially free of cells affected by the disease or condition. Different diseases or conditions can be associated with either up-regulation (or activation) or down-regulation (or inhibition) of different markers. As used herein, "activation or activated" can refer to an active state of the marker, e.g., a phosphorylation state, a DNA methylation state, or a DNA acetylation state. Similarly, "inhibition or inhibited" can refer to a repressed state or an inactivated state of the marker, e.g., a de-phosphorylation state, a ubiquitination state, a DNA de-methylation state.

Methods described herein can comprise extracting or enriching markers from cell-free bodily fluids. Any known extraction and enrichment methods can be used herein. Methods described herein also comprise at least one of the following steps before determination of various profiles: i) lysing the =2n phagocytic, non-phagocytic, or control cells; and ii) extracting cellular contents from the lysed cells. Any known cell lysis and extraction methods can be used herein. At least one or more markers of a disease or condition may be present in the combination sample. There may be no marker present in the cellular contents of the non-phagocytic, =2n phagocytic, or control cells.

Methods described herein also may comprise at least one of the following steps before determination of various profiles: i) lysing the phagocytic, >2n phagocytic cells, or =2n phagocytic cells; and ii) extracting cellular contents from the lysed phagocytic, >2n phagocytic, or =2n phagocytic cells. The cellular contents of the phagocytic or >2n phagocytic cells may comprise various types of materials that they have engulfed, such as viable diseased cells, dead diseased cells, apoptotic diseased cells, circulating tumor cells, infectious agents, fetal cells, trophoblasts, or fragments thereof. At least one or more markers of a disease or condition may be present in the cellular contents of the phagocytic or >2n phagocytic cells.

Methods described herein may also comprise at least one of the following steps before determination of various profiles: i) lysing the circulating vesicles; and ii) extracting the contents from the lysed circulating vesicles. The contents of the circulating vesicles may comprise various types of materials, such as proteins and nucleic acids. At least one or more markers of a disease or condition may be present in the contents of the circulating vesicles.

Methods described herein may also comprise at least one of the following steps before determination of various profiles: i) lysing the circulating diseased cells; and ii) extracting the contents from the lysed circulating diseased cells. At least one or more markers of a disease or condition may be present in the contents of the circulating diseased cells.

Methods described herein may further comprise comparing the identified difference of the disease or condition-specific markers to a repository of at least one markers known in the art. Such comparison can further confirm the presence of the disease or condition. The repository of the known markers can be obtained by data mining. The term "data mining", as used herein, refers to a process of finding new data patterns, relations, or correlations derived from the known data of the databases and of extracting practicable information in the future. Typically a computer-based system can be trained on data to perform the data mining, e.g., to classify the input data and then subsequently used with new input data to make decisions based on the training data. These systems include, but are not limited, expert systems, fuzzy logic, non-linear regression analysis, multivariate analysis, decision tree classifiers, and Bayesian belief networks.

The phagocytic cells, >2n phagocytic cells, circulating vesicles, circulating diseased cells, control cells, and =2n phagocytic cells may be isolated from a bodily fluid sample, tissues, or cells. Exemplary bodily fluid samples can be whole blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid. The phagocytic cells, >2n phagocytic cells, and =2n phagocytic cells may be isolated from white blood cells. The >2n phagocytic cells and the =2n phagocytic cells may be separated from a population of phagocytic cells.

The cell-free bodily fluid may come from a bodily fluid sample. Exemplary bodily fluid samples can be whole blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, fluid from a preimplantation embryo, maternal urine, maternal saliva, placental sample, fetal blood, lavage and cervical vaginal fluid, interstitial fluid, buccal swab sample, sputum, bronchial lavage, Pap smear sample, or ocular fluid. The cell-free bodily fluids may be obtained by separating cells from the bodily fluid sample by methods known in the art, such as extraction, centrifugation, and filtration.

A component for use in a combination sample may be obtained by removing cells from a bodily fluid. A component for use in a combination sample may be obtained by destroying (e.g., lysing) cells in a bodily fluid. This may be taken in combination, for example, by removing some populations of cells and destroying other populations of cells. A sample of whole blood may be used to create a combination sample, for example, by removing red blood cells, serum, and T cells, and using the remainder as the combination sample.

Tissue or fluid samples including cells having a DNA content of 2n may be obtained post separation (e.g., via centrifugation) of non-cellular fraction of fluids obtained by puncture of a vein or artery followed by the withdrawal of blood, tissue biopsies, bronchoalveolar lavage, nasal lavage, eye lavage, peritoneal cavity lavage, vaginal lavage, bladder lavage, rectal lavage, fine needle aspiration of spinal fluid, synovial fluid aspiration, and the like. Cell free bodily fluids are obtained post separation (e.g., via centrifugation) of cellular fraction of fluids obtained by puncture of a vein or artery followed by the withdrawal of blood, tissue biopsies, bronchoalveolar lavage, nasal lavage, eye lavage, peritoneal cavity lavage, vaginal lavage, bladder lavage, rectal lavage, fine needle aspiration of spinal fluid, synovial fluid aspiration, and the like.

In the methods described herein, cell separation/isolation/purification methods are used to isolate populations of cells from bodily fluid sample, cells, or tissues of a subject. A skilled worker can use any known cell separation/isolation/purification techniques to isolate phagocytic cells, >2n phagocytic cells, diseased cells, control cells, and =2n phagocytic cells from a bodily fluid, or to separate >2n phagocytic cells from =2n phagocytic cells. Exemplary techniques include, but are not limited to, using antibodies, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof.

In the methods described herein, cell separation/isolation/purification methods are used to isolate populations of circulating diseased cells from bodily fluid sample, cells, or tissues of a subject. Circulating diseased cells may be rare or in low quantity in a bodily fluid. Therefore, enrichment techniques (e.g., magnetic enrichment) may be used to enrich circulating diseased cells before the isolation. A skilled worker can use any known cell separation/isolation/purification techniques to isolate circulating diseased cells from a bodily fluid. Exemplary techniques include, but are not limited to, using antibodies, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, micro-fluidic technique, fiber-optic array-scanning technique, laser-scanning cytometry technique, multiphoton intravital flow cytometry, photoacoustic flowmetry, nanoparticles targeting cell surface antigens, staining circulating diseased cells with detectable secreted products, or a combination thereof. Circulating diseased cells may have different physical properties compared to normal circulating cells, such as difference in size, density, charge, migratory properties, and some properties of specific cell types (e.g., melanocytic granules in circulating melanoma cells). A skilled worker can use any known cell separation/isolation/purification techniques based on such different properties to isolate circulating diseased cells. For example, differences in buoyant density may be used to separate circulating diseased cells (e.g., circulating tumor cells) from normal blood cells through gradient centrifugation. Filtration-based approaches may be used isolate circulating diseased cells (e.g., circulating tumor cells) based on their increased sizes compared to normal circulating cells. Antibody-based isolation approaches may be used to capture circulating diseased cells, which express epithelia cell surface markers that are absent from normal circulating blood cells. For example, conjugation of antibodies against epithelial cell adhesion molecule (EpCAM) to magnetic beads, followed by purification of captured cells through a magnetic field, may be used to enrich circulating tumor cells from the blood of patients with cancers of the breast, prostate, and colon. Circulating diseased cells (e.g., transcervical cells) may be collected by the RareCellect™ device (Genetic Technologies) or similar devices.

The phagocytic cells, >2n phagocytic cells, diseased cells, control cells, and =2n phagocytic cells may be isolated by using a product secreted by the cells. The phagocytic cells, >2n phagocytic cells, diseased cells, control cells, and =2n phagocytic cells may be isolated by using a cell surface target (e.g., receptor protein) on the surface of the cells. The cell surface target may be a protein that has been engulfed by >2n phagocytic cells. The cell surface target may be expressed by cells on their plasma membranes. The cell surface target may be an exogenous protein that is translocated on the plasma membranes, but not expressed by the cells (e.g., the >2n phagocytic cells). The cell surface target may be a marker of the disease or condition to be detected.

The circulating vesicles may be isolated using chromatographic isolation, affinity isolation, or ultracentrifugation.

In certain aspects of the methods described herein, analytes include nucleic acids, proteins, lipids, carbohydrates, metabolites, or any combinations of these. In certain aspects of the methods described herein, markers include nucleic acids, proteins, lipids, carbohydrates, metabolites, or any combinations of these. As used herein, the term "nucleic acid" is intended to include DNA molecules (e.g., cDNA or genomic DNA), RNA molecules (e.g., mRNA), DNA-RNA hybrids, and analogs of the DNA or RNA generated using nucleotide analogs. The nucleic acid molecule can be a nucleotide, oligonucleotide, double-stranded DNA, single-stranded DNA, multi-stranded DNA, complementary DNA, genomic DNA, non-coding DNA, messenger RNA (mRNAs), microRNA (miRNAs), small nucleolar RNA (snoRNAs), ribosomal RNA (rRNA), transfer RNA (tRNA), small interfering RNA (siRNA), heterogeneous nuclear RNAs (hnRNA), or small hairpin RNA (shRNA). The nucleic acid may be a transrenal nucleic acid. A transrenal nucleic acid is an extracellular nucleic acid that is excreted in the urine, See, e.g., U.S. Patent Publication No. 20100068711 and U.S. Patent Publication No. 20120021404.

As used herein, the term "amino acid" includes organic compounds containing both a basic amino group and an acidic carboxyl group. Included within this term are natural amino acids (e.g., L-amino acids), modified and unusual amino acids (e.g., D-amino acids and β-amino acids), as well as amino acids which are known to occur biologically in free or combined form but usually do not occur in proteins. Natural protein occurring amino acids include alanine, arginine, asparagine, aspartic acid, cysteine, glutamic acid, glutamine, glycine, histidine, isoleucine, leucine, lysine, methionine, phenylalanine, serine, threonine, tyrosine, tryptophan, proline, and valine. Natural non-protein amino acids include arginosuccinic acid, citrulline, cysteine sulfuric acid, 3,4-dihydroxyphenylalanine, homocysteine, homoserine, ornithine, 3-monoiodotyrosine, 3,5-diiodotryosine, 3, 5, 5-triiodothyronine, and 3,3',5,5'- tetraiodothyronine. Modified or unusual amino acids include D-amino acids, hydroxylysine, 4-hydroxyproline, N-Cbz-protected amino acids, 2,4-diaminobutyric acid, homoarginine, norleucine, N-methylaminobutyric acid, naphthylalanine, phenylglycine, .alpha.-phenylproline, tert-leucine, 4-aminocyclohexylalanine, N-methyl-norleucine, 3 ,4-dehydroproline, N,N-dimethylaminoglycine, N-methylaminoglycine, 4-aminopiperidine-4-carboxylic acid, 6-aminocaproic acid, trans-4-(aminomethyl)-cyclohexanecarboxylic acid, 2-, 3-, and 4-(aminomethyl)- benzoic acid, 1-aminocyclopentanecarboxylic acid, 1-aminocyclopropanecarboxylic acid, and 2-benzyl-5-aminopentanoic acid.

As used herein, the term "peptide" includes compounds that consist of two or more amino acids that are linked by means of a peptide bond. Peptides may have a molecular weight of less than 10,000 Daltons, less than 5,000 Daltons, or less than 2,500 Daltons. The term "peptide" also includes compounds containing both peptide and non-peptide components, such as pseudopeptide or peptidomimetic residues or other non-amino acid components. Such compounds containing both peptide and non-peptide components may also be referred to as a "peptide analog."

As used herein, the term "protein" includes compounds that consist of amino acids arranged in a linear chain and joined together by peptide bonds between the carboxyl and amino groups of adjacent amino acid residues. Proteins used in methods described herein include, but are not limited to, amino acids, peptides, antibodies, antibody fragments, cytokines, lipoproteins, or glycoproteins.

As used herein, the term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, and antibody fragments (*e*.*g*., Fab or F(ab')₂, and Fv). For the structure and properties of the different classes of antibodies, see e.g., Basic and Clinical Immunology, 8th Edition, Daniel P. Sties, Abba I. Terr and Tristram G. Parsolw (eds), Appleton & Lange, Norwalk, Conn., 1994, page 71 and Chapter 6.

As used herein, the term "cytokine" refers to a secreted protein or active fragment or mutant thereof that modulates the activity of cells of the immune system. Examples of cytokines include, without limitation, interleukins, interferons, chemokines, tumor necrosis factors, colony-stimulating factors for immune cell precursors, and the like.

As used herein, the term "lipoprotein" includes negatively charged compositions that comprise a core of hydrophobic cholesteryl esters and triglyceride surrounded by a surface layer of amphipathic phospholipids with which free cholesterol and apolipoproteins are associated. Lipoproteins may be characterized by their density (e.g. very-low-density lipoprotein (VLDL), low-density lipoprotein (LDL) and high density lipoprotein (HDL)), which is determined by their size, the relative amounts of lipid and protein. Lipoproteins may also be characterized by the presence or absence of particular modifications (e.g. oxidization, acetylation, or glycation).

As used herein, the term "glycoprotein" includes glycosides which have one or more oligo- or polysaccharides covalently attached to a peptide or protein. Exemplary glycoproteins can include, without limitation, immunoglobulins, members of the major histocompatibility complex, collagens, mucins, glycoprotein IIb/IIIa, glycoprotein-41 (gp41) and glycoprotein-120 (gp12), follicle-stimulating hormone, alpha-fetoprotein, erythropoietin, transferrins, alkaline phosphatase, and lectins.

As used herein, the term "lipid" includes synthetic or naturally-occurring compounds which are generally amphipathic and biocompatible. Lipids typically comprise a hydrophilic component and a hydrophobic component. Exemplary lipids include, but are not limited to fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, sulfatide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, ceramide, low density lipoproteins (LDLs), very low density lipoproteins (VLDLs), high density lipoproteins (HDLs), sphingoid base-1-phosphates or derivatives thereof.

As used herein, the term "carbohydrate" includes, but is not limited to, compounds that contain oxygen, hydrogen and carbon atoms, typically (CH₂O)ₙ wherein n is an integer. Exemplary carbohydrates include, but are not limited to, monosaccharides, disaccharides, polysaccharides, or oligosaccharides.

As used herein, the term "metabolite" includes any molecule used in metabolism. Metabolites can be products, substrates, or intermediates in metabolic processes. Included within this term are primary metabolites, secondary metabolites, organic metabolites, or inorganic metabolites. Metabolites include, without limitation, amino acids, peptides, acylcarnitines, monosaccharides, lipids and phospholipids, prostaglandins, hydroxyeicosatetraenoic acids, hydroxyoctadecadienoic acids, steroids, bile acids, and glycolipids and phospholipids. Exemplary metabolites can be sphingolipids, glycosphingolipids, sphingosine, ceramide, sphingomyelin, sphingosylphosphorylcholin, dihydrosphingosine, phoshatidylcholine, phosphatidylinositol, phosphatidylserine, lysophoshatidylcholine, lysophosphatidylinositol, lysophosphatidylserine, plasmenylphoshatidylcholine, plasmanylphoshatidylcholine, proteinogenic amino acids, Alanine, Aspartic acid, Glutamic acid, Phenylalanine, Glycine, Histidine, Leucine, Isoleucine, Lysine, Methionine, Proline, Arginine, Serine, Threonine, Valine, Tryptophan, Tyrosine, asymmetrical dimethyl arginine, symmetrical dimethyl arginine, Glutamine, Asparagine, Nitrotyrosine, Hydroxyproline, Kynurenine, 3-Hydroxy kynurenine, non-proteinogenic amino acids, Ornithine, Citrulline, acylcarnitines, carnitine, free carnitine, acylcarnitine, hydroxylacylcarnitine, dicarboxylacylcarnitines, reducing monosaccharides, hexose, pentose, deoxyhexose, creatinine, creatine, spermidine spermine, putrescine, dopamine, serotonin, prostaglandins, hydoxyeicosatetraeneoic acid, Hydroxyoctadecadienoic acid, leukatrienes, thromboxanes, bile acids, sterols, cholesterols, vitamins and cofactors, drugs, and drug metabolites.

A sample may comprise one or more stabilizers for a cell or an analyte such as DNA, RNA, protein, and/or lipid. For example, a sample may comprise a DNA stabilizer, an RNA stabilizer, and/or a protein stabilizer. Stabilizers are well known in the art and include, for example, DNAse inhibitors, RNAse inhibitors, and protease inhibitors or equivalents thereof.

Profiles of at least one or more markers of a disease or condition may be compared. This comparison can be quantitative or qualitative. Quantitative measurements can be taken using any of the assays described herein. For example, sequencing, direct sequencing, random shotgun sequencing, Sanger dideoxy termination sequencing, targeted sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

Quantitative comparisons can include statistical analyses such as t-test, ANOVA, Krustal-Wallis, Wilcoxon, Mann-Whitney, and odds ratio. Quantitative differences can include differences in the levels of markers between profiles or differences in the numbers of markers present between profiles, and combinations thereof. Examples of levels of the markers can be, without limitation, gene expression levels, nucleic acid levels, protein levels, lipid levels, and the like. Qualitative differences can include, but are not limited to, activation and inactivation, protein degradation, nucleic acid degradation, and covalent modifications.

The profile may be a nucleic acid profile, a protein profile, a lipid profile, a carbohydrate profile, a metabolite profile, or a combination thereof. The profile can be qualitatively or quantitatively determined.

A nucleic acid profile can be, without limitation, a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

The nucleic acid profile can be determined by any methods known in the art to detect genotypes, single nucleotide polymorphisms, gene mutations, gene copy numbers, DNA methylation states, DNA acetylation states, chromosome dosages. Exemplary methods include, but are not limited to, polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, co-amplification at lower denaturation temperature-PCR (COLD-PCR), multiplex PCR, methyl-binding PCR analysis, or a combination thereof.

As used herein, the term "sequencing" is used in a broad sense and refers to any technique known in the art that allows the order of at least some consecutive nucleotides in at least part of a nucleic acid to be identified, including without limitation at least part of an extension product or a vector insert. Exemplary sequencing techniques include targeted sequencing, single molecule real-time sequencing, electron microscopy-based sequencing, transistor-mediated sequencing, direct sequencing, random shotgun sequencing, Sanger dideoxy termination sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof.. Sequencing may comprise an detecting the sequencing product using an instrument, for example but not limited to an ABI PRISM® 377 DNA Sequencer, an ABI PRISM® 310, 3100, 3100-Avant, 3730, or 373OxI Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. Sequencing may comprise emulsion PCR. Sequencing may comprise a high throughput sequencing technique, for example but not limited to, massively parallel signature sequencing (MPSS).

A protein profile can be a protein expression profile, a protein activation profile, or a combination thereof. A protein activation profile can comprise determining a phosphorylation state, an ubiquitination state, a myristoylation state, or a conformational state of the protein.

A protein profile can be detected by any methods known in the art for detecting protein expression levels, protein phosphorylation state, protein ubiquitination state, protein myristoylation state, or protein conformational state. A protein profile can be determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.

A lipid profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof. Further methods for analyzing lipid content in a biological sample are known in the art (See, e.g., Kang et al. (1992) Biochim. Biophys. Acta. 1128:267; Weylandt et al. (1996) Lipids 31 :977; J. Schiller et al. (1999) Anal. Biochem. 267:46; Kang et al. (2001) Proc. Natl. Acad. Sci. USA 98:4050; Schiller et al. (2004) Prog. Lipid Res. 43:499). One exemplary method of lipid analysis is to extract lipids from a biological sample (e.g. using chloroform-methanol (2:1, vol/vol) containing 0.005% butylated hydroxytoluene (BHT, as an antioxidant)), prepare fatty acid methyl esters (e.g., using 14% BF3-methanol reagent), and quantify the fatty acid methyl esters (e.g., by HPLC, TLC, by gas chromatography-mass spectroscopy using commercially available gas chromatographs, mass spectrometers, and/or combination gas chromatograph/mass spectrometers). Fatty acid mass is determined by comparing areas of various analyzed fatty acids to that of a fixed concentration of internal standard.

A carbohydrate profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

A metabolite profile can be determind by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

As used herein, the "difference" between different profiles detected by the methods described herein can refer to different gene copy numbers, different DNA, RNA, protein, lipid, or carbohydrate expression levels, different DNA methylation states, different DNA acetylation states, and different protein modification states. The difference can be a difference greater than 1 fold. The difference may be a 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference. In some embodiments, the difference is any fold difference between 1-10, 2-10, 5-10, 10-20, or 10-100 fold.

The difference is differential gene expression (DGE), e.g. DGE of phagocytes vs. non-phagocytes or >2n phagocytes vs. =2n phagocytes. DGE can be measured as X = log₂(Y_{P}) - log₂(Y_{NP}). The DGE may be any number, provided that it is significantly different between the combination sample and the =2n phagocytes, non-phagocytes, control cells, or repository of markers. For example, a 2-fold increased in gene expression could be represented as X = log₂(Y_{P}) - log₂(Y_{NP}) = log₂(Y_{P}/Y_{NP}) =log₂(2) = 1, while a 2-fold decrease in gene expression could be represented as X = log₂(Y_{P}) - log₂(Y_{NP}) = log₂(Y_{P}/Y_{NP}) =log₂(1/2) = -1. Down-regulated genes have X < 0, while up-regulated genes have X > 0. See, *e.g.,* Efron, J Am Stat Assoc 104:1015-1028 (2009).

A general principle of assays to detect markers involves preparing a sample or reaction mixture that may contain the marker (e.g., one or more of DNA, RNA, protein, polypeptide, carbohydrate, lipid, metabolite, and the like) and a probe under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one example of such a method, a sample from a subject, which is to be assayed for presence and/or concentration of marker, can be anchored onto a carrier or solid phase support. Also, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS(N-hydroxy-succinimide) using techniques known in the art (e.g., biotinylation kit, Pierce Chemicals, Rockford, IL), and immobilized in the wells of streptavidin-coated 96 well plates (Pierce Chemical). The surfaces with immobilized assay components can be prepared in advance and stored.

Other suitable carriers or solid phase supports for such assays include any material capable of binding the class of molecule to which the marker or probe belongs. Well known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In certain exemples, the probe, when it is the unanchored assay component, can be labeled for the purpose of detection and readout of the assay, either directly or indirectly, with detectable labels discussed herein and which are well-known to one skilled in the art.

It is also possible to directly detect marker/probe complex formation without further manipulation or labeling of either component (marker or probe), for example by utilizing the technique of fluorescence energy transfer (see, for example, U.S. Patent Nos. 5,631,169 and 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

Determination of the ability of a probe to recognize a marker can be accomplished without labeling either assay component (probe or marker) by utilizing a technology such as real-time Biomolecular Interaction Analysis (BIA) (see, e.g., Sjolander, S. and Urbaniczky, C, 1991, Anal. Chem. 63:2338 2345 and Szabo et al, 1995, Curr. Opin. Struct. Biol. 5:699 705). As used herein, "BIA" or "surface plasmon resonance" is a technology for studying biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas and Minton (1993) Trends Biochem. Sci. 18:284). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard (1998) J. Mol. Recognit. 11 :141; Hage and Tweed (1997) J. Chromatogr. B. Biomed. Sci. Appl. 12:499). Gel electrophoresis may also be employed to separate complexed assay components from unbound components (see, e.g., Ausubel et al, ed., Current Protocols in Molecular Biology, John Wiley & Sons, New York, 1987 1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In certain exemples, the level of mRNA corresponding to the marker can be determined either by *in situ* and/or by *in vitro* formats in a biological sample using methods known in the art. Many expression detection methods use isolated RNA. For in vitro methods, any RNA isolation technique that does not select against the isolation of mRNA can be utilized for the purification of RNA from blood cells (see, e.g., Ausubel et al, ed., Current Protocols in Molecular Biology, John Wiley & Sons, New York 1987 1999). Additionally, large numbers of cells and/or samples can readily be processed using techniques well known to those of skill in the art, such as, for example, the single-step RNA isolation process of Chomczynski (1989, U.S. Patent No. 4,843,155).

Isolated mRNA can be used in hybridization or amplification assays that include, but are not limited to, Southern or Northern analyses, polymerase chain reaction analyses and probe arrays. In certain exemples, a diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a marker described herein. Other suitable probes for use in the diagnostic assays are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array. A skilled artisan can readily adapt known mRNA detection methods for use in detecting the level of mRNA encoded by the markers described herein.

An alternative method for determining the level of mRNA corresponding to a marker described herein in a sample involves the process of nucleic acid amplification, e.g., by RT-PCR (the experimental embodiment set forth in U.S. Patent Nos. 4,683,195 and 4,683,202), COLD-PCR (Li et al. (2008) Nat. Med. 14:579), ligase chain reaction (Barany, 1991, Proc. Natl. Acad. Sci. USA, 88:189), self sustained sequence replication (Guatelli et al., 1990, Proc. Natl. Acad. Sci. USA 87:1874), transcriptional amplification system (Kwoh et al. (1989) Proc. Natl. Acad. Sci. USA 86:1173), Q- Beta Replicase (Lizardi et al. (1988) Bio/Technology 6:1197), rolling circle replication (U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the sample (*e.g.,* a bodily fluid (*e.g.,* blood cells)) prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, *e*.*g*., a housekeeping gene that is constitutively expressed. Suitable genes for normalization include housekeeping genes such as the actin gene, or epithelial cell-specific genes. This normalization allows the comparison of the expression level in a patient sample from one source to a patient sample from another source, e.g., to compare a combination sample from an individual to a =2n phagocytic or a non-phagocytic blood cell from the individual.

A protein or polypeptide corresponding to a marker may be detected. An agent for detecting a protein or polypeptide can be an antibody capable of binding to the polypeptide, such as an antibody with a detectable label. As used herein, the term "labeled," with regard to a probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. Antibodies can be polyclonal or monoclonal. An intact antibody, or a fragment thereof (*e*.*g*., Fab or F(ab')2) can be used. In one format, antibodies, or antibody fragments, can be used in methods such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well known supports or carriers include glass, polystyrene, polypropylene, polyethylene, dextran, nylon, amylases, natural and modified celluloses, polyacrylamides, gabbros, magnetite and the like.

A variety of formats can be employed to determine whether a sample contains a protein that binds to a given antibody. Examples of such formats include, but are not limited to, competitive and non-competitive immunoassay, enzyme immunoassay (EIA), radioimmunoassay (RIA), antigen capture assays, two-antibody sandwich assays, Western blot analysis, enzyme linked immunoabsorbant assay (ELISA), a planar array, a colorimetric assay, a chemiluminescent assay, a fluorescent assay, and the like. Immunoassays, including radioimmmunoassays and enzyme-linked immunoassays, are useful in the methods described herein. A skilled artisan can readily adapt known protein/antibody detection methods for use in determining whether cells (e.g., bodily fluid cells such as blood cells) express a marker described herein.

One skilled in the art will know many other suitable carriers for binding antibody or antigen, and will be able to adapt such support for use with the present disclosure. For example, protein isolated from cells (e.g., bodily fluid cells such as blood cells) can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In certain exemples, assays are provided for diagnosis, prognosis, assessing the risk of developing a disease, assessing the efficacy of a treatment, monitoring the progression or regression of a disease, and identifying a compound capable of ameliorating or treating a disease. An exemplary method for these methods involves obtaining a bodily fluid sample from a test subject and contacting the bodily fluid sample with a compound or an agent capable of detecting one or more of the markers of the disease or condition, *e.g.,* marker nucleic acid (*e.g.,* mRNA, genomic DNA), marker peptide (*e.g.,* polypeptide or protein), marker lipid (*e.g.,* cholesterol), or marker metabolite (*e*.*g*., creatinine) such that the presence of the marker is detected in the biological sample. An agent for detecting marker mRNA or genomic DNA may be a labeled nucleic acid probe capable of hybridizing to marker mRNA or genomic DNA. The nucleic acid probe can be, for example, a full-length marker nucleic acid or a portion thereof. Other suitable probes for use in the diagnostic assays described herein are described herein.

As used herein, a compound capable of ameliorating or treating a disease or condition can include, without limitations, any substance that can improve symptoms or prognosis, prevent progression of the disease or condition, promote regression of the disease or condition, or eliminate the disease or condition.

The methods described herein can also be used to detect genetic alterations in a marker gene, thereby determining if a subject with the altered gene is at risk for developing a disease and/or disorder associated with cancer and/or an infectious agent, and/or one or more other disorders described herein characterized by misregulation in a marker protein activity or nucleic acid expression, such as cancer. The methods may include detecting, in a cell free bodily fluid sample from the subject, the presence or absence of a genetic alteration characterized by an alteration affecting the integrity of a gene encoding a marker peptide and/or a marker gene. For example, such genetic alterations can be detected by ascertaining the existence of at least one of: 1) a deletion of one or more nucleotides from one or more marker genes; 2) an addition of one or more nucleotides to one or more marker genes; 3) a substitution of one or more nucleotides of one or more marker genes, 4) a chromosomal rearrangement of one or more marker genes; 5) an alteration in the level of a messenger RNA transcript of one or more marker genes; 6) aberrant modification of one or more marker genes, such as of the methylation pattern of the genomic DNA; 7) the presence of a non-wild type splicing pattern of a messenger RNA transcript of one or more marker genes; 8) a non-wild type level of a one or more marker proteins; 9) allelic loss of one or more marker genes; and 10) inappropriate post-translational modification of one or more marker proteins. As described herein, there are a large number of assays known in the art which can be used for detecting alterations in one or more marker genes.

Detection of the alteration may involve the use of a probe/primer in a polymerase chain reaction (PCR) (see, e.g., U.S. Patent Nos. 4,683,195, 4,683,202 and 5,854,033), such as real-time PCR, COLD-PCR (Li et al. (2008) Nat. Med. 14:579), anchor PCR, recursive PCR or RACE PCR, or, alternatively, in a ligation chain reaction (LCR) (see, e.g., Landegran et al. (1988) Science 241:1077; Prodromou and Pearl (1992) Protein Eng. 5:827; and Nakazawa et al. (1994) Proc. Natl. Acad. Sci. USA 91:360), the latter of which can be particularly useful for detecting point mutations in a marker gene (see Abravaya et al. (1995) Nucleic Acids Res. 23:675). This method can include the steps of collecting a sample of cell free bodily fluid from a subject, isolating nucleic acid (e.g., genomic, mRNA or both) from the sample, contacting the nucleic acid sample with one or more primers which specifically hybridize to a marker gene under conditions such that hybridization and amplification of the marker gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al., (1990) Proc. Natl. Acad. Sci. USA 87:1874), transcriptional amplification system (Kwoh et al., (1989) Proc. Natl. Acad. Sci. USA 86:1173), Q Beta Replicase (Lizardi et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

Alternatively, mutations in one or more marker genes from a sample can be identified by alterations in restriction enzyme cleavage patterns. For example, sample and control DNA is isolated, optionally amplified, digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Pat. No. 5,498,531) can be used to score for the presence of specific mutations by development or loss of a ribozyme cleavage site.

Also, genetic mutations in one or more of the markers described herein can be identified by hybridizing a sample and control nucleic acids, e.g., DNA or RNA, to high density arrays containing hundreds or thousands of oligonucleotides probes (Cronin et al. (1996) Human Mutation 7: 244; Kozal et al. (1996) Nature Medicine 2:753). For example, genetic mutations in a marker nucleic acid can be identified in two dimensional arrays containing light-generated DNA probes as described in Cronin, M. T. et al. supra. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

Yet, any of a variety of sequencing reactions known in the art can be used to directly sequence a marker gene and detect mutations by comparing the sequence of the sample marker gene with the corresponding wild-type (control) sequence. Examples of sequencing reactions include those based on techniques developed by Maxam and Gilbert ((1977) Proc. Natl. Acad. Sci. USA 74:560) or Sanger ((1977) Proc. Natl. Acad. Sci. USA 74:5463). It is also contemplated that any of a variety of automated sequencing procedures can be utilized when performing the diagnostic assays ((1995) Biotechniques 19:448), including sequencing by mass spectrometry (see, e.g., PCT International Publication No. WO 94/16101; Cohen et al. (1996) Adv. Chromatogr. 36:127-162; and Griffin et al. (1993) Appl. Biochem. Biotechnol. 38:147).

Other methods for detecting mutations in a marker gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes formed by hybridizing (labeled) RNA or DNA containing the wild-type marker sequence with potentially mutant RNA or DNA obtained from a tissue sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digesting the mismatched regions. Either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl. Acad. Sci. USA 85:4397; Saleeba et al. (1992) Methods Enzymol. 217:286. The control DNA or RNA can be labeled for detection.

The mismatch cleavage reaction may employ one or more proteins that recognize mismatched base pairs in double-stranded DNA (so called "DNA mismatch repair" enzymes) in defined systems for detecting and mapping point mutations in marker cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657). According to an exemple, a probe based on a marker sequence, e.g., a wild-type marker sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

Alterations in electrophoretic mobility may be used to identify mutations in marker genes. For example, single strand conformation polymorphism (SSCP) may be used to detect differences in electrophoretic mobility between mutant and wild type nucleic acids (Orita et al. (1989) Proc. Natl. Acad. Sci. USA 86:2766, see also Cotton (1993) Mutat. Res. 285:125; and Hayashi (1992) Genet. Anal. Tech. Appl. 9:73). Single-stranded DNA fragments of sample and control marker nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. The subject method may utilize heteroduplex analysis to separate double stranded heteroduplex molecules on the basis of changes in electrophoretic mobility (Keen et al. (1991) Trends Genet. 7:5).

The movement of mutant or wild-type fragments in polyacrylamide gels containing a gradient of denaturant may be assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. Further, a temperature gradient may be used in place of a denaturing gradient to identify differences in the mobility of control and sample DNA (Rosenbaum and Reissner (1987) Biophys. Chem. 265:12753).

Examples of other techniques for detecting point mutations include, but are not limited to, selective oligonucleotide hybridization, selective amplification or selective primer extension. For example, oligonucleotide primers may be prepared in which the known mutation is placed centrally and then hybridized to target DNA under conditions which permit hybridization only if a perfect match is found (Saiki et al. (1986) Nature 324:163; Saiki et al. (1989) Proc. Natl. Acad. Sci. USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant disclosure. Oligonucleotides used as primers for specific amplification may carry the mutation of interest in the center of the molecule (so that amplification depends on differential hybridization) (Gibbs et al. (1989) Nucl. Acids Res. 17:2437) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain instances amplification may also be performed using Taq ligase for amplification (Barany (1991) Proc. Natl. Acad. Sci. USA 88:189). In such cases, ligation will occur only if there is a perfect match at the 3' end of the 5' sequence making it possible to detect the presence of a known mutation at a specific site by looking for the presence or absence of amplification.

This disclosure also provides a method for identifying one or more markers of a disease or condition comprising: a) determining a first profile of analytes from a sample comprising a cell-free bodily fluid from a subject having said disease or condition and a population of phagocytic cells, or a population of >2n phagocytic cells, from a subject having said disease or condition; determining a second profile of analytes from a population of =2n phagocytic cells, or a population of non-phagocytic cells, from the subject having said disease or condition; identifying a set of differences between the first and second profiles, wherein the first set of differences is specific to the first profile relative to the second profile; b) determining a third profile of analytes from a sample comprising a cell-free bodily fluid from a subject having said disease or condition and a population of phagocytic cells, or a population of >2n phagocytic cells, from a control subject not having said disease or condition; determining a fourth profile of analytes from a population of =2n phagocytic cells, or a population of non-phagocytic cells, from the control subject not having said disease or condition; identifying a set of differences between the third and fourth profiles, wherein the second set of differences is specific to the third profile relative to the fourth profile; and c) identifying one or more analytes specific to the set of differences identified in a) relative to the set of differences identified in b), the identified analytes in c) being markers of said disease or condition. This disclosure also provides a method for identifying one or more markers of a disease or condition comprising: a) determining a first profile of analytes from a sample comprising a cell-free bodily fluid from a subject having said disease or condition and a population of phagocytic cells, or a population of >2n phagocytic cells, from a subject having said disease or condition; b) comparing the first profile to a second profile derived from a repository of analytes from a control subject not having said disease or condition; c) identifying a set of differences between the first and second profiles, wherein the set of differences is specific to the first profile relative to the second profile; and d) identifying one or more analytes specific to the set of differences, the identified analytes being markers of said disease or condition. Further, the method may further comprise: e) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a set of differences between the fifth and sixth profiles, wherein the set of differences is specific to the fifth profile relative to the sixth profile; and f) identifying at least one of the one or more markers of c) present in the set of differences identified in d). This disclosure also provides a method for identifying one or more markers that may be used in the treatment of a disease or condition. For example, a marker (e.g., protein or gene) identified by a method described herein may be used as a molecular target for a therapeutic agent. A marker identified by a method described herein also may be used in any of the other methods described herein, e.g., for monitoring the progression or regression of a disease or condition. The one or more markers identified by the methods described herein may have therapeutic potential. For example, if a marker is identified as being up-regulated (or down-regulated) in circulating diseased cells from a subject having a disease or condition , a compound or an agent that is capable of down-regulating (up-regulating) said marker may be useful in treating said disease or condition. Similarly, a gene/protein/lipid/carbohydrate expression profile, a single nucleotide polymorphism profile, a gene mutation profile, a gene copy number profile, a DNA methylation profile, a DNA acetylation profile, a chromosome dosage profile, a gene expression profile, or a combination thereof may be useful in these methods.

An exemplary method for detecting the presence or absence of an analyte *(e.g.,* DNA, RNA, protein, polypeptide, carbohydrate, lipid or the like) corresponding to a marker described herein in a biological sample involves obtaining a bodily fluid sample (e.g., blood) from a test subject and contacting the bodily fluid sample with a compound or an agent capable of detecting one or more markers. Detection methods described herein can be used to detect one or more markers in a biological sample *in vitro* as well as *in vivo.* For example, *in vitro* techniques for detection of mRNA include Northern hybridizations and in situ hybridizations. *In vitro* techniques for detection of a polypeptide corresponding to a marker described herein include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of a polypeptide corresponding to a marker described herein include introducing into a subject a labeled antibody directed against the polypeptide. For example, the antibody can be labeled with a radioactive marker whose presence and location in a subject can be detected by standard imaging techniques. Because each marker is also an analyte, any method described herein to detect the presence or absence of a marker can also be used to detect the presence or absence of an analyte.

The marker that is useful in the methods described herein can include any mutation in any one of the above-identified markers. Mutation sites and sequences can be identified, for example, by databases or repositories of such information, e.g., The Human Gene Mutation Database (www.hgmd.cf.ac.uk), the Single Nucleotide Polymorphism Database (dbSNP, www.ncbi.nlm.nih.gov/projects/SNP), and the Online Mendelian Inheritance in Man (OMIM) website (www.ncbi.nlm.nih.gov/omim).

A marker that is useful in the methods described herein can include any marker that is known to be associated with a disease or condition. Markers that can be used herein can be any marker that has been well-characterized as associated with a specific disease or condition, or any markers that have been identified by the methods described herein.

The markers may comprise at least one gene selected from the group consisting of AKT2, BAK1, EGFR, ERBB2, ETS2, FOS, JUN, MAP2K1, MMP2, PDGFB, RB1, SERPINB2, SNCG, and SPP1. The one or more markers may comprise at least one gene selected from the group consisting of AKT1, AKT2, BAK2, CDC25A, E2F1, EGFR, ERBB2, FOS, JUN, MAP2K1, MMP2, NFKB1, PDGFB, PIK3R1, PNN, RB1, SERPINB2, SERPINB5, SNCG, SPP1, TERT, TIMP3, and TP53. The one or more markers may comprise at least one gene selected from the group consisting of CASP8, CASP9, COL18A1, ETS2, HTATIP2, MMP9, SRC, and TWIST1. The one or more markers may comprise at least one gene selected from the group consisting of AKT1, APAF1, ATM, CDC25A, CDKN1A, ETS2, FOS, IL8, ITGA4, ITGA6, ITGAV, JUN, MAP2K1, NFKBIA, PLAU, PLAUR, RAF1, SERPINB2, SYK, TIMP1, TNF, TNFRSF10B, and TNFRSF1A. The markers may comprise at least one gene selected from the group consisting of ACP2, AK2, AKT3, ARL5B, ATP2B3, BGN, BRAF, BTG2, CAMKK2, CAPG, CAPN12, CPLX2, DENND5A, DNA2, FAM104A, FNIP1, GFRA4, GLUD1, GNAQ, GP1BB, HNRPLL, HOXA2, HPS3, INPP4A, ITGAV, KLHL23, LANCL2, LYPD6, MAPKAPK3, MEF2A (includes, EG:4205), MEF2C, NVL, PCYT1A, PGLYRP4, PLOD1, PPP1CB, PRKAB2, PROS1, PTPRE, RASA4 (includes,EG:10156), RBMS2, RBPJ, STAT5B, THBS1, TRIB1, TRIM2, TSPAN6, and ZDHHC21. The markers may comprise at least one gene selected from the group consisting of B4GALT5, BOP1, CCL2, CCL3, CCL3L1, CCRL2, CD83, CLEC4G, CLIC4, CTSC, CTSO, CXCL10, FCGR3A, FPR3, HBA1, HBB, LRMP, MAP1LC3B2, MS4A4A, MSR1, MYADML, NIDI, PF4, PION, RNF217, SAMD9L, SERPING1, and SPARC. The markers may comprise at least one gene selected from the group consisting of ACOT9, AMPD2, ARHGAP15, BATF2, C3AR1, C5orf41, CCL3, CCL3L1, CD63, CHST11, CHSY1, CLEC4G, CTSZ, CXorf21, CYTH4, CYTIP, DLEU2, DNAJA1, DOCK8, DTX3L, DUSP6, EPSTI1, ERF, F2RL1, FYB, GABRB2, GBP5, GLRX, GNB4, ICAM1, IFI35, IFIH1, IFNAR2, IL1R1, IRF1, ITGA5, LAP3, LAPTM5, LCP2, MAP1LC3B, MAP1LC3B2, MICAL2, MT1DP, MT1JP, MT1M, MT2A, MYADML, NEK6, NINJ2, NNMT, NT5C3L, NUB 1, PDE4B, PLOD1, PML, PRKCB, PSMB9, RCN3, RGS4, RNASE6, RTP4, SAMD9L, SEL1L, SERPING1, SETX, SIGLEC10, SKIL, SLC7A7, SNORA21, SP100, SP110, SP140, SSFA2, STAT2, STK17B, STK3, TDRD7, TMCC1, TMPRSS11E2, TNFRSF1B, TPM1, TRIM21,TXNDC4, UBE2L6, UBE2W, USP18, VAV1, WARS, WIPF1, and WIPI1. The markers may comprise at least one gene selected from the group consisting of ADAR, ADM, ALAS1, ANKRD22, ARHGAP27, B3GNT5, BCL10, C12orf35, C15orf29, C2orf59, CD177, CEACAM1, CPEB2, DDX58, F2RL1, GDPD3, GNAI3, HIST2H3A, HIST2H3D, HIST2H4A, HMGCR, HSPA6, HSPC159, IL4R, IMPA2, KPNB1, KREMEN1, KRT23, LDLR, LOC100130904, LTB4R, MAEA, MARK2, MBOAT2, MPZL3, N4BP1, NBEAL2, NMI, NPEPPS, PARP14, PGM2, PPIF, PXN, RALBP1, ROD1, RPS6KA1, S100P, SERTAD2, SLC9A1, SLPI, SP110, SPINT1, ST14, TBC1D3, TNFRSF9, TRIM21, UPP1, VPS24, ZBTB34, and ZNF256.

The markers may comprise at least one biomarker selected from the group consisting of ACTN4, BC020163, CMIP, CNN2, EDNRB, GPM6B, KIT, MGC40222, NAMPT, PRAME, RPL18, RPL21, RPS15, TMEM80, TRIB2, TTC3, and VDAC1. The markers may be ACTN4, BC020163, CMIP, CNN2, EDNRB, GPM6B, KIT, MGC40222, NAMPT, PRAME, RPL18, RPL21, RPS15, TMEM80, TRIB2, TTC3, and VDAC1. These markers are useful in the diagnosis, prognosis, or monitoring of melanoma, or discriminating between different types of skin lesions, for example, melanoma and naevi (See, e.g., Wachsman et al., "Noninvasive genomic detection of melanoma," Br J Dermatol. 2011 Apr;164(4):797-806).

The marker that is useful in the methods described herein for prenatal or pregnancy-related diseases or conditions may include those disclosed in, for example, United States Patents 7,655,399, 7,651,838, 6,660,477, 6,172,198, 5,594,637, 5,514,598, 6,258,540, 6,664,056, 7,235,359, and 7,645,576, United States Patent Application Publications 20090162842, 20090155776, 20070207466, 20060019278, 20040086864, 20020045176, 20010051341, 20020192642, 20040009518, 20040203037, 20050282185, 20060252071, 20070275402, 20080153090, 20090170102, 20090061425, 20020045176, 20040137452, 20050164241, 20060019278, 20060252068, 20060252071, 20060257901, 20070141625, 20070218469, 20070275402, 20090155776, 20090162842, 20090170102, 20090317797, 20100120056, 20100120076, and 20100137263 and International Patent Application Publications WO/2006/026020, WO/2002/068685, WO/2005/111626, WO/2009/055487, WO/2009/001392, and WO/2008/014516.

The marker that is useful in the methods described herein for neurological or neuropsychiatric diseases or conditions include those disclosed in, for example in United States Patents 7,723,117, 6,867,236, United States Patent Application Publications 20060115854, 20060115855, 20060166283, 20060234301, 20060259990, 20060259991, 20070162983, 20070264197, 20080026405, 20080038730, 20080051334, 20080152589, 20080220013, 20080261226, 20080269103, 20080286263, 20090041862, 20090239241, 20090275046, 20090318354, 20090324611, 20100009352, 20100021929, 20100028356, 20100055722, 20100062463, 20100075891, 20100105623, 20100124756, 20100159486, 20100167937, 20100169988, 20100167320, 20100112587, 20100098705, 20100068705, 20100009356, 20090305265, 20100124746, 20100092983, 20070148661, 20070141625, 20100120050, 20090155230, 20090274709, International Patent Application Publications WO/2004/040016, WO/2004/071269, WO/2005/033341, WO/2005/052592, WO/2005/103712, WO/2005/114222, WO/2006/020269, WO/2006/048778, WO/2006/050475, WO/2006/061609, WO/2006/105907, WO/2006/133423, WO/2006/134390, WO/2007/098585, WO/2007/119179, WO/2008/010660, WO/2008/014314, WO/2008/028257, WO/2008/046509, WO/2008/046510, WO/2008/046511, WO/2008/046512, WO/2008/063369, WO/2008/085035, WO/2008/095261, WO/2008/100596, WO/2008/120684, WO/2008/125651, WO/2008/127317, WO/2008/132464, WO/2009/000520, WO/2009/001392, WO/2009/068591, WO/2009/074331, WO/2009/100131, WO/2010/005750, WO/2010/011506, WO/2010/019553, WO/2010/059242, WO/2010/061283, WO/2010/063009, WO/2010/066000, WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/07556.

The marker that is useful in the methods described herein for cardiovascular diseases or conditions may include those disclosed in, for example in United States Patents 7,670,769, 7,445,886, 7,432,107, 7,157,235, and 7,009,038, United States Patent Application Publications 20100167320, 20100112587, 20100098705, 20100068705, 20100009356, 20090305265, 20100124746, 20100092983, 20070148661, 20070141625, 20100120050, 20090155230, and 20090274709, and International Patent Application Publications WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/075566.

The marker that is useful in the methods described herein for kidney-associated diseases or conditions may include those disclosed in, for example in United States Patents 7,488,584, 7,459,280, 7,294,465, and 7,662,578, United States Patent Application Publications 20100143951, 20100124746, 20100120056, 20100120041, 20100081142, 20090155230, and 20090239242, International Patent Application Publications WO/2010/059996, WO/2010/054389, WO/2010/048347, WO/2010/048497, WO/2010/054167, WO/2010/048346, WO/2010/046137, WO/2010/025434, WO/2010/018185, WO/2010/012306, WO/2009/122387, WO/2009/083950, WO/2009/080780, WO/2009/060035, WO/2009/059259, WO/2008/154238, WO/2008/089936, WO/2008/084331, WO/2008/042012, WO/2007/131345, WO/2005/012907, WO/2004/024098, WO/2003/019193, WO/2007/112999, WO/2007/082733, WO/2006/073941, WO/2010/068686, WO/2010/022210, and WO/2009/127644.

The marker that is useful in the described herein for autoimmune or immune-related diseases or conditions may include those disclosed in, for example 7,604,948, 7,670,764, 6,986,995, and 6,631,330, United States Patent Application Publication 20070141625, 20090263474, 20100075891, 20100104579, 20100105086, 20100131286, 20090176217, 20090202469, 20020119118, 20090258025, 20100137393, 20100120629, 20090318392, 20090196927, 20090023166, 20080227709, 20080039402, 20080026378, 20070224638, 20070218519, 20060210562, 20050266432, 20050164233, 20050130245, 20090130683, 20090110667, 20090054321, 20090023166, and 20080274118, and International Patent Application Publication WO/2009/043848, WO/2010/053587, WO/2010/046503, WO/2010/039714, WO/2009/100342, WO/2009/053537, WO/2009/017444, WO/20081156867, WO/2008/147938, WO/2008/129296, WO/2008/137835, WO/2008/082519, WO/2008/064336, WO/2008/043782, WO/2008/043725, WO/2007/047907, WO/2006/125117, WO/2006/114661, WO/2006/020899, WO/2005/114222, WO/2005/007836, WO/2004/076639, WO/2004/050704, and WO/2001/014881.

Also provided herein are kits that comprise marker detection agents that detect at least one or more of the markers identified by the methods described herein. Also provided herein are methods of treating or preventing a disease or condition in a subject comprising administering to said subject an agent that modulates the activity or expression or disrupts the function of at least one or more of the markers identified by the methods described herein.

The following examples are set forth as being representative of the present invention. These examples are not to be construed as limiting the scope of the invention as these and other equivalent embodiments will be apparent in view of the present disclosure and accompanying claims.

### Examples

Representative Method I for the Separation of Phagocytic Cells with DNA Content of 2n from Non-Phagocytic Cells and the Analysis of Expression Profiles
1. Separate blood sample into plasma and buffy coat including WBC sample. Coat plates to receive WBC sample with avidin.
2. Add biotinylated antibody to non-phagocytic blood cell (e.g., T cells) to the wells, incubate for 30 min at RT, wash wells.
3. Add magnetic beads.
4. Add WBC blood sample.
5. Incubate at 37 °C (30 minutes - 1 hour).
6. Following phagocytosis of beads by phagocytic cells and binding of avidin-biotin-antibody to non-phagocytic cells, place plate on top of magnet and wash (the phagocytic cells that internalized the magnetic beads and the non-phagocytic cells bound to the antibody will stay; all other cells will be washed away).
7. Remove magnet and collect phagocytic cells and separate into phagocytic cells with DNA equal to 2n and DNA greater than 2n. Non-phagocytes and phagocytes having DNA equal to 2n are referred to as cells having DNA equal to 2n.

### EXAMPLE 2

Representative Method II for the Separation of Phagocytic Cells from Non-Phagocytic Cells
1. Separate blood sample into plasma and buffy coat including WBC sample.
2. Cytospin WBC on glass slides.
3. Fix cells in acetone/methanol (-20 °C for 5 minutes).
4. Stain with hematoxylin and eosin stain and anti-T cell antibody.
5. Isolate T cells (non-phagocytic) and macrophages (phagocytic) using laser capture microscopy (LCM). Separate into phagocytic cells with DNA equal to 2n and DNA greater than 2n. Non-phagocytes and phagocytes having DNA equal to 2n are referred to as cells having DNA equal to 2n.

### EXAMPLE 3

Representative Method III for the Separation of Phagocytic Cells from Non-Phagocytic Cells
1. Separate plasma from whole blood.
2. Use magnetic antibody-conjugated beads to isolate non-phagocytic (e.g., T cells) and phagocytic cells (e.g., neutrophils and/or macrophages and/or monocytes) from whole blood. Separate into phagocytic cells with DNA equal to 2n and DNA greater than 2n. Non-phagocytes and phagocytes having DNA equal to 2n are referred to as cells having DNA equal to 2n.

### EXAMPLE 4

Representative Method IV for the Separation of Phagocytic Cells from Non-Phagocytic Cells and the Analysis of Expression Profiles
1. Separate blood sample into plasma and buffy coat including WBC sample. Stain WBC with fluorescent antibodies specific against a particular cell subpopulation (e.g., neutrophils, macrophages, monocytes, T cells and the like) and a DNA stain, (e.g., Hoechst 33342, Propidium iodide).
2. Sort the cells (e.g., by FACS).

### EXAMPLE 5

Representative Method for the Analysis of Expression Profiles
1. Create a combination sample by combining two or more different components selected from: a cell-free bodily fluid isolated from a subject, phagocytic cells isolated from the subject, >2n phagocytic cells isolated from the subject, circulating vesicles isolated from the subject, and circulating diseased cells isolated from the subject.
2. Isolate RNA from the combination sample and from a control sample with a component selected from: =2n phagocytic cells isolated from the subject, non-phagocytic cells isolated from the subject, and control cells isolated from the subject. Prepare cDNA or cRNA and use to differentiate genetic profiles (e.g., a cancer gene array) between the combination sample and the control sample.
3. Isolate DNA from the combination sample and from the control sample. Run DNA arrays and compare the profiles obtained from the combination sample and the control sample.
4. Isolate protein from the combination sample and from the control sample. Run Western blots using antibodies to known proteins overexpressed by human tumors (e.g., PSA and PSMA in prostate cancer; CEA in colon cancer; and CA125 in ovarian cancer), and compare the profiles obtained from the combination sample and the control sample.
5. Isolate lipids from the combination sample and from the control sample. Compare quantity and quality of lipids, for example using HPLC, between the combination sample and the control sample.

### EXAMPLE 6

A fraction of pregnancies are complicated by the presence of genetic abnormalities in the fetus, and considerable clinical interest has focused on development of assays to be able to detect and characterize these abnormalities. Among the most common problems is the presence of chromosomal aneuploidies in which one or more of the chromosomes is present at other than the normal abundance. The incidence of aneuploidies increase in frequency with maternal age and encompass potentially viable pregnancies with deviations from diploid number of chromosomes 13, 18 and 21, or abnormal numbers of X chromosomes in either male or female fetuses. Detection of these aneuploid conditions can occur by direct analysis of fetal cells collected by amniocentesis or chorionic villus sampling (CVS) and using traditional cytogenetic karyotyping or array comparative genome hybridization (CGH), quantitative PCR (qPCR) or sequencing of fetal genomic DNA, but these approaches carry a risk of fetal or maternal morbidity and miscarriage as a consequence of the trauma of cell collection. Additionally, these invasive cell collection procedures are only effective when applied late in the first trimester of pregnancies, and earlier genotyping is preferred to enable earlier and better decisions on at risk pregnancies.

In order to avoid the morbidity risks of invasive testing, non-invasive procedures have been developed to detect fetal aneuploidies by analyzing the sequence composition of degraded genomic fetal DNA present at low levels in maternal blood, also known as circulating cell-free fetal DNA (ccff DNA). However, these procedures rely on the exclusion of intact maternal genomic DNA from cellular components of blood to increase the observable signals that the procedures rely upon. By contrast, other methods use purely cellular fractions, but these methods may have high levels of maternal cell contamination (Bianchi et al., Proc Natl Acad Sci USA. 87(9):3279-3283 (1990); Bianchi et al., Am J Hum Genet. 61(4):822-829 (1997)).

In contrast to the methods mentioned above, and as proof-of-principle of the methods of the invention, an exemplary method was developed that harnesses the power of procedures reliant on cfff DNA and procedures reliant on cellular fractions alone. Here, the approach explicitly includes a cell-free bodily fluid and phagocytic blood cells expected to contain fetal genomic markers scavenged by phagocytosis of fetal cells in the placenta and other compartments. Evidence of fetal DNA existing in a maternal sample is provided by the presence of paternal markers, which are DNA sequences that do not exist in the mother and are novel when comparing a strictly maternal DNA sample to a combination sample of maternal and fetal DNA. In practice, paternal markers may be seen by DNA sequencing analyses in selected segments of the human genome which contain polymorphic sites. Polymorphic sites are often single nucleotide polymorphisms (SNPs), and distinguish alleles. Markers of this type are informative when the mother is homozygous for a SNP sequence and the father has a variant SNP passed to the fetus. For example, if the mother carries A/A nucleotides at a particular SNP location, and the father carries G/G nucleotides at that same SNP location, the G allele of the SNP will be found at a proportion indicating the relative amount of fetal DNA in a sample. Using sequencing techniques, the proportion of fetal material may be calculated by comparing the number of "A" bearing molecules (the maternal-derived material) to the number of "G" molecules (the fetal-derived material). Exemplary assays for calculating fetal DNA content in a sample using this rationale are described in, for example, Chiu et al., Proc Natl Acad Sci USA 105:20458-63 (2008); Ehrich et al., Am J Obstet Gynecol 204:205 e201-211 (2011); and Sehnert et al., Clin Chem 57:1042-9 (2011), each of which is herein incorporated by reference in its entirety. Calculation of fetal DNA content is accurate, as exemplified by Tynan et al. (J Mol Diagn 13(4):382-9 (2011)) in a study indicating that the confidence level of correctly detecting the presence of fetal DNA in a combination sample with 6 or more informative SNPs was greater than 99.9%. Where the mother is homozygous, the fetal fraction is seen directly as the variant fraction, while if the mother is heterozygous the fetal allelic fraction is added to the maternal, and may distort the observed ratio if the fetus is homozygous. These cases undoubtedly exist in the data shown, but have not been analyzed to simplify the analysis.

To produce an unbiased panel of potentially informative SNPs, criteria were selected such that all populations should carry the reference and alternative SNPs in similar proportions and the minor allele frequency (MAF) should be greater than 40%. Catalogs of SNPs having these qualities are commercially available (Durbin et al., Nature 467(7319):1061-1073 (2010) and McVean et al., Nature 491:56-65 (2012)). MAFs are the frequencies at which the least common allele for a given gene occurs in a population. MAFs of greater than 40% were targeted to increase the likelihood of finding heterozygotes in the fetal fraction, while at the same time having abundant homozygotes in the maternal fraction, thus increasing the likelihood of finding informative SNPs. An initial set of 167 markers was chosen for analysis. Based on previous analyses, for any mother-fetus combination sample (e.g., a cell-free bodily fluid in combination with phagocytes), 10-20% of the panel SNPs would be expected to be informative. PCR primers were designed that contained the 167 SNP markers and were of a small enough size (80-120 bp) so that the probability of amplification from partially degraded DNA would be greatest. The list of SNPs and their genomic references and the primer sequences used to amplify them are provided in Table 1.

**Table 1: SNPs and genomic references**

| **GenBan k Target ID** | **Target Interval** | **Left Primer With Tag** | **Right Primer With Tag** |
|---|---|---|---|
| rs2937436.G.A | chr5:313 25718-3132572 8 | ACACTGACGACATGGTTCTACAA AAGAAAGGTATCATCTGAAGTT | TACGGTAGCAGAGACTTGGTCTT AGAACTACTTACTTTTACTTATTA GGA |
| rs6946634.A.G | chr7:790 93092-7909310 2 | ACACTGACGACATGGTTCTACAG GATTTTAAAATGAAAATTGAAGA AGTA | TACGGTAGCAGAGACTTGGTCTT GTTGTTCTTTGGTCTGTAAAAT |
| rs1060922.T.C | chr18:72 2398-722408 | ACACTGACGACATGGTTCTACATT AGTACCAGAACCACTGC | TACGGTAGCAGAGACTTGGTCTA TTTACCTGATTTTAATCATCAGAT |
| rs11734833.A.G | chr4:101 108065-1011080 75 | ACACTGACGACATGGTTCTACAA CAGGTATTCATCATTCACTC | TACGGTAGCAGAGACTTGGTCTG TTTCCCCTGTTCTTAAGTG |
| rs10490392.G.T | chr2:186 658561-1866585 71 | ACACTGACGACATGGTTCTACAA TCAGCTTATGCTGATGATAATC | TACGGTAGCAGAGACTTGGTCTA AAATAGTTTTAGTAATGAAGTTA GCA |
| rs1872575.G.A | chr3:113804975-113804985 | ACACTGACGACATGGTTCTACATT AAATGACCTGGATTGATCAG | TACGGTAGCAGAGACTTGGTCTC TTTTGTCTTTGACTAAATGAATCT |
| rs4241779.A.G | chr4:184600597-184600607 | ACACTGACGACATGGTTCTACAA TTGGATGCAATTGCTCAG | TACGGTAGCAGAGACTTGGTCTA CTGTTTAGACATCCATGCA |
| rs1958716.A.G | chr14:20 528317-2052832 7 | ACACTGACGACATGGTTCTACAG GAACTTCAAATTTTCTTCTTTG | TACGGTAGCAGAGACTTGGTCTA GGGTTGACCTACATGTC |
| rs3085724.TCA.T | chr3:156255511-156255523 | ACACTGACGACATGGTTCTACATT TGAGCTTTACAAATAAACATACA | TACGGTAGCAGAGACTTGGTCTA TCCCAGTGCTAATTAAACAA |
| rs5880678.TC.T | chr6:146756123-146756134 | ACACTGACGACATGGTTCTACAG CTGCCTTAAGTAGGAAGA | TACGGTAGCAGAGACTTGGTCTG TGCTTTGTCAGAATTCCC |
| rs10741130.T.C | chr10:27475440-27475450 | ACACTGACGACATGGTTCTACAG AAACAGATACCTCCTATTTTGA | TACGGTAGCAGAGACTTGGTCTC AAGGCTAGACTAAAAGGAAAA |
| rs2305641.G.A | chr12:69646910-69646920 | ACACTGACGACATGGTTCTACATT CATTCTTTGGGAGTAAATGA | TACGGTAGCAGAGACTTGGTCTC TGTATCACTAGCTACTAAATCAA AA |
| rs1042201.G.A | chr3:151 546037-151546047 | ACACTGACGACATGGTTCTACAA | TACGGTAGCAGAGACTTGGTCTA |
| | | AAACCTCAGAAAATTTGCATT | AAACAGAAAAGTGCCTATTTAAG |
| rs3806.G.A | chrl3:103330714-103330724 | ACACTGACGACATGGTTCTACAG CGTATTCTAAAATAGGAAACAAG | TACGGTAGCAGAGACTTGGTCTA TAGTGGATGAAAACATGCATT |
| rs9307834.A.C | chr4:76453846-76453856 | ACACTGACGACATGGTTCTACAG TATTTAAGAATTTTCTGTTTTAAT GC | TACGGTAGCAGAGACTTGGTCTG TGGGTTAAATCCCATTTAGA |
| rs6919254.T.C | chr6:136599400-136599410 | ACACTGACGACATGGTTCTACAC ATAAGCTGAAAGGCCAG | TACGGTAGCAGAGACTTGGTCTA TGACCCATCTGAGTCTATC |
| rs1338.T.C | chr10:27475689-27475699 | ACACTGACGACATGGTTCTACAA GTTAAAAGGCTGAAAGGAATA | TACGGTAGCAGAGACTTGGTCTA GTTTTCATAAAGTGCAATAAACA |
| rs2999278.C.T | chr10:44053581-44053591 | ACACTGACGACATGGTTCTACAA GGAGGAAAGCTCATGTAA | TACGGTAGCAGAGACTTGGTCTA AGTCTACCCTTTGTTCTTAC |
| rs60029104.G.A | chr2:186658052-186658062 | ACACTGACGACATGGTTCTACAT ATCACGTAAAGGCAAATGT | TACGGTAGCAGAGACTTGGTCTT GAAGTTGAAGTACTTTTCGATA |
| rs1862066.G.A | chr2:186671908-186671918 | ACACTGACGACATGGTTCTACAA GAAAATTTAGCAAGAAGACTAAC | TACGGTAGCAGAGACTTGGTCTT GAAACTGAAACCTCATCAC |
| rs4518168.G.A | chr3:978 06612-9780662 2 | ACACTGACGACATGGTTCTACAG TTCAAAATTTCATGCAATGGT | TACGGTAGCAGAGACTTGGTCTA GTCATTAAAGTGGGTATTTGTA |
| rs10816767.G.A | chr9:111782666-111782676 | ACACTGACGACATGGTTCTACAG CATTCTGTATGTAAGGCC | TACGGTAGCAGAGACTTGGTCTG GAAGTTCAAATTTGACAGC |
| rs7138345.G.A | chr12:104290853-104290863 | ACACTGACGACATGGTTCTACAT AAACTGATGGAAGCAATGAAT | TACGGTAGCAGAGACTTGGTCTG TAGGTACTCAGCTTGAAAC |
| rs6917033.G.A | chr6:42627430-42627440 | ACACTGACGACATGGTTCTACAC ATAACCTTTGTATTGCAGCC | TACGGTAGCAGAGACTTGGTCTA TACATGTAACGAATTGTCTTTG |
| rs115032519.T.C | chr6:30002877-30002887 | ACACTGACGACATGGTTCTACACT ATTGCCTGGATCCCAT | TACGGTAGCAGAGACTTGGTCTA AGCTTAGGCTTTCAGATTTTAC |
| rs3747562.A.G | chr12:42854201-42854211 | ACACTGACGACATGGTTCTACAC CGGATTTCAATGTCATAGTTC | TACGGTAGCAGAGACTTGGTCTG TACATCTGCCAGTGCTC |
| rs2240859.A.G | chr3:42787465-42787475 | ACACTGACGACATGGTTCTACAG AATTTGTTGAGCCCGAC | TACGGTAGCAGAGACTTGGTCTT ATTTCAGGTTTTGGCCAGA |
| rs1050287.T.C | chr12:27954503-27954513 | ACACTGACGACATGGTTCTACAA GCATAGCAATGTAAAAGGAAT | TACGGTAGCAGAGACTTGGTCTA CTCATTCATAATGCCTCCTTA |
| rs1573612.T.C | chr12:12047425-12047435 | ACACTGACGACATGGTTCTACAG CATCACCAATGTTCCAA | TACGGTAGCAGAGACTTGGTCTG AGGGTTCCAAACATACTG |
| rs1230381.A.G | chr5:96254205-96254215 | ACACTGACGACATGGTTCTACATT ATCAAGAAGGCCAGGAA | TACGGTAGCAGAGACTTGGTCTG ACCATTCATTTGGAATGCT |
| rs9880307.C.A | chr3:16974606-16974616 | ACACTGACGACATGGTTCTACATC TAAGAAAATGAAACGTACCT | TACGGTAGCAGAGACTTGGTCTG TCCTTCACTCAAATTTCCT |
| rs3800989.A.G | chr7:141360581-141360591 | ACACTGACGACATGGTTCTACAA ACCATTACTTGACAGAAAGG | TACGGTAGCAGAGACTTGGTCTG TTGAAGCCTCACCTTTTAT |
| rs28394363.G.A | chr3:33428295-33428305 | ACACTGACGACATGGTTCTACAA TCTGATAAAATGACCCTACTC | TACGGTAGCAGAGACTTGGTCTC ACCTAAGTTTACAATGAAGG |
| rs7337667.G.A | chr13:97489453-97489463 | ACACTGACGACATGGTTCTACAG GAAATTAATACTGATAAAGACTA TCAG | TACGGTAGCAGAGACTTGGTCTTT TAGTCTTATCTTTCGCAAAACATA |
| rs58050565.ATTT .A | chr9:88937847-88937860 | ACACTGACGACATGGTTCTACAT GAAGATAAGTTTACTGAATTCAT AAA | TACGGTAGCAGAGACTTGGTCTC TCCTAGTGGAACTTAATAAAATA AGT |
| rs3734110.T.C | chr5:13701532-13701542 | ACACTGACGACATGGTTCTACAA GTTCAGTAAACCAGAAACC | TACGGTAGCAGAGACTTGGTCTG TGAACATAGCTGGTTTACA |
| rs5930933.C.T | chrX:135431354 135431364 | ACACTGACGACATGGTTCTACAT ACCCCTGTTTCATACCC | TACGGTAGCAGAGACTTGGTCTC TCAGCTTCAGTACTATGAGG |
| rs831618.T.G | chr11:33728610-33728620 | ACACTGACGACATGGTTCTACAG ATCCAGCTAACTTTGCATA | TACGGTAGCAGAGACTTGGTCTT GGTACAGGATTCTAAGCT |
| rs2104772.T.A | chr9:117808781-117808791 | ACACTGACGACATGGTTCTACAC AATTTCGTATTCAGTAGCCT | TACGGTAGCAGAGACTTGGTCTG TCTGAGCCACTGGAAATA |
| rs2269991.A.G | chr7:79090132-7909014 2 | ACACTGACGACATGGTTCTACAC CTTTTGTAATTGTAGGCATTC | TACGGTAGCAGAGACTTGGTCTG GGGATAGATTCAGCTACT |
| rs3766601.C.T | chr1:100 388231-100388241 | ACACTGACGACATGGTTCTACAT GATAACTTATTTTAAGATGCCTTA AAA | TACGGTAGCAGAGACTTGGTCTA AATTCTATCTAGGAAATAAGTTCT ATGA |
| rs428463 2.C.G | chr16:70 606551-70606561 | ACACTGACGACATGGTTCTACAT | TACGGTAGCAGAGACTTGGTCTT |
| | | AGTATGGGGATTTTAGCATG | CTTCTGTACTGACCCTTC |
| rs9389248.T.C | chr6:135282652-135282662 | ACACTGACGACATGGTTCTACAT AACATCAGATGAGGTCACT | TACGGTAGCAGAGACTTGGTCTG GTTGGCTGTTTATGGTTT |
| rs1054975.T.C | chr3:9406832-9406842 | ACACTGACGACATGGTTCTACAC CACTAACCAACTCCTAGAT | TACGGTAGCAGAGACTTGGTCTA CAGTGGCTCCAATAGTG |
| rs6438874.G.A | chr3:124739888-124739898 | ACACTGACGACATGGTTCTACAC AGACTGACCGTCAAAGA | TACGGTAGCAGAGACTTGGTCTA AGCTTAACAACTCCACTG |
| rs832068.G.A | chr3:97754143-97754153 | ACACTGACGACATGGTTCTACATT TTCCGGGGAAGGTTTT | TACGGTAGCAGAGACTTGGTCTA CCTCACTTCTTTGTTTGC |
| rs840388.T.G | chr5:34018886-34018896 | ACACTGACGACATGGTTCTACAA CTGGAAAATTACCAAGGC | TACGGTAGCAGAGACTTGGTCTC CGCAAATTCTAAATCTTAAAACA |
| rs7023954.G.A | chr9:21816754-21816764 | ACACTGACGACATGGTTCTACAT GATGCCTTAATTTTGGGG | TACGGTAGCAGAGACTTGGTCTC CAGAATTTAAGTTAAATTTAAATT ATCCT |
| rs7808823.T.C | chr7:129989874-1299898 84 | ACACTGACGACATGGTTCTACAG AACAAGGTGGACTTCTG | TACGGTAGCAGAGACTTGGTCTG CTGTAAGCAAGTCCATGA |
| rs35350755.T.G | chr10:123549687-123549697 | ACACTGACGACATGGTTCTACAA AACAAAGCCAATGATTTCC | TACGGTAGCAGAGACTTGGTCTG AAAACTCCAAGTACTGCC |
| rs3802980.A.G | chr11:5706307-5706317 | ACACTGACGACATGGTTCTACAG GTTAGGATTCACTCACCA | TACGGTAGCAGAGACTTGGTCTA TTGGCCCATAGTTTATCTTTC |
| rs2942.G.A | chr6:146755136-146755146 | ACACTGACGACATGGTTCTACAC ACGTGAAGACCAATGAG | TACGGTAGCAGAGACTTGGTCTG TCTTGGTGCTGGTATCT |
| rs6921.T.A | chr8:103838872-103838882 | ACACTGACGACATGGTTCTACAC AGACATTTCTTAAAAGTTACCGT | TACGGTAGCAGAGACTTGGTCTC CTTGAACTTCATGGCATA |
| rs1133181.C.A | chr2:55402268-55402278 | ACACTGACGACATGGTTCTACAG AGTTTTCAATCAAATACTTATTCA G | TACGGTAGCAGAGACTTGGTCTG TCGAATTTTGCATAGAAGTAGA |
| rs3837134.CT.C | chr7:27221061-27221072 | ACACTGACGACATGGTTCTACATC TTTCCAAATGTTGCAAGA | TACGGTAGCAGAGACTTGGTCTTT TTCTTTTGCCATTAGTTGAT |
| rs2414105.A.G | chr15:51868369-51868379 | ACACTGACGACATGGTTCTACAA GTCATTTGCCAAAATAACAATA | TACGGTAGCAGAGACTTGGTCTA AATAGTATGTAATAAGATTTCAA AAGT |
| rs7305115.A.G | chr12:72372858-72372868 | ACACTGACGACATGGTTCTACAC CACTGTACCCAGTACATC | TACGGTAGCAGAGACTTGGTCTG ATCATTGACCACTTTATGGT |
| rs2177142.G.A | chr7:22459320-22459330 | ACACTGACGACATGGTTCTACAC GGATCCATGTTGACAGA | TACGGTAGCAGAGACTTGGTCTA CAGCATTGGATTGCTCA |
| rs2400478.T.C | chr5:147510862-147510872 | ACACTGACGACATGGTTCTACATC TTCTTGAATGCCATAAAGTA | TACGGTAGCAGAGACTTGGTCTG GCTTTAAATCCTTTGGACA |
| rs6895049.T.G | chr5:52248869-52248879 | ACACTGACGACATGGTTCTACAA CCAAGAATACATTTAGCATCT | TACGGTAGCAGAGACTTGGTCTC CTGATTTTCCATCCTAGATAA |
| rs8128.A.C | chr1:115110679-115110689 | ACACTGACGACATGGTTCTACAA CTTAAACATCAATGGTTTTGG | TACGGTAGCAGAGACTTGGTCTA CAATTTAGCAGGTAGAAGAAAA |
| rs3828977.G.A | chr7:29519494-29519504 | ACACTGACGACATGGTTCTACAT GACTGGATACCGCTTCT | TACGGTAGCAGAGACTTGGTCTC GGCTTCTGAATAAGGATG |
| rs12102203.A.G | chr15:51791555-51791565 | ACACTGACGACATGGTTCTACAT AGATTTAAAGGTCGAATGATCT | TACGGTAGCAGAGACTTGGTCTG AAGCATGCTGTCAAATTT |
| rs7974559.A.T | chr12:5023955-5023965 | ACACTGACGACATGGTTCTACAC | TACGGTAGCAGAGACTTGGTCTA |
| | | ATCTACTAGCATTGGTCC | AGTTTATCTTGGCTTTGGTG |
| rs2686817.C.A | chr7:47968923-47968933 | ACACTGACGACATGGTTCTACATC CCCGAAATCCATCATC | TACGGTAGCAGAGACTTGGTCTT CATGAACCCTGTTCTTAATT |
| rs1884214.C.T | chr14:37149132-37149142 | ACACTGACGACATGGTTCTACAT AAATTTTATTTCATGTTTCCTACTT G | TACGGTAGCAGAGACTTGGTCTA TAGGTATTTTAAAACATTGTTTAA AACA |
| rs1053959.C.A | chr9:114359620-114359630 | ACACTGACGACATGGTTCTACAA TACAGTCCCTTCCAACTC | TACGGTAGCAGAGACTTGGTCTG TTGGCTATCCTACTAATAGTGA |
| rs3780190.A.G | chr9:139099069-139099079 | ACACTGACGACATGGTTCTACAA GTTGTTGAAAGAATCAGCT | TACGGTAGCAGAGACTTGGTCTA TGGATGTATTCATAAGACCATG |
| rs1019503.G.A | chr5:96254813-96254823 | ACACTGACGACATGGTTCTACAT ACAGATAAGTTTTAGAAAGACTC AA | TACGGTAGCAGAGACTTGGTCTA TTGCTCCAAAGTGGATTG |
| rs10883098.C.G | chr10:100218191-100218201 | ACACTGACGACATGGTTCTACAG AAGCTTCCTACCTACCA | TACGGTAGCAGAGACTTGGTCTG CCTATTTGAGCTAGAACAAA |
| rs6979945.G.A | chr7:82386121-82386131 | ACACTGACGACATGGTTCTACAA AGCAATTTGATTTATTTCTTTTGG | TACGGTAGCAGAGACTTGGTCTC TAAAGGTGAAGAATTACATGAAA AT |
| rs147147897.CTA .C | chr16:70192457-70192469 | ACACTGACGACATGGTTCTACACT CCTTCTAGACATTTCTTGC | TACGGTAGCAGAGACTTGGTCTC TAACAAGCCAACCAGAAC |
| rs4692500.A.C | chr4:31147268-31147278 | ACACTGACGACATGGTTCTACAA TGGGCTATTCCCAACTT | TACGGTAGCAGAGACTTGGTCTA GACTGGTCCATTATCTGAG |
| rs4727038.G.C | chr7:148979912-148979922 | ACACTGACGACATGGTTCTACAT GGAAAGTTTTCTACTTTGCT | TACGGTAGCAGAGACTTGGTCTG CAAAGTTCCATTAACAACTAC |
| rs8692.C.T | chr16:84211479-84211489 | ACACTGACGACATGGTTCTACAA GCACCGAAAGCATCATA | TACGGTAGCAGAGACTTGGTCTT GTTTATCATGCCTAAGGGA |
| rs59508481.C.T | chr3:153839955-153839965 | ACACTGACGACATGGTTCTACAA ACGGGTTACTAATTACGG | TACGGTAGCAGAGACTTGGTCTC TCCTATGTACCGTCCTG |
| rs201131957.TTC.T | chr6:87993564-87993576 | ACACTGACGACATGGTTCTACAG TGCACCAATTGTAATGAG | TACGGTAGCAGAGACTTGGTCTT AGTCTGAAGAAATCTAACAGGAT |
| ..G.GT | chr15:52102326-52102337 | ACACTGACGACATGGTTCTACAA TCCTTTAATTAGTTTATGGCTTCA | TACGGTAGCAGAGACTTGGTCTC CTAAATGGGTGAAAGGG |
| rs7096.G. A | chr9:115234607-115234617 | ACACTGACGACATGGTTCTACACT GCTAGTGCTGCTTTAC | TACGGTAGCAGAGACTTGGTCTA AAGATCTAATTGGCTTTTATTAGC |
| ..GTC.G | chr12:10311906-10311918 | ACACTGACGACATGGTTCTACAC CCCACTTGTCCCAAAAT | TACGGTAGCAGAGACTTGGTCTA AAGGTATCAATCCTTGGTTT |
| rs10486985.G.A | chr7:82386078-82386088 | ACACTGACGACATGGTTCTACAC ACTTCACCCTTAAATAAAGC | TACGGTAGCAGAGACTTGGTCTT GCTGGTCTTACCCTAGT |
| rs931606.G.A | chr4:79443846-79443856 | ACACTGACGACATGGTTCTACATT TCCCCTTCAAATAGGACA | TACGGTAGCAGAGACTTGGTCTA AGTCAAATTCAATTCCTCCTAG |
| ..G.GA | chr11:108345511-1083455 22 | ACACTGACGACATGGTTCTACAA CTTGGTGCAGTCTTCTA | TACGGTAGCAGAGACTTGGTCTG AAGAACTTTGAGTCAGCC |
| rs30169. T.G | chr5:13719018-13719028 | ACACTGACGACATGGTTCTACATT TCTGGACTCACCACTAT | TACGGTAGCAGAGACTTGGTCTC TTCAGATGTCCATTAAATTTGC |
| rs487088 7.G.A | chr8:125061891-125061901 | ACACTGACGACATGGTTCTACATT CTTTTCTTCTTTAGGGGAAG | TACGGTAGCAGAGACTTGGTCTG GTGATAGCATAACCTGG |
| rs1133182.C.T | chr2:554 02221-55402231 | ACACTGACGACATGGTTCTACATT | TACGGTAGCAGAGACTTGGTCTG |
| | | AGGTATCTGTTCTGCTTGAT | TCTGAATAAGTATTTGATTGAAA AC |
| rs36004074.G.A | chr2:186665428-186665438 | ACACTGACGACATGGTTCTACAA GCAGAAGAATGTCAACTTTTA | TACGGTAGCAGAGACTTGGTCTA AGGTCTGACATGTAATCTACTT |
| rs114154393.T.C | chr6:30999993-31000003 | ACACTGACGACATGGTTCTACAG ACCTGTTTCATAGGCAC | TACGGTAGCAGAGACTTGGTCTG TGAAGTCCATTCCAGGG |
| rs1047481.G.A | chr5:52249472-52249482 | ACACTGACGACATGGTTCTACAC CAGAAATGTATTTTAAAGAATTTC AG | TACGGTAGCAGAGACTTGGTCTA GTAGATTCCAATGAACCATAATG |
| rs2293250.G.A | chr3:33434827-33434837 | ACACTGACGACATGGTTCTACAA AAACAAATGATTTGATTTACCTGA | TACGGTAGCAGAGACTTGGTCTC ACCAAATTAATCAGGTTTACAG |
| rs10849174.T.C | chr12:5025090-5025100 | ACACTGACGACATGGTTCTACAG ACTGGTTGATTCTTCTGC | TACGGTAGCAGAGACTTGGTCTC CCAAACTCATAACAACAAC |
| rs7011928.G.A | chr8:28421981-28421991 | ACACTGACGACATGGTTCTACACT CATACAAATAAGAATTGGTAGC | TACGGTAGCAGAGACTTGGTCTA TCATCAGGGGCAAGTTA |
| rs3733690.G.C | chr5:140569523-140569533 | ACACTGACGACATGGTTCTACAG AGGCATTTCTTACTAGAATCC | TACGGTAGCAGAGACTTGGTCTG AGGCAATGAATTTAAAGTCAATA |
| rs1299331.A.G | chr3:106965488-106965498 | ACACTGACGACATGGTTCTACAG GTCTTAAGACAGGAAGCTA | TACGGTAGCAGAGACTTGGTCTT CTGATTCCAGTCATAGCTC |
| rs2278711.C.T | chr2:47085394-47085404 | ACACTGACGACATGGTTCTACAT GTTCCCTTTCGGATGAG | TACGGTAGCAGAGACTTGGTCTA GTTATTTTGAGCAGATGGTT |
| rs11692815.G.A | chr2:68415763-68415773 | ACACTGACGACATGGTTCTACAA CTCGCTGTACTAAAGGAT | TACGGTAGCAGAGACTTGGTCTA TGAAATTAAAAGGCTAGGAAAGA |
| rs116485895.T.C | chr4:69176794-69176804 | ACACTGACGACATGGTTCTACAC CTTCCTGATGTAATCCTCA | TACGGTAGCAGAGACTTGGTCTC AAGGAAATGGGGTTCTTT |
| rs2161036.G.T | chr2:186659355-186659365 | ACACTGACGACATGGTTCTACAA GTGGAAGAACTTTTGAATAAGT | TACGGTAGCAGAGACTTGGTCTTT GGCAGTTTCATTAAGTGG |
| rs17229201.T.C | chr2:186656952-186656962 | ACACTGACGACATGGTTCTACAA AATACTTAAGTATGTAGTCAAGTT AATTT | TACGGTAGCAGAGACTTGGTCTG CCTGTATCGATAAGTTTCAA |
| rs2230854.T.C | chr13:103275382-103275392 | ACACTGACGACATGGTTCTACAG ATGATGGAAACCTGCTC | TACGGTAGCAGAGACTTGGTCTA CCACTTTATCACCTCATAAATCT |
| rs4647554.A.G | chr9:97862697-97862707 | ACACTGACGACATGGTTCTACAA AAAGTTCCTGGAATTTTCCA | TACGGTAGCAGAGACTTGGTCTG TTTTGAGGTATGAGCAAAGT |
| rs704697.T.C | chr11:33724776-33724786 | ACACTGACGACATGGTTCTACAG GGGTTAAATAAGAAGTTGATGA | TACGGTAGCAGAGACTTGGTCTT CATAGGGACTTTGCCTC |
| rs30168.G.A | chr5:13719085-13719095 | ACACTGACGACATGGTTCTACAG TTGGCAAATTTAATGGACA | TACGGTAGCAGAGACTTGGTCTA TGGACATAATCATAGAAACTGA |
| rs55938253.A.G | chr2:186661669-186661679 | ACACTGACGACATGGTTCTACAA GTTTTGATCAGACCATGAAAG | TACGGTAGCAGAGACTTGGTCTG ACAAGACTTGCTTTAATGC |
| rs10931200.C.A | chr2:186664959-186664969 | ACACTGACGACATGGTTCTACAA GAAACAAATCATTTTCTATGCAT | TACGGTAGCAGAGACTTGGTCTG AGTAGAAAATTGCCAGTCT |
| rs6928.C.G | chr22:22115000-22115010 | ACACTGACGACATGGTTCTACAC AGATACAAAGCAGTTTCAGA | TACGGTAGCAGAGACTTGGTCTC AGGTTTACCTAAAGCTTGTT |
| rs2282022.T.C | chr13:41374182-41374192 | ACACTGACGACATGGTTCTACAA CTAAACAGAATTTGGAAACCA | TACGGTAGCAGAGACTTGGTCTC CTTTGCAATCTTGAAGCTT |
| rs1613780.G.T | chr1:144865846-144865856 | ACACTGACGACATGGTTCTACATC TGGAAACATGACTCAGTT | TACGGTAGCAGAGACTTGGTCTC TAACTTCTACAGTCAGGGC |
| rs7893462.A.G | chr10:28228861-28228871 | ACACTGACGACATGGTTCTACAA GAAAGAACGCCAACAAC | TACGGTAGCAGAGACTTGGTCTA TCATTGAAAACCTTGTCAAG |
| rs2275145.G.A | chr14:35242824-35242834 | ACACTGACGACATGGTTCTACATT TGTACCTGTCTTTTACTATGA | TACGGTAGCAGAGACTTGGTCTG GAAAACAAGGAAAATGGGA |
| rs9225.C.T | chr2:47086108-47086118 | ACACTGACGACATGGTTCTACAC CCCTCCTTAAGCATTGT | TACGGTAGCAGAGACTTGGTCTG ATGCTCAAAGTATGATTTCAC |
| rs1130072.T.C | chr9:14615931-14615941 | ACACTGACGACATGGTTCTACAA AGAAATACTTCAGTCCAACA | TACGGTAGCAGAGACTTGGTCTTT ATGTATTCTGGATAACTGAAAAC A |
| rs732774.C.T | chr13:52523804-52523814 | ACACTGACGACATGGTTCTACAG GATCAATGTCAGTAGATTATTTAA AA | TACGGTAGCAGAGACTTGGTCTT GGATTGTAATCGGTTTTATCG |
| rs1359300.G.A | chr1:144922579-144922589 | ACACTGACGACATGGTTCTACAA GTTTCCTTGAGGTTCTGA | TACGGTAGCAGAGACTTGGTCTG CTGAATTCAGATTCTTCAAG |
| rs2549782.G.T | chr5:96230996-96231006 | ACACTGACGACATGGTTCTACAT | TACGGTAGCAGAGACTTGGTCTA |
| | | ACCTCCTAGTGGTTTGG | CAGCGATAAGTTCCATG |
| rs9289122.C.G | chr3:118867043-118867053 | ACACTGACGACATGGTTCTACAG GGGATTTAAATTTCAGGATG | TACGGTAGCAGAGACTTGGTCTT GAAACTTGGTAAGGATCTTC |
| rs4766310.G.A | chr12:5022037-5022047 | ACACTGACGACATGGTTCTACAG CTACTGACCGATGTTTAAAA | TACGGTAGCAGAGACTTGGTCTG AGTCACTAGGTTTTCTGTTTT |
| rs4617548.A.G | chr11:16133409-16133419 | ACACTGACGACATGGTTCTACACT ACTTACCTGGTTTGTATGTTA | TACGGTAGCAGAGACTTGGTCTC GCTCATGATCCCAATTTT |
| rs10057908.T.G | chr5:102884929-102884939 | ACACTGACGACATGGTTCTACAA GTAAAACTGAATTTCAAGATGC | TACGGTAGCAGAGACTTGGTCTA TTTAGATACATAATAAAATTCGA GCTA |
| rs72901775.T.C | chr1:1647810-1647820 | ACACTGACGACATGGTTCTACAA AGTCCTCAACTGACCCA | TACGGTAGCAGAGACTTGGTCTA GAAGAACAGGATAAAGCTC |
| rs2255546.C.T | chr5:96249111-96249121 | ACACTGACGACATGGTTCTACAT GGAAGGAAAGGTTATCAAGA | TACGGTAGCAGAGACTTGGTCTA GAAGATGGGTCCAATTTTC |
| rs2548538.T.A | chr5:96232138-96232148 | ACACTGACGACATGGTTCTACACT CCCTGTTTAGGATGACTA | TACGGTAGCAGAGACTTGGTCTA TTTCCTGTATTTGAGTCGG |
| rs177083.C.T | chr2:32447714-3244772 4 | ACACTGACGACATGGTTCTACAG | TACGGTAGCAGAGACTTGGTCTG |
| | | TGACCTGCTAAAGAAATACA | ATAAAGGCCAAGGAACAG |
| rs6876143.A.G | chr5:17380266-17380276 | ACACTGACGACATGGTTCTACAT GAAAACTGTCTTTCATTAGCT | TACGGTAGCAGAGACTTGGTCTA ATACCATGTCTGCCTGT |
| rs9841174.T.C | chr3:167184874-167184884 | ACACTGACGACATGGTTCTACAC ATGAATACAATGGCAAATAATAA T | TACGGTAGCAGAGACTTGGTCTG TGGATTTTCAAGATGCAAAG |
| rs116571982.G.A | chr6:31023144-31023154 | ACACTGACGACATGGTTCTACAA AGACTTGAACCCTATTAGAAAA | TACGGTAGCAGAGACTTGGTCTG AATACATGTAGCTTACATGGC |
| rs4898.T.C | chr7:82386202-82386212 | ACACTGACGACATGGTTCTACATT CTTCACCTTTAGTCAGGTTA | TACGGTAGCAGAGACTTGGTCTA TCATCTAAACCAATGGCAAG |
| rs202026726.T.TA | chr3:86990606-86990617 | ACACTGACGACATGGTTCTACAA AAGTATGTTGCATTTTAAAAGAAT TAT | TACGGTAGCAGAGACTTGGTCTC ACCTTTTAGAAAAGGATAAGTAA T |
| rs11537748.A.G | chr15:31668690-31668700 | ACACTGACGACATGGTTCTACATT TTGCTGACTGTTTCCATA | TACGGTAGCAGAGACTTGGTCTG GGGTAATCCTGATTTCAAAA |
| rs14026.G.A | chr2:55404790-55404800 | ACACTGACGACATGGTTCTACAA GTAGCTTAATGCCAACTTT | TACGGTAGCAGAGACTTGGTCTA GAAACAACCATCTTTATCTTTTG |
| rsl780138.A.G | chr10:38501650-38501660 | ACACTGACGACATGGTTCTACAA AAGTGAAGACTCCTTCTACC | TACGGTAGCAGAGACTTGGTCTA GTTAAACTATTGTTTTAAAAGCCT T |
| rs3800951.T.G | chr7:99758132-99758142 | ACACTGACGACATGGTTCTACAC ATCGAAGTACTCAGCCA | TACGGTAGCAGAGACTTGGTCTT CGATTTGGGGTCTTCAT |
| rs1230382.T.A | chr5:96254350-96254360 | ACACTGACGACATGGTTCTACAT ACCATTTGGTTTAAGCCTTAC | TACGGTAGCAGAGACTTGGTCTG GCAGATTCGACTTCATTT |
| rs201009141.CCT.C | chr7:27221060-27221072 | ACACTGACGACATGGTTCTACATC TTTCCAAATGTTGCAAGA | TACGGTAGCAGAGACTTGGTCTTT TTCTTTTGCCATTAGTTGAT |
| rs2350917.T.C | chr8:62576972-62576982 | ACACTGACGACATGGTTCTACATC TAATCTGAAATAATTAAAACCTTT CTAT | TACGGTAGCAGAGACTTGGTCTC CATAGCCATGAATAAAGGG |
| rs114783926.A.G | chr6:31026430-31026440 | ACACTGACGACATGGTTCTACAG AGGTCACTAGCATTAGCA | TACGGTAGCAGAGACTTGGTCTG TTTGGGAGCATAGACTTT |
| rs36261.G.A | chr19:8327240-8327250 | ACACTGACGACATGGTTCTACAG GCAACTGGACAGATCTG | TACGGTAGCAGAGACTTGGTCTC ATTTGTTTTAATTGGACCCTTTT |
| rs10851500.T.C | chr15:51783816-51783826 | ACACTGACGACATGGTTCTACAG | TACGGTAGCAGAGACTTGGTCTT |
| | | TTACCTTTTCAATGCATCTTC | ATGATTCCAGCAATTCAGA |
| rs5930932.T.C | chrX:135431232-135431242 | ACACTGACGACATGGTTCTACAT ATTCAGGTTTCCCCATCT | TACGGTAGCAGAGACTTGGTCTTT TCTAACAAGGAAGTCATCTTT |
| rs10226437.C.T | chr7:129984986-129984996 | ACACTGACGACATGGTTCTACAA GCATAACTGCTTTCTTTTCTAT | TACGGTAGCAGAGACTTGGTCTTT CGTAGTTCAGCTGTTCA |
| rs7512.G.C | chr17:10583710-10583720 | ACACTGACGACATGGTTCTACAG CAAAACTGAGTATGTTTACTTTA | TACGGTAGCAGAGACTTGGTCTG TGACATACCAAATGGAAATAAG |
| rs1168308.A.G | chr12:66742179-66742189 | ACACTGACGACATGGTTCTACAC CTGTTTGATAATTTGTTGCAT | TACGGTAGCAGAGACTTGGTCTA TAGGTGGTGGTAAATGTTTTG |
| rs4260880.T.C | chr8:69020492-69020502 | ACACTGACGACATGGTTCTACAA GTTTCTTATCCCAAAACATCA | TACGGTAGCAGAGACTTGGTCTC TTGCTCCGAAGATTTGTT |
| rs1801249.A.G | chr13:52515350-52515360 | ACACTGACGACATGGTTCTACAC ATCGCTAGAAATGGTTAAAC | TACGGTAGCAGAGACTTGGTCTA TTGGACCATTTAGAAATAACCAC |
| rs35382.A.G | chr5:59821539-59821549 | ACACTGACGACATGGTTCTACAA ATCCTAATAGATCATCTAATTGTT | TACGGTAGCAGAGACTTGGTCTA |
| | | TATC | AGTTTATTGAGTGCAGATGTC |
| rs8042.T.A | chr8:28431196-28431206 | ACACTGACGACATGGTTCTACAA GACAGCTAGGTCTGTCT | TACGGTAGCAGAGACTTGGTCTT GTAACAGTAAAATGGGGAAAG |
| rs1044352.G.T | chr4:31147870-31147880 | ACACTGACGACATGGTTCTACAA CAGACCAGAAGAAATTGTC | TACGGTAGCAGAGACTTGGTCTA CCTAAGATTATTTAGGTCTTTAGA TT |
| rs1747930.C.G | chr1:144916744-144916754 | ACACTGACGACATGGTTCTACAG GTAGTAGATAACTGTTCCACT | TACGGTAGCAGAGACTTGGTCTA TGGGGTAATCATTTTGACTG |
| rs2161916.G.A | chr2:171073883-171073893 | ACACTGACGACATGGTTCTACAA GCTCAACTCACCAGTAC | TACGGTAGCAGAGACTTGGTCTT ACCAAGGCATTCAACAAG |
| rs2213842.A.G | chr6:112423806-112423816 | ACACTGACGACATGGTTCTACAA TAGTACATGGATCATTGAGTAGA | TACGGTAGCAGAGACTTGGTCTC TGTTAGGGCAAGTCCAG |
| rs2070203.G.A | chr16:70303576-70303586 | ACACTGACGACATGGTTCTACAG TGCAGCATACTTACCCA | TACGGTAGCAGAGACTTGGTCTG GATTGACATGGCCTACC |
| rs116072190.A.C | chr6:31023182-31023192 | ACACTGACGACATGGTTCTACAA AGACTTGAACCCTATTAGAAAA | TACGGTAGCAGAGACTTGGTCTT CTTAGGAATACATGTAGCTTACA |
| rs7562137.A.G | chr2:186625766-186625776 | ACACTGACGACATGGTTCTACACT CGTTTTCTTTTAGGACATACA | TACGGTAGCAGAGACTTGGTCTC ATTATCCTGTACATCTGCC |
| rs10141087.G.C | chr14:37147337-37147347 | ACACTGACGACATGGTTCTACAG AAGATGGATTCCCTAGGT | TACGGTAGCAGAGACTTGGTCTG CCATCTGAGTTTTAAACGA |
| rs3453.T.C | chr21:35819059-35819069 | ACACTGACGACATGGTTCTACACT CACTACTGCAAATGCAT | TACGGTAGCAGAGACTTGGTCTA CCATGTACTGTTCTAAGCT |
| rs4349706.T.C | chr5:147516594-147516604 | ACACTGACGACATGGTTCTACAT AGGACGAATGACAGGAA | TACGGTAGCAGAGACTTGGTCTA AGACAAGCTCCGAAGAAT |
| rs546577.A.C | chr9:104172932-104172942 | ACACTGACGACATGGTTCTACAA AACCTATTAATTCATCTGTTTTAA CC | TACGGTAGCAGAGACTTGGTCTG CGTTTGACAGAATTGGT |
| rs6940018.G.C | chr6:136599389-136599399 | ACACTGACGACATGGTTCTACAC ATAAGCTGAAAGGCCAG | TACGGTAGCAGAGACTTGGTCTC GAAAGATACATTTGAACATGAC |
| rs61575744.G.GT | chr1:41972271-41972282 | ACACTGACGACATGGTTCTACATT TCCTTTTGTACGTCAGACT | TACGGTAGCAGAGACTTGGTCTT CTTGCAATGTTGGTATGGTA |
| rs1958715.G.A | chr14:20528203-20528213 | ACACTGACGACATGGTTCTACAA TAAAGAACTTCTGGAACCTTTC | TACGGTAGCAGAGACTTGGTCTC TAGTAAAATAAACTCGGTCACT |
| rs1852450.C.A | chr12:16397730-16397740 | ACACTGACGACATGGTTCTACATC AGTGCACTAACCAACA | TACGGTAGCAGAGACTTGGTCTT CTTTCTGATCAGCATCTTG |
| rs1052480.T.C | chr1:115591054-115591064 | ACACTGACGACATGGTTCTACAT AAAATCTATTGTTGCTTAGTGGTG | TACGGTAGCAGAGACTTGGTCTG TTCTGTTCCATGTACGATT |
| rs2528843.G.T | chr7:22478203-22478213 | ACACTGACGACATGGTTCTACAG ATTTTCTAGATGAAGCTGAAAC | TACGGTAGCAGAGACTTGGTCTC CTTCCAAGACTAGGAGTT |
| rs974110.A.G | chr6:66112405-66112415 | ACACTGACGACATGGTTCTACAC CTGCAAGGATAATCTTTCT | TACGGTAGCAGAGACTTGGTCTG TGCATCTGTTCACCAATA |
| rs2564990.T.C | chr5:53816778-53816788 | ACACTGACGACATGGTTCTACAT GGACATCAGTACTAATTTTAGTAG A | TACGGTAGCAGAGACTTGGTCTTT TAATACAGGCCAACAGC |
| rs35952402.A.AC TC | chr5:147516910-147516923 | ACACTGACGACATGGTTCTACAT GGGATTTCTTTGTCACTATCT | TACGGTAGCAGAGACTTGGTCTG TCCACCAATGGGTTTAG |
| rs257376.G.A | chr7:106799993-106800003 | ACACTGACGACATGGTTCTACAG | TACGGTAGCAGAGACTTGGTCTC |
| | | GACCTTGCATGGAAATTA | ATACTTTTGCTTCATGCAG |
| rs840385.T.C | chr5:34020354-34020364 | ACACTGACGACATGGTTCTACAA TGAACTTAAAACTTCAGGGG | TACGGTAGCAGAGACTTGGTCTA CAGAAGTCATTTGCAAAGAA |
| rs316214.G.C | chr5:96497634-96497644 | ACACTGACGACATGGTTCTACAA TGCAAAGCAAGGGTATTTAA | TACGGTAGCAGAGACTTGGTCTC CTGTAATTATCTAATAAATTGGCT TAA |

Primers were designed to be compatible with pooling, so that they could be amplified by PCR in a multiplex configuration with limited sample amounts. Massive parallel sequencing gives a high read depth, such that MAFs may be reliably detected even when the fraction of fetal material in the combination samples is 1% or lower.

10 ml peripheral blood was collected from each of three pregnant volunteers (second trimester) into a standard EDTA blood collection tube. T-cell lymphocytes and monocytes were isolated from 5 ml of blood each using the RosetteSep™ Human T Cell Enrichment Cocktail (Stem Cell Technologies, 15061) and RosetteSep™ Human Monocyte Enrichment Cocktail (Stem Cell Technologies, 15068) according to the manufacturer's instructions. Plasma supernatant was centrifuged twice at high speed for 10 minutes to pellet and remove residual cells, and split into two samples per volunteer. 1% of resuspended monocytes, as defined by CD14 expression were added to a plasma sample to create a combination sample. By defining monocytes by CD14 expression, it is possible that the cell population also contains other phagocytes such as macrophages and neutrophils. A second plasma sample was used as a cell-free control. DNA was purified from the combination and cell-free control samples using the QIAamp Circulating Nucleic Acid Kit (Qiagen, 55114), and from the T-cell lymphocytes using the DNeasy Blood & Tissue Kit (Qiagen, 69506). DNA concentration purified from T-cell lymphocytes was measured using the Qubit®dsDNA BR Assay Kit (Life Technologies, Q32853). After DNA extraction, library preparation was performed using the primers listed in Table 1, and massive parallel sequencing was performed on the Illumina MiSeq™ sequencer.

A summary of the procedure as performed is as follows:
Collect 20 ml blood in EDTA tube from each of 3 pregnant volunteers, second trimester.
Pipette blood into 15 or 50 ml conical tubes according to following: a) 5mL RosetteSEP Tcell; b) 10 ml RosetteSEP monocyte; and c) 5 ml RosetteSEP monocyte.
Add 10 µl 0.5M EDTA to tubes a) and c) and 20ul 0.5 M EDTA to tube b).
Add 250 µl Ab cocktail (a mixture of antibodies directed at human cell types) to tubes a) and c) and 500ul to tube b).
Incubate 10 minutes at room temperature.
Dilute with equal volume (of original sample) of SEP buffer containing density separation medium.
Layer blood over 15 ml ficoll in a SepMate tube.
Spin 10 minutes at 1200 x g.
Pour the supernatant off into three fresh 50 mL conical tubes. Set one tube aside on ice and label it 100%.
Pellet the cells in the two remaining tubes by centrifuging at 500 x g for 10 min.
Decant the supernatants into fresh 50 ml conical tube, combining the supernatants from the two remaining tubes into a single tube, label plasma.
Re-suspend the T-cell pellet in 5 ml PBS and the monocyte pellet in 10 ml phosphate-buffered saline (PBS). Set aside on ice
Spin the plasma at maximum speed for 10 min to pellet residual cells.
Decant the supernatant into a fresh 50 ml tube and repeat the spin on maximum speed for 10 min.
Decant the supernatant into a fresh 50 ml tube. Mix by inverting several times.
Divide the plasma supernatant into three 50 ml tubes with 5ml in each. Label the tubes 0%, 1%, and 5%.
Mix the monocytes well by vortexing and inverting several times. Pipette 50 µl into the 1% plasma tube and 250 µl into the 5% plasma tube.
Perform DNA extraction using a ccff kit (with carrier RNA) on the following samples: 1) 100% monocytes; 2) 5% monocytes; 3) 1% monocytes; and 4) 0% monocytes.
Perform DNA extraction using a DNeasy Blood & Tissue Kit (Qiagen, 69506) for the following samples of pelleted cells: 1) the monocyte pellet; and 2) the T-cell pellet.

Generate MAF multiplex PCR amplicons as follows: generate 48 multiplex primer pools from the MAF primers described above. Mix and dilute primers to 1 µM in pools. Modify PCR conditions as described below from the Access Array™ System for Illumina Sequencing Systems User Guide (Fluidigm).

| | |
|---|---|
| 10X Fast Start High fidelity Reaction Buffer (Roche) | 0.5 µL |
| 25 mM MgCl₂ (Roche) | 0.9 µL |
| 5 mM dNTPS mix | 0.2 µL |
| 5U/uL Fast Start High Fidelity Enzyme Blend (Roche | 0.05 µL |
| PCR certified Water | 1.12 µL |
| DNA sample | 0.83 µL |
| Total Master mix | ∼4 µL |
| Primer mix 1 µM | 1 µL |

Run samples on an ABI 9700 thermocycler with a 384 well head under the thermocycle profile below.

| |
|---|
| 50 °C for 2 minutes |
| 70 °C for 20 minutes |
| 95 °C for 10 minutes |
| 10 cycles of the three steps above |
| 95 °C for 15 seconds |
| 60 °C for 30 seconds |
| 72 °C for 1 minute |
| 2 cycles of the three steps above |
| 95 °C for 15 seconds |
| 80 °C for 30 seconds |
| 60 °C for 30 seconds |
| 72 °C for 1 minute |
| 8 cycles of the four steps above |
| 95 °C for 15 seconds |
| 60 °C for 30 seconds |
| 72 °C for 1 minute |
| 72 cycles of the three steps above |
| 95 °C for 15 seconds |
| 80 °C for 30 seconds |
| 60 °C for 30 seconds |
| 72 °C for 1 minute |
| 8 cycles of the four steps above |
| 95 °C for 15 seconds |
| 60 °C for 30 seconds |
| 72 °C for 1 minute |
| 5 cycles of the three steps above |
| 95 °C for 15 seconds |
| 80 °C for 30 seconds |
| 60 °C for 30 seconds |
| 72 °C for 1 minute |

Pool multiplex reactions from the samples into a single tube and dilute 1:100 in PCR grade water. Attach Fluidigm Barcode indexes per the Access Array™ System for Illumina Sequencing Systems User Guide (Fluidigm).

| | |
|---|---|
| 10X Fast Start High Fidelity Reaction Buffer (Roche) | 2.0 µL |
| 25 mM MgCl₂ (Roche) | 3.6 µL |
| 5 mM dNTP mix | 1.0 µL |
| 5 U/µL Fast Start High Fidelity Enzyme Blend (Roche) | 0.2 µL |
| PCR certified Water | 6.8 µL |
| | |
| Total Master mix | ∼14 µL |
| Primer mix 1 µM (Fluidigm) | 4 µL |
| Diluted pooled PCR multiplexes | 2 µL |
| Total | 20 µL |

| |
|---|
| 95 °C for 10 minutes |
| 16 cycles of the three steps below |
| 95 °C for 15 seconds |
| 60 °C for 30 seconds |
| 72 °C for 1 minute |
| 72 °C for 3 minutes |
| 4 °C for 5 minutes |

Barcode samples in triplicate and pool them at 3 x 15 µL. Purify the pools using Agencourt AmPure XP beads at a 1:1 mix and elute in 30 µL. Quantify samples with Qubit® Fluorometric Quantitiation (Life Technologies) and check on a 2100 Bioanalyzer (Agilent Technologies Genomics) for primer dimers. Normalize samples to a concentration of 10 nM for sequencing preparation.

Library purification and generation: prepare pooled, barcoded, and purified samples per the Illumina MiSeq™ manual for library generation. Dilute 10 nM samples to 2nM at 10 µL and add 10 µL 0.2 N NaOH. Incubate samples incubated at room temperature for 5 minutes and add 980 µL of HTI buffer to generate a 20 pM library. Further dilute the library to 12.5 pM in HTI buffer and load with a 1% phiX spike.

Sequencing: Run 600 µL of each sample on an Illumina MiSeq™ at 12.5 pM with FL1 primers prepared per the Access Array™ System for Illumina Sequencing Systems User Guide (Fluidigm). Load FL1 primers at 500 nM into an Illumina MiSeq™ reagent cartridge for sequencing and run the samples using 2x151 reads with adaptor trimming as Adapter Read 2-TGTAGAACCATGTCGT Adapter Read 1-AGACCAAGTCTCTGCT.

By a conservative estimate, the data shows 16 combination sample-specific MAFs that are informative, i.e., SNPs for which the mother was homozygous and the fetus was heterozygous (Figure 1, Table 2). This value is very near the theoretical 10% (16/167) expectation (Tynan et al., J Mol Diagn. 13(4):382-9 (2011)), confirming fetal content in the sample. Also, the presence of 16 informative MAFs falls above the level for 99.9% confidence established in Tynan et al., J Mol Diagn. 13(4):382-9 (2011). Thus, the fetal DNA content in the combination sample was confirmed. Figures 1 and 2 show potentially informative alleles from two assays plotted against the percent of reads of each allele in maternal and combination samples. A rough estimate of informative MAFs can be seen by the boxed off sections at the top and bottom of the graph. In general, informative MAFs are seen where an allele in a combination sample shows major and minor frequencies. The significance of these differenced can be determined by a number of statistical analyses known in the art (for example, values that are five standard deviations from the mean value or other statistical measures). Figure 2 in particular shows tight clustering of data around the 50% mark, demonstrating a low standard deviation. Moreover, Figure 5 shows the improvement in data between a combination sample (cell-free bodily fluid plus 1% monocytes) when compared to a cell-free bodily fluid or T-cell sample alone (Figures 3 and 4, respectively). Figures 6 and 7 are simplified to show only the informative markers, and demonstrate that the combination sample produces "cleaner" data than a cell-free bodily fluid sample without monocytes. Figures 8 and 9 show exemplary informative markers. Both depict a maternal only sample wherein the reference allele reads are 100% and the alternative allele reads are 100%, and a combination sample that contains reads of the alterative allele (and, necessarily, fewer reads of the reference allele such that the values add to 100%). By contrast, the exemplary non-informative marker depicted in Figure 10 has reads of approximately 50% of reference and alternative alleles in both the maternal only and the combination sample. In addition, reproducibility in combination samples is better than in corresponding cell-free bodily fluid samples (Table 3). In Table 3, the differences between Assay 1 and Assay 2 may be because a low-performing marker was removed in Assay 2 and one of the markers in Assay 2 also was rejected due to a threshold effect with the computational percentage (to include all base calls at a position rather than only the reference and alternative markers).

It has been proposed that a qualitative size difference exists between cell-free circulating DNA and maternal DNA (Fan et al., Clin Chem. 56(8): 1279-86 (2010)). The nature of this size difference may result from the pathway by which the DNA is produced. Fetal circulating cell-free DNA is thought to be almost exclusively generated via apoptosis of liberated fetal cells, and it exists in the plasma at nucleosomal lengths preserved in apoptotic bodies. By contrast, maternal circulating cell-free DNA is more likely to be a mixture of cells produced from apoptosis and necrosis as well as autophagy and mitotic catastrophe. The DNA produced during the highly ordered apoptotic process may exist in a less degraded state than the DNA produced during other cell death pathways. DNA produced during non-apoptitic cell death is exposed to a variety of harmful subcellular components including DNA damaging reactive oxygen species. Without wishing to be bound by theory, the increased relative degradation of the maternal circulating cell-free DNA may contribute to noise in amplification-based assays aimed at detecting aneuploidies from circulating cell-free DNA. Thus, the methods of using combinations of two or more different components selected from cell-free bodily fluids, phagocytic cells, circulating vesicles, and circulating diseased cells may contribute higher quality maternal DNA and reduce signal variance.

**Table 2: 16 informative markers**

| **Sample** | **Chromosome** | **Position** | **Ref. allele** | **Alt. allele** | **A counts** | **C counts** | **G counts** | **T counts** |
|---|---|---|---|---|---|---|---|---|
| maternal | chr3 | 113804978 | G | A | 187335 | 10 | 464 | 23 |
| combination | chr3 | 113804978 | G | A | 126447 | 6 | 10980 | 29 |
| maternal | chr6 | 42627433 | G | A | 223746 | 111 | 704 | 144 |
| combination | chr6 | 42627433 | G | A | 262863 | 294 | 13527 | 292 |
| maternal | chr3 | 42787468 | A | G | 111491 | 15 | 401 | 21 |
| combination | chr3 | 42787468 | A | G | 269893 | 39 | 14459 | 327 |
| maternal | chr5 | 13701535 | T | C | 4 | 83731 | 0 | 35 |
| combination | chr5 | 13701535 | T | C | 0 | 31414 | 0 | 2162 |
| maternal | chr9 | 115234610 | G | A | 172747 | 24 | 655 | 98 |
| combination | chr9 | 115234610 | G | A | 116353 | 40 | 3831 | 192 |
| maternal | chr5 | 13719021 | T | G | 100 | 4 | 166680 | 7 |
| combination | chr5 | 13719021 | T | G | 240 | 65 | 311800 | 11697 |
| maternal | chr8 | 125061894 | G | A | 19879 | 16 | 103 | 4 |
| combination | chr8 | 125061894 | G | A | 45848 | 21 | 4760 | 25 |
| maternal | chr4 | 69176797 | T | C | 67 | 599 | 16 | 130431 |
| combination | chr4 | 69176797 | T | C | 93 | 3729 | 40 | 120555 |
| maternal | chr5 | 13719088 | G | A | 280520 | 36 | 1267 | 40 |
| combination | chr5 | 13719088 | G | A | 226691 | 50 | 8320 | 130 |
| maternal | chr13 | 41374185 | T | C | 0 | 82 | 2 | 38046 |
| combination | chr13 | 41374185 | T | C | 18 | 4788 | 16 | 66696 |
| maternal | chr2 | 32447717 | C | T | 99 | 1212 | 16 | 280044 |
| combination | chr2 | 32447717 | C | T | 143 | 21563 | 15 | 217092 |
| maternal | chr5 | 17380269 | A | G | 18 | 0 | 12863 | 1 |
| combination | chr5 | 17380269 | A | G | 1725 | 0 | 14276 | 7 |
| maternal | chr7 | 99758135 | T | G | 278 | 19 | 313255 | 28 |
| combination | chr7 | 99758135 | T | G | 429 | 114 | 455545 | 20924 |
| maternal | chr17 | 10583713 | G | C | 306 | 13 | 418048 | 10 |
| combination | chr17 | 10583713 | G | C | 277 | 11325 | 310119 | 20 |
| maternal | chr12 | 66742182 | A | G | 20 | 4 | 44686 | 2 |
| combination | chr12 | 66742182 | A | G | 998 | 0 | 26251 | 2 |

| | | | | | | | | |
|---|---|---|---|---|---|---|---|---|
| Ref. Allele is the references allele, Alt. allele is the alternate allele, and A, C, G, and T counts represent the number of reads of each of the four DNA bases in the samples | | | | | | | | |

**Table 3: Comparison of results, four samples**

| | Assay 1 | | Assay 2 | |
|---|---|---|---|---|
| | T-cell vs. cell-free bodily fluid | T-cell vs. combination sample (cell free + 1% monocyte) | T-cell vs. cell-free bodily fluid | T-cell vs. combination sample (cell free + 1% monocyte) |
| # Informative markers | 14 | 8 | 38 | 33 |
| Mean estimate of fetal DNA content | 5.8% | 5.6% | 10.7% | 6.05% |
| Standard deviation of fetal DNA estimate | 2.54% | 2.07% | 4.3% | 1.75% |

The proof-of-principle here shows that diagnosis of, for example, fetal aneuploidy can be accomplished by selecting an appropriate panel of informative MAF SNPs weighted to the chromosomes of interest (e.g., chromosome 21), determination of fetal content by read depth computation, and comparison to control standards.

## Claims

1. A method for diagnosing or aiding in the diagnosis of a disease or condition, or assessing the risk of developing a disease or condition, or prognosing or aiding in the prognosis of a disease or condition, in a fetus comprising:
a) determining a profile of one or more markers of the disease or condition using a combination sample isolated from a maternal subject carrying the fetus, the sample comprising a cell-free bodily fluid isolated from the maternal subject and at least one of a phagocytic cell isolated from the maternal subject or a circulating diseased cell isolated from the maternal subject, wherein at least one of the phagocytic cells comprises fetal cells or fragments thereof and at least one of the circulating diseased cells is a fetal cell;
b) determining, using the profile determined from the combination sample in step a), a control maternal profile of at least one of the one or more markers; and
c) identifying a difference between the profile of a) and the profile of b), wherein the difference is indicative of the presence of said disease or condition, or of the risk of developing said disease or condition, or of the prognosis of said disease or condition, respectively, in the fetus.

2. A method for diagnosing or aiding in the diagnosis of a disease or condition, or assessing the risk of developing a disease or condition, or prognosing or aiding in the prognosis of a disease or condition, in a fetus comprising:
a) determining a profile of one or more markers of the disease or condition using a combination sample isolated from a maternal subject carrying the fetus, the sample comprising an analyte isolated from a cell-free bodily fluid isolated from the maternal subject and at least one of an analyte isolated from a population of phagocytic cells isolated from the maternal subject or an analyte isolated from a population of circulating diseased cells isolated from the maternal subject, wherein at least one of the phagocytic cells comprises fetal cells or fragments thereof and at least one of the circulating diseased cells is a fetal cell;
b) determining using the profile determined from the combination sample in step a), a control maternal profile of at least one of the one or more markers; and
c) identifying a difference between the profile of a) and the profile of b), wherein the difference is indicative of the presence of said disease or condition, or of the risk of developing said disease or condition, or of the prognosis of said disease or condition, respectively, in the fetus.

3. The method of any one of claims 1-2, wherein the phagocytic cells are neutrophils, macrophages, monocytes, dendritic cells, foam cells, mast cells, eosinophils, keratinocytes, or mixtures thereof.

4. The method of any one of claims 1-3, wherein the cell-free bodily fluid is separated from a bodily fluid.

5. The method of claim 4, wherein the bodily fluid is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, maternal urine, maternal saliva, placental sample, lavage and cervical vaginal fluid, interstitial fluid, buccal swab sample, sputum, bronchial lavage, Pap smear sample, or ocular fluid.

6. The method of any one of claims 1-5, wherein the cell-free bodily fluid is plasma or serum.

7. The method of any one of claims 1-6, wherein the one or more markers are nucleic acids, proteins, or combinations thereof.

8. The method of claim 7, wherein the nucleic acids are nucleotides, oligonucleotides, DNAs, RNAs, or DNA-RNA hybrids.

9. The method of claim 8, wherein the DNAs are double-stranded DNAs, single-stranded DNAs, multi-stranded DNAs, complementary DNAs, genomic DNAs, or non-coding DNAs.

10. The method of claim 8, wherein the RNAs are messenger RNAs (mRNAs), microRNAs (miRNAs), small nucleolar RNAs (snoRNAs), ribosomal RNAs (rRNAs), transfer RNAs (tRNAs), small interfering RNAs (siRNAs), heterogeneous nuclear RNAs (hnRNAs), or small hairpin RNAs (shRNAs).

11. The method of any one of claims 1-10, wherein the profile of a) and the profile of b) are selected from: a nucleic acid profile, a protein profile, and a combination thereof.

12. The method of claim 11, wherein the profile of a) and the profile of b) are determined by a qualitative assay, a quantitative assay, or a combination thereof.

13. The method of any claim 12, wherein the quantitative assay uses sequencing, targeted sequencing, single molecule real-time sequencing, electron microscopy-based sequencing, transistor-mediated sequencing, direct sequencing, random shotgun sequencing, Sanger dideoxy termination sequencing, exon sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, multiplex PCR, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser desorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, multiplex PCR, quantitative PCR, real-time PCR, fluorescence assay, colorimetric assay, chemiluminescent assay, or a combination thereof.

14. The method of claim 11, wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

15. The method of claim 11, wherein the nucleic acid profile is determined by polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophesis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser desorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, Hpall tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, co-amplification at lower denaturation temperature-PCR (COLD-PCR), multiplex PCR, methyl-binding PCR analysis, or a combination thereof.

16. The method of claim 11, wherein the nucleic acid profile is determined by a sequencing technique selected from the group consisting of targeted sequencing, single molecule real-time sequencing, exon sequencing, electron microscopy-based sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof.

17. The method of any one of claims 1-16, further comprising determining at least one diagnostic parameter of said disease or condition.

18. The method of claim 17, wherein the diagnostic parameter is determined by physical inspection, visual inspection, biopsy, scanning, histology, radiology, imaging, ultrasound, use of a commercial kit, genetic testing, immunological testing, analysis of bodily fluids, or monitoring neural activity.

## Patentansprüche

1. Verfahren zur Diagnose oder Hilfe in der Diagnose einer Krankheit oder Erkrankung oder zur Bestimmung des Risikos dafür eine Krankheit oder Erkrankung zu entwickeln oder zur Prognose oder Hilfe bei der Prognose einer Krankheit oder Erkrankung in einem Fötus, umfassend:
a) Bestimmung eines Profils von einem oder mehreren Markern der Krankheit oder Erkrankung unter Verwendung einer Kombinationsprobe, die aus einem mütterlichen Individuum, das den Fötus trägt, isoliert wurde, wobei die Probe eine zellfreie Körperflüssigkeit, isoliert aus dem mütterlichen Individuum, und mindestens eine phagozytische Zelle, isoliert aus dem mütterlichen Individuum, oder eine zirkulierende erkrankte Zelle, isoliert aus dem mütterlichen Individuum, umfasst, wobei mindestens eine der phagozytischen Zellen fötale Zellen oder Fragment davon umfasst und mindestens eine der zirkulierenden erkrankten Zellen eine fötale Zelle ist;
b) Bestimmung eines mütterlichen Kontrollprofils von mindestens einem des einen Markers oder der mehreren Marker unter Verwendung des aus der Kombinationsprobe im Schritt a) bestimmten Profils; und
c) Identifizierung eines Unterschieds zwischen dem Profil aus a) und dem Profil aus b), wobei der Unterschied die Anwesenheit der Krankheit oder Erkrankung, oder das Risiko die Krankheit oder Erkrankung zu entwickelnoder die Prognose der Krankheit oder Erkrankung in dem Fötus anzeigt.

2. Verfahren zur Diagnose oder Hilfe in der Diagnose einer Krankheit oder Erkrankung oder zur Bestimmung des Risikos dafür eine Krankheit oder Erkrankung zu entwickeln oder zur Prognose oder Hilfe bei der Prognose einer Krankheit oder Erkrankung in einem Fötus, umfassend:
a) Bestimmung eines Profils von einem oder mehreren Markern der Krankheit oder Erkrankung unter Verwendung einer Kombinationsprobe, die aus einem mütterlichen Individuum, das den Fötus trägt, isoliert wurde, wobei die Probe einen Analyten umfasst, der aus einer zellfreien Körperflüssigkeit, isoliert aus dem mütterlichen Individuum, isoliert wurde und mindestens einen Analyten umfasst, der aus einer Population phagozytischer Zellen, isoliert aus dem mütterlichen Individuum, isoliert wurde oder einen Analyten umfasst, der aus einer Population zirkulierender erkrankter Zellen, isoliert aus dem mütterlichen Individuum, isoliert wurde, wobei mindestens eine der phagozytischen Zellen fötale Zellen oder Fragment davon umfasst oder mindestens eine der zirkulierenden erkrankten Zellen eine fötale Zelle ist;
b) Bestimmung, unter Verwendung des aus der Kombinationsprobe im Schritt a) bestimmten Profils, eines mütterlichen Kontrollprofils von mindestens einem des einen Markers oder der mehreren Marker; und
c) Identifizierung eines Unterschieds zwischen dem Profil aus a) und dem Profil aus b), wobei der Unterschied die Anwesenheit von der Krankheit oder Erkrankung, das Risiko die Krankheit oder Erkrankung zu entwickeln oder die Prognose der Krankheit oder Erkrankung in dem Fötus anzeigt.

3. Verfahren nach einem der Ansprüche 1-2, wobei die phagozytischen Zellen Neutrophile, Makrophagen, Monozyten, dendritische Zellen, Schaumzellen, Mastzellen, Eosinophile, Keratinozyten oder Gemische davon sind.

4. Verfahren nach einem der Ansprüche 1-3, wobei die zellfreie Körperflüssigkeit von einer Körperflüssigkeit abgetrennt wurde.

5. Verfahren nach Anspruch 4, wobei die Körperflüssigkeit Blut, Urin, Stuhl, Speichel, Lymphflüssigkeit, Rückenmarksflüssigkeit, Gelenkschmiere, Zystenflüssigkeit, Abszitesflüssigkeit, Pleuraerguss, Flüssigkeit, die von einer schwangeren Frau im ersten Trimester erhalten wurde, Flüssigkeit, die von einer schwangeren Frau im zweiten Trimester erhalten wurde, Flüssigkeit, die von einer schwangeren Frau im dritten Trimester erhalten wurde, mütterliches Blut, Fruchtwasser, eine Chorionzottenprobe, mütterlicher Urin, mütterlicher Speichel, eine Plazentraprobe, Spülung- und Gebärmutterhals-vaginale Flüssigkeit, interstitielle Flüssigkeit, eine Wangenabstrichprobe, Sputum, Bronchialspülung, eine Pap-Abstrich-Probe oder Augenflüssigkeit ist.

6. Verfahren nach einem der Ansprüche 1-5, wobei die zellfreie Körperflüssigkeit Plasma oder Serum ist.

7. Verfahren nach einem der Ansprüche 1-6, wobei der eine Marker oder die mehreren Marker Nukleinsäuren, Proteine oder Kombinationen davon sind.

8. Verfahren nach Anspruch 7, wobei die Nukleinsäuren Nukleotide, Oligonukleotide, DNAs, RNAs oder DNA-RNA Hybride sind.

9. Verfahren nach Anspruch 8, wobei die DNAs doppel-strängige DNAs, einzelsträngige DNAs, multi-strängige DNAs, komplementäre DNAs, genomische DNAs oder nicht-kodierende DNAs sind.

10. Verfahren nach Anspruch 8, wobei die RNAs Boten-RNAs (mRNAs), MicroRNAs (miRNA), kleine nukleäre RNAs (snoRNAs), ribosomale RNAs (rRNAs), Transfer-RNAs (tRNAs), kleine interferierende RNAs (siRNAs), heterogene nukleäre RNAs (hnRNAs) oder kleine Haarnadel-RNAs (shRNAs) sind.

11. Verfahren nach einem der Ansprüche 1-10, wobei das Profil nach a) und das Profil nach b) ausgewählt sind aus: einem Nukleinsäureprofil, einem Proteinprofil und einer Kombination davon.

12. Verfahren nach Anspruch 11, wobei das Profil nach a) und das Profil nach b) mittels einem qualitativen Assay, einem quantitativen Assay oder einer Kombination davon bestimmt werden.

13. Verfahren nach Anspruch 12, wobei der quantitative Assay Sequenzierung, gezielte Sequenzierung, Einzelmolekül-Echtzeit-Sequenzierung, Elektronenmikroskopie-basierte Sequenzierung, Transistor-vermittelte Sequenzierung, direkte Sequenzierung, Zufall-Schrotflinten ("shotgun")-Sequenzierung, Sanger-Dideoxy-Abbruch-Sequenzierung, Exon-Sequenzierung, ganzes Genom Sequenzierung, Sequenzierung mittels Hybridisierung, Pyro-Sequenzierung, Kapillarelektrophorese, Gelelektrophorese, Duplex-Sequenzierung, Ring- Sequenzierung, Einzelbasen-Erweiterung-Sequenzierung, Festphasen-Sequenzierung, Hochdurchsatz-Sequenzierung, massive-parallele Signatursequenzierung, Emulsion-PCR, Multiplex PCR, Co-Amplifikation bei geringer Denaturierungstemperatur-PCR (COLD-PCR), Sequenzierung mittels eines reversiblen Farbe-Terminators, gepaarte Enden Sequenzierung, kurzfristige ("near term") Sequenzierung, Exonuklease-Sequenzierung, Sequenzierung mittels Ligation, Kurz-Lese ("short read")-Sequenzierung, EinzelMolekül-Sequenzierung, Sequenzierung durch Synthese, Echtzeit-Sequenzierung, reverser Terminator Sequenzierung, Nanoporen-Sequenzierung, 454-Sequenzierung, Solexa-Genom-Analysator-Sequenzierung, SOLiD®-Sequenzierung, MS-PET-Sequenzierung, Massenspektrometrie, Matrix-Assistierte Laser-Desorption-Ionisierung mit Flugzeitanalyse (MALDI-TOF) Massenspektrometrie, Elektrospray-Ionisierung (ESI) Massenspektrometrie, Oberflächenverstärkte Laser-Desorption-Ionisierung mit Flugzeitanalyse (SELDI-TOF) Massenspektrometrie, Quadrupol-Flugzeitanalyse (Q-TOF) Massenspektrometrie, atmosphärischer Druck Photoionisierung-Massenspektrometrie (APPI-MS), Fourier-Transfomation-Massenspektrometrie (FTMS), Matrix-Assistierte Laser-Desorption-Ionisierung-Fourier Transformation-Ion Zyklotron Resonanz (MALDI-TF-ICR) Massenspektrometrie, sekundäre lonen-Massenspektrometrie (SIMS), Polymerase Kettenreaktion (PCR) Analyse, Multiplex-PCR, quantitative PCR, Echtzeit-PCR, Fluoreszenz-Assay, cholorimetrischer Assay, chemielumeneszenter Assay oder eine Kombination davon.

14. Verfahren nach Anspruch 11, wobei das Nukleinsäureprofil ein genotypisches Profil, ein Einzelnukleotidpolymophismus-Profil, ein Genmutation-Profil, ein Genekopienanzahl-Profil, ein DNA-Methylierungs-Profil, ein DNA-Acetylierungs-Profil, ein Chromosom-Dosierungs-Profil, ein Genexpression-Profil oder eine Kombination davon ist.

15. Verfahren nach Anspruch 11, wobei das Nukleinsäureprofil bestimmt wird durch Polymerase Kettenreaktion (PCR) Analyse, Sequenzierungs-Analyse, elektrophoretische Analyse, Restiktionsfragment-Längenpolymorphismus (RFLP) Analyse, Northern-Blot-Analyse, quantiative PCR, reverse Transkriptase PCR Analyse (RT-PCR), Allel-spezifische Oligonukelotid Hybridisierungs-Analyse, vergleichende genomische Hybridisierung, Heteroduplex Mobilität Assay (HMA), Einzelstrang-Konformationspolymophismus (SSCP), denaturierende Gradientengel-Elektrophorese (DGGE), RNAse Fehlpaarungs ("mismatch")-Analyse, Massenspektrometrie, Tandem-Massenspektrometrie, Matrix-Assistierte Laser-Desorption-Ionisierung mit Flugzeitanalyse (MALDI-TOF) Massenspektrometrie, Elektrospray-Ionisierung (ESI) Massenspektrometrie, Oberflächenverstärkte Laser-Desorption-Ionisierung mit Flugzeitanalyse (SELDI-TOF) Massenspektrometrie, Quadrupol-Flugzeitanalyse (Q-TOF) Massenspektrometrie, atmosphärischer Druck Photoionisierungs-Massenspektrometrie (APPI-MS), Fourier-Transfomations-Massenspektrometrie (FTMS), Matrix-Assistierte Laser-Desorption-Ionisierung-Fourier Transformation-Ion Zyklotron Resonanz (MALDI-TF-ICR) Massenspektrometrie, sekundäre lonen-Massenspektrometrie (SIMS), Oberflächenplasmonresonanz, Southern-Blot-Analyse, In-situ-Hybridisierung, Fluoreszenz-in-situ-Hybridisierung (FISH), chromogene In-situ-Hybridisierung (CISH), Immunohistochemie (IHC), Microarray, vergleichende genomische Hybridisierung, Karyotypisierung, Multiplex Ligations-abhängige Sonden-Amplifikation (MLPA), quantitative Multiplex-PCR kurzer fluoreszierender Fragmente (QMPSF), Mikroskopie, Methylierungsabhängige PCR (MSP) Assay, Hpall-winzige-Fragmente Anreicherung mittels Ligation-vermittelter PCR (HELP) Assay, radioaktives Acetat Markierung-Assays, colorimetrische DNA Acetylierungs-Assay, Chromatinimmunopräzipitation kombiniert mit Mikroarray (ChIP-on-chip) Assay, Restriktion-Orientierungspunkt genomisches Scannen, methylierte DNA Immunopräzipitation (MeDIP), Molekularbruch-Licht Assay für auf DNA-Adenin-Methyltransferase-Aktivität, chromatographische Auftrennung, Methylierungssensitive Restriktionsenzym-Analyse, Bisulfit-vermittelte Konversion von nicht-methyliertem Cytosin zu Uracil; Co-Amplifikation hei niedrigerer Denaturierungstemperatur-PCR (COLD-PCR), Multiplex-PCR, Methylbindungs-PCR-Analyse oder eine Kombination davon.

16. Verfahren nach Anspruch 11, wobei das Nukleinsäureprofil durch eine Sequenzierungstechnik bestimmt wird, ausgewählt aus gezielter Sequenzierung, Einzelmolekül-Echtzeit-Sequenzierung, Exon-Sequenzierung, Elektronenmikroskopie-basierter Sequenzierung, Transistor-vermittelter Sequenzierung, direkter Sequenzierung, Zufall-Schrotflinten ("shotgun")-Sequenzierung, Sanger-Dideoxy-Abbruch-Sequenzierung, ganzes Genom Sequenzierung, Sequenzierung mittels Hybridisierung, Pyro-Sequenzierung, Kapillarelektrophorese, Gelelektrophorese, Duplex-Sequenzierung, Ring-Sequenzierung, Einzelbasen-Erweiterungs-Sequenzierung, Festphasen-Sequenzierung, Hochdurchsatz-Sequenzierung, massive-parallele Signatursequenzierung, Emulsions-PCR, Co-Amplifikation bei geringer Denaturierungstemperatur-PCR (COLD-PCR), Multiplex-PCR, Sequenzierung mittels eines reversiblen Farbe-Terminators, gepaarte Enden Sequenzierung, kurzfristige ("near term") Sequenzierung, Exonuklease-Sequenzierung, Sequenzierung mittels Ligation, Kurz-Lese ("short read")-Sequenzierung, EinzelMolekül-Sequenzierung, Sequenzierung durch Synthese, Echtzeit-Sequenzierung, reverser Terminator Sequenzierung, Nanoporen-Sequenzierung, 454-Sequenzierung, Solexa-Genom-Analysator-Sequenzierung, SOLiD®-Sequenzierung, MS-PET-Sequenzierung, Massenspektrometrie und einer Kombination davon.

17. Verfahren nach einem der Ansprüche 1-16, weiterhin umfassend die Bestimmung mindestens eines diagnostischen Parameters der Krankheit oder Erkrankung.

18. Verfahren nach Anspruch 17, wobei der diagnostische Parameter mittels physikalischer Inspektion, visueller Inspektion, Biopsie, Scannen, Histologie, Radiologie, Bildgebung, Ultraschall, Verwendung eines kommerziellen Kits, genetischen Tests, immunologischen Tests, Analyse von Körperflüssigkeiten oder der Überwachung neuraler Aktivität bestimmt wird.

## Revendications

1. Méthode pour diagnostiquer ou aider au diagnostic d'une maladie ou d'un état, ou pour déterminer le risque de développer une maladie ou un état, ou pour pronostiquer ou aider au pronostic d'une maladie ou d'un état, chez un foetus, comprenant:
a) la détermination d'un profil d'un ou plusieurs marqueurs de la maladie ou de l'état par utilisation d'un échantillon en combinaison isolé auprès d'un sujet maternel portant le foetus, l'échantillon comprenant un fluide corporel sans cellules isolé à partir du sujet maternel et au moins l'une parmi une cellule phagocytaire isolée à partir du sujet maternel ou une cellule malade en circulation isolée à partir du sujet maternel, où au moins l'une des cellules phagocytaires comprend des cellules foetales ou des fragments de celles-ci et au moins l'une des cellules malades en circulation est une cellule foetale;
b) la détermination, par utilisation du profil déterminé à partir de l'échantillon en combinaison dans l'étape a), d'un profil maternel témoin d'au moins l'un parmi le ou les marqueurs; et
c) l'identification d'une différence entre le profil de a) et le profil de b), laquelle différence est indicative de la présence de ladite maladie ou dudit état, ou du risque de développer ladite maladie ou ledit état, ou du pronostic de ladite maladie ou dudit état, respectivement, chez le foetus.

2. Méthode pour diagnostiquer ou aider au diagnostic d'une maladie ou d'un état, ou pour déterminer le risque de développer une maladie ou un état, ou pour pronostiquer ou aider au pronostic d'une maladie ou d'un état, chez un foetus, comprenant:
a) la détermination d'un profil d'un ou plusieurs marqueurs de la maladie ou de l'état par utilisation d'un échantillon en combinaison isolé auprès d'un sujet maternel portant le foetus, l'échantillon comprenant un analyte isolé à partir d'un fluide corporel sans cellules isolé à partir du sujet maternel et au moins l'un parmi un analyte isolé à partir d'une population de cellules phagocytaires isolées à partir du sujet maternel ou un analyte isolé à partir d'une population de cellules malades en circulation isolées à partir du sujet maternel, où au moins l'une des cellules phagocytaires comprend des cellules foetales ou des fragments de celles-ci et au moins l'une des cellules malades en circulation est une cellule foetale;
b) la détermination, par utilisation du profil déterminé à partir de l'échantillon en combinaison dans l'étape a), d'un profil maternel témoin d'au moins l'un parmi le ou les marqueurs; et
c) l'identification d'une différence entre le profil de a) et le profil de b), laquelle différence est indicative de la présence de ladite maladie ou dudit état, ou du risque de développer ladite maladie ou ledit état, ou du pronostic de ladite maladie ou dudit état, respectivement, chez le foetus.

3. Méthode selon l'une quelconque des revendications 1 et 2, dans laquelle les cellules phagocytaires sont des neutrophiles, des macrophages, des monocytes, des cellules dendritiques, des cellules spumeuses, des mastocytes, des éosinophiles, des kératinocytes, ou des mélanges de ceux-ci.

4. Méthode selon l'une quelconque des revendications 1 à 3, dans laquelle le fluide corporel sans cellules est séparé à partir d'un fluide corporel.

5. Méthode selon la revendication 4, dans laquelle le fluide corporel est le sang, l'urine, les selles, la salive, la lymphe, le liquide céphalo-rachidien, le liquide synovial, le fluide cystique, l'ascite, une effusion pleurale, un fluide obtenu auprès d'une femme enceinte au cours du premier trimestre, un fluide obtenu auprès d'une femme enceinte au cours du deuxième trimestre, un fluide obtenu auprès d'une femme enceinte au cours du troisième trimestre, le sang maternel, le liquide amniotique, un échantillon de villosités choriales, l'urine maternelle, la salive maternelle, un échantillon de placenta, un lavage et le fluide vaginal cervical, le fluide interstitiel, un échantillon d'écouvillonnage buccal, les crachats, un lavage bronchique, un échantillon de test Pap, ou le fluide oculaire.

6. Méthode selon l'une quelconque des revendications 1 à 5, dans laquelle le fluide corporel sans cellules est le plasma ou le sérum.

7. Méthode selon l'une quelconque des revendications 1 à 6, dans laquelle le ou les marqueurs sont des acides nucléiques, des protéines, ou des combinaisons de ceux-ci.

8. Méthode selon la revendication 7, dans laquelle les acides nucléiques sont des nucléotides, des oligonucléotides, des ADN, des ARN ou des hybrides d'ADN-ARN.

9. Méthode selon la revendication 8, dans laquelle les ADN sont des ADN double brin, des ADN simple brin, des ADN multibrins, des ADN complémentaires, des ADN génomiques, ou des ADN non codants.

10. Méthode selon la revendication 8, dans laquelle les ARN sont des ARN messagers (ARNm), des micro-ARN (ARNmi), des petits ARN nucléolaires (ARNsno), des ARN ribosomiaux (ARNr), des ARN de transfert (ARNt), des petits ARN interférants (ARNsi), des ARN nucléaires hétérogènes (ARNhn), ou des petits ARN en épingle à cheveux (ARNsh).

11. Méthode selon l'une quelconque des revendications 1 à 10, dans laquelle le profil de a) et le profil de b) sont choisis parmi: un profil d'acides nucléiques, un profil de protéines, et une combinaison de ceux-ci.

12. Méthode selon la revendication 11, dans laquelle le profil de a) et le profil de b) sont déterminés par un dosage qualitatif, un dosage quantitatif, ou une combinaison de ceux-ci.

13. Méthode selon la revendication 12, dans laquelle le dosage quantitatif utilise un séquençage, un séquençage ciblé, un séquençage en temps réel d'une seule molécule, un séquençage basé sur une microscopie électronique, un séquençage à médiation par un transistor, un séquençage direct, un séquençage en aveugle aléatoire, un séquençage par terminaison au didésoxy de Sanger, un séquençage d'exons, un séquençage de génome complet, un séquençage par hybridation, un pyro-séquençage, une électrophorèse capillaire, une électrophorèse sur gel, un séquençage en duplex, un séquençage cyclique, un séquençage par extension de base unique, un séquençage en phase solide, un séquençage à haut débit, un séquençage à signature massivement parallèle, une PCR en émulsion, une PCR en multiplex, une PCR par co-amplification à température de dénaturation plus basse (COLD-PCR), un séquençage par terminateur de colorant réversible, un séquençage par extrémités appariées, un séquençage proche du terme, un séquençage d'exonucléases, un séquençage par ligature, un séquençage à lecture courte, un séquençage d'une seule molécule, un séquençage par synthèse, un séquençage en temps réel, un séquençage par terminateur inverse, un séquençage à nanopores, un séquençage 454, un séquençage par analyseur de génome Solexa, un séquençage SOLiD®, un séquençage MS-PET, une spectrométrie de masse, une spectrométrie de masse à temps de vol par désorption-ionisation laser assistée par matrice (MALDI-TOF), une spectrométrie de masse par ionisation de pulvérisation d'électrons (ESI), une spectrométrie de masse à temps de vol par désorption/ionisation laser amplifiée en surface (SELDI-TOF), une spectrométrie de masse à temps de vol quadripôle (Q-TOF), une spectrométrie de masse par photo-ionisation sous la pression atmosphérique (APPI-MS), une spectrométrie de masse à transformée de Fourier (FTMS), une spectrométrie de masse par désorption-ionisation laser assistée par matrice, transformée de Fourier et résonance de cyclotron ionique (MALDI-FT-ICR), une spectrométrie de masse à ions secondaires (SIMS), une analyse d'amplification en chaîne par polymérase (PCR), une PCR en multiplex, une PCR quantitative, une PCR en temps réel, un dosage de fluorescence, un dosage colorimétrique, un dosage chimioluminescent, ou une combinaison de ceux-ci.

14. Méthode selon la revendication 11, dans laquelle le profil d'acides nucléiques est un profil génotypique, un profil de polymorphismes de nucléotides isolés, un profil de mutations géniques, un profil de nombre de copies d'un gène, un profil de méthylation d'ADN, un profil d'acétylation d'ADN, un profil de dosage de chromosomes, un profil d'expression d'un gène, ou une combinaison de ceux-ci.

15. Méthode selon la revendication 11, dans laquelle le profil d'acides nucléiques est déterminé par analyse par amplification en chaîne par polymérase (PCR), analyse par séquençage, analyse électrophorétique, analyse de polymorphisme de longueur de fragment de restriction (RFLP), analyse par transfert Northern, PCR quantitative, analyse PCR par transcriptase inverse (RT-PCR), analyse d'hybridation d'oligonucléotides à spécificité d'allèle, hybridation génomique comparative, dosage de mobilité d'hétéroduplex (HMA), polymorphisme de conformation de simple brin (SSCP), électrophorèse sur gel à gradient dénaturant (DGGE), analyse de mésappariement d'ARNase, spectrométrie de masse, spectrométrie de masse en tandem, spectrométrie de masse à temps de vol par désorption-ionisation laser assistée par matrice (MALDI-TOF), spectrométrie de masse par ionisation de pulvérisation d'électrons (ESI), spectrométrie de masse à temps de vol par désorption/ionisation laser amplifiée en surface (SELDI-TOF), spectrométrie de masse à temps de vol quadripôle (Q-TOF), spectrométrie de masse par photo-ionisation sous la pression atmosphérique (APPI-MS), spectrométrie de masse à transformée de Fourier (FTMS), spectrométrie de masse par désorption-ionisation laser assistée par matrice, transformée de Fourier et résonance de cyclotron ionique (MALDI-FT-ICR), spectrométrie de masse à ions secondaires (SIMS), résonance plasmonique de surface, analyse par transfert Southern, hybridation in situ, hybridation in situ avec fluorescence (FISH), hybridation chromogénique in situ (CISH), immunohistochimie (IHC), puce à ADN, hybridation génomique comparative, caryotypage, amplification de sonde dépendante d'une ligature multiplex (MLPA), PCR multiplex quantitative de fragments fluorescents courts (QMPSF), microscopie, dosage PCR spécifique d'une méthylation (MSP), dosage PCR à médiation par un enrichissement en minuscules fragments Hpall par ligature (HELP), dosages de marquage à l'acétate radioactif, dosage d'acétylation d'ADN colorimétrique, dosage d'immunoprécipitation de chromatine combinée à une puce à ADN (ChIP-sur-puce), balayage génomique de points de repère de restriction, immunoprécipitation d'ADN méthylé (MeDIP), dosage de lumière de dissociation moléculaire pour l'activité de l'ADN adénine méthyltransférase, séparation chromatographique, analyse par enzyme de restriction sensible à la méthylation, conversion entraînée par le bisulfite de cytosine non méthylée en uracile, PCR par co-amplification à température de dénaturation réduite (COLD-PCR), PCR en multiplex, analyse PCR de liaison de méthyle, ou une combinaison de ceux-ci.

16. Méthode selon la revendication 11, dans laquelle le profil d'acides nucléiques est déterminé par une technique de séquençage choisie dans le groupe constitué par un séquençage ciblé, un séquençage en temps réel d'une seule molécule, un séquençage d'exons, un séquençage basé sur une microscopie électronique, un séquençage à médiation par un transistor, un séquençage direct, un séquençage en aveugle aléatoire, un séquençage par terminaison au didésoxy de Sanger, un séquençage de génome complet, un séquençage par hybridation, un pyro-séquençage, une électrophorèse capillaire, une électrophorèse sur gel, un séquençage en duplex, un séquençage cyclique, un séquençage par extension de base unique, un séquençage en phase solide, un séquençage à haut débit, un séquençage à signature massivement parallèle, une PCR en émulsion, une PCR par co-amplification à température de dénaturation plus basse (COLD-PCR), une PCR en multiplex, un séquençage par terminateur de colorant réversible, un séquençage par extrémités appariées, un séquençage proche du terme, un séquençage d'exonucléases, un séquençage par ligature, un séquençage à lecture courte, un séquençage d'une seule molécule, un séquençage par synthèse, un séquençage en temps réel, un séquençage par terminateur inverse, un séquençage à nanopores, un séquençage 454, un séquençage par analyseur de génome Solexa, un séquençage SOLiD®, un séquençage MS-PET, une spectrométrie de masse, et une combinaison de ceux-ci.

17. Méthode selon l'une quelconque des revendications 1 à 16, comprenant en outre la détermination d'au moins un paramètre diagnostique de ladite maladie ou dudit état.

18. Méthode selon la revendication 17, dans laquelle le paramètre diagnostique est déterminé par inspection physique, inspection visuelle, biopsie, scanner, histologie, radiologie, imagerie, ultrasons, utilisation d'un kit du commerce, test génétique, test immunologique, analyse de fluides corporels, ou surveillance d'activité neurale.
